# EUROPÄISCHE PATENTSCHRIFT

(11) **EP 2 858 980 B1**
(45) Veröffentlichungstag und Bekanntmachung des Hinweises auf die Patenterteilung: **26.06.2019**
(21) Anmeldenummer: 13723667.5
(22) Anmeldetag: 06.05.2013
(51) Int. Cl.: C07D 311/82, C07D 219/02, C07D 221/18, C07D 223/26, C07D 333/76, C07C 211/61, C07D 407/04, C07C 217/92, C07D 265/38, C07D 279/36, C07F 7/08, C07D 213/38, C07D 307/91, C09K 11/06, H01L 51/00

(54) **PHENANTHRENVERBINDUNGEN FÜR ORGANISCHE ELEKTRONISCHE VORRICHTUNGEN**
PHENANTHRENES FOR USE IN ORGANIC ELECTRONIC DEVICES
PHENANTHRÈNES POUR L'UTILISATION DANS DES DISPOSITIFS ÉLECTRONIQUES ORGANIQUES

(30) Priorität: 06.06.2012 DE 102012011335
(43) Veröffentlichungstag der Anmeldung: 15.04.2015
(73) Patentinhaber: Merck Patent GmbH, 64293 Darmstadt (DE)
(72) Erfinder: MUJICA-FERNAUD, Teresa, 64283 Darmstadt (DE); BUESING, Arne, 65929 Frankfurt am Main (DE); VOGES, Frank, 67098 Bad Duerkheim (DE); KROEBER, Jonas Valentin, 60311 Frankfurt am Main (DE)
(86) Internationale Anmeldenummer: PCT/EP2013/001333
(87) Internationale Veröffentlichungsnummer: WO 2013/182263

(56) Entgegenhaltungen:
- WO-A1-2005/104264
- WO-A1-2011/136482
- WO-A1-2011/136484
- WO-A1-2012/018120
- WO-A2-2012/099376
- US-A1- 2010 109 555
- XIAOMING LIU ET AL: "New rhenium(i) complexes with substituted diimine ligands for highly efficient phosphorescent devices fabricated by a solution process", JOURNAL OF MATERIALS CHEMISTRY, Bd. 22, Nr. 8, 1. Januar 2012 (2012-01-01) , Seite 3485, XP055073444, ISSN: 0959-9428, DOI: 10.1039/c2jm14222h
- ARTIS KLAPARS ET AL: "A General and Efficient Copper Catalyst for the Amidation of Aryl Halides and the N-Arylation of Nitrogen Heterocycles", JOURNAL OF THE AMERICAN CHEMICAL SOCIETY, ACS PUBLICATIONS, US, Bd. 123, 7. Dezember 2001 (2001-12-07), Seiten 7727-7729, XP002211988, ISSN: 0002-7863, DOI: 10.1021/JA016226Z

## Beschreibung

Die vorliegende Erfindung betrifft neue organische Verbindungen, die Verwendung der Verbindung in einer elektronischen Vorrichtung, sowie eine elektronische Vorrichtung enthaltend wenigstens eine der Verbindungen. Weiterhin betrifft die vorliegende Erfindung ein Verfahren zur Herstellung der Verbindungen sowie Zusammensetzungen und Formulierungen enthaltend wenigstens eine der Verbindungen.

Die Entwicklung von funktionellen Verbindungen zur Verwendung in elektronischen Vorrichtungen ist aktuell Gegenstand intensiver Forschung. Ziel ist hierbei insbesondere die Entwicklung von Verbindungen, mit denen verbesserte Eigenschaften der elektronischen Vorrichtungen in einem oder mehreren relevanten Punkten erzielt werden können, wie beispielsweise Leistungseffizienz, Lebensdauer oder Farbkoordinaten des emittierten Lichts.

Unter dem Begriff elektronische Vorrichtung werden gemäß der vorliegenden Erfindung unter anderem organische integrierte Schaltungen (OICs), organische Feld-Effekt-Transistoren (OFETs), organische Dünnfilmtransistoren (OTFTs), organische lichtemittierende Transistoren (OLETs), organische Solarzellen (OSCs), organische optische Detektoren, organische Photorezeptoren, organische Feld-Quench-Devices (OFQDs), organische lichtemittierende elektrochemische Zellen (OLECs), organische Laserdioden (O-Laser) und organische Elektrolumineszenzvorrichtungen (OLEDs) verstanden.

Von besonderem Interesse ist die Bereitstellung von Verbindungen zur Verwendung in den zuletzt genannten, als OLEDs bezeichneten elektronischen Vorrichtungen. Der allgemeine Aufbau sowie das Funktionsprinzip von OLEDs ist dem Fachmann bekannt und unter anderem in US 4539507, US 5151629, EP 0676461 und WO 1998/27136 beschrieben.

Betreffend die Leistungsdaten von OLEDs sind noch weitere Verbesserungen erforderlich, insbesondere in Hinblick auf eine breite kommerzielle Verwendung, beispielsweise in Anzeigevorrichtungen oder als Lichtquellen. Von besonderer Bedeutung sind in diesem Zusammenhang die Lebensdauer, die Effizienz und die Betriebsspannung der OLEDs sowie die realisierten Farbwerte. Zudem ist es wünschenswert, dass die Verbindungen zur Verwendung als Funktionsmaterialien in elektronischen Vorrichtungen eine hohe thermische Stabilität und eine hohe Glasübergangstemperatur aufweisen und sich unzersetzt sublimieren lassen.

In diesem Zusammenhang besteht insbesondere Bedarf an alternativen Lochtransportmaterialien. Bei Lochtransportmaterialien gemäß dem Stand der Technik steigt im Allgemeinen die Spannung mit der Schichtdicke der Lochtransportschicht an. In der Praxis wäre häufig eine höhere Schichtdicke der Lochtransportschicht wünschenswert, dies hat jedoch oftmals eine höhere Betriebsspannung und schlechtere Leistungsdaten zur Folge. In diesem Zusammenhang besteht Bedarf an neuen Lochtransportmaterialien, die eine hohe Ladungsträgerbeweglichkeit aufweisen, so dass dickere Lochtransportschichten mit lediglich geringem Anstieg der Betriebsspannung realisiert werden können.

Im Stand der Technik ist die Verwendung insbesondere von Arylaminverbindungen und Carbazolverbindungen als Lochtransportmaterialien für OLEDs bekannt.

Die Anmeldung WO 2010/083871 offenbart die Verwendung von Dihydroacridin-Derivaten, welche mit einer oder mehreren Arylaminogruppen substituiert sind, als Funktionsmaterialien in OLEDs, bevorzugt als Lochtransport- und Lochinjektionsmaterialien.

KR 2011047803 offenbart Phenanthrene, die Diamine oder Monoamine darstellen können, wobei die Amingruppe Im Fall des Monoamins nicht via Position 3 des Phenanthrens gebunden ist.

JP 1992321649 offenbart aromatische tertiäre Amine, die zwei Alkengruppen enthalten. Offenbart wird zudem eine Einzelverbindung, die ein Phenanthren zeigt, das in Position 3 eine Amingruppe enthält, wobei das Amin weiterhin mit zwei aromatischen Gruppen substituiert ist, die ihrerseits je eine Aklengruppe enthalten.

US 2008/0182129 offenbart mit amoratischen Aminen substituierte Anthracene, wobei die aromatische Gruppe auch ein Phenanthren sein kann.

WO 2012/018120 A1 offenbart substituierte Phenanthrenamine, die als Matrixmaterial in OLEDs und als Lochtransport- oder Lochinjektionsmaterial verwendet werden.

US 2010/109555 A1 offenbart Phenanthrenderivate, die als Lochtransport- und Elektronentransportmaterialien in OLEDs eingesetzt werden.

X. Liu et al. (J. Mater. Chem., 2012, 22, 3485-3492) offenbart phosphoreszierende Metallkomplexe mit phenanthrolinhaltigen Liganden.

In WO 2011/136482 werden substituierte Phenanthrene als Ladungstransport Verbindungen beschrieben. Die hierin offenbarten Phenanthrene sind wenigstens zweifach substituiert wobei beide Substituenten eine Amingruppe enthalten.

Es besteht jedoch unverändert Bedarf an neuen Lochtransport- und Lochinjektionsmaterialien zur Verwendung in OLEDs. Insbesondere besteht Bedarf an Materialien, mit denen die oben genannten, hoch erwünschten Verbesserungen der Leistungsdaten und Eigenschaften der OLEDs erreicht werden können.

Ebenfalls besteht Bedarf an neuen Matrixmaterialien zur Verwendung in OLEDs sowie in anderen elektronischen Vorrichtungen. Insbesondere besteht Bedarf an Matrixmaterialien für phosphoreszierende Dotanden sowie an Matrixmaterialien für Mixed-Matrix-Systeme, welche bevorzugt zu guter Effizienz, hoher Lebensdauer und geringer Betriebsspannung der elektronischen Vorrichtungen führen.

Der vorliegenden Erfindung liegt somit die Aufgabe zu Grunde, Verbindungen bereitzustellen, welche sich zur Verwendung in elektronischen Vorrichtungen wie beispielsweise OLEDs eignen, und welche insbesondere als Lochtransportmaterialien und/oder als Lochinjektionsmaterialien und/oder als lichtemittierende Materialien und/oder als Matrixmaterialien eingesetzt werden können.

Im Rahmen der vorliegenden Erfindung wurde überraschend gefunden, dass sich Verbindungen der unten angegebenen Formel (1) ausgezeichnet für die oben genannten Verwendungen eignen.

Gegenstand der Erfindung ist somit eine Verbindung einer Formel (1) 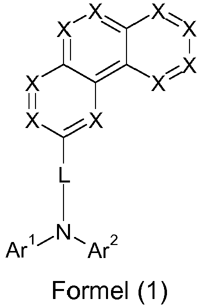 wobei die verwendeten Symbole und Indices die in Anspruch 1 angegebene Bedeutung haben.

Die Zählweise am Phenanthren ist dabei wie folgt festgelegt.

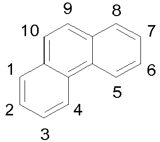

Eine Arylgruppe im Sinne dieser Erfindung enthält 6 bis 60 aromatische Ringatome; eine Heteroarylgruppe im Sinne dieser Erfindung enthält 5 bis 60 aromatische Ringatome, von denen mindestens eines ein Heteroatom darstellt. Die Heteroatome sind bevorzugt ausgewählt aus N, O und S. Dies stellt die grundlegende Definition dar. Werden in der Beschreibung der vorliegenden Erfindung andere Bevorzugungen angegeben, beispielsweise bezüglich der Zahl der aromatischen Ringatome oder der enthaltenen Heteroatome, so gelten diese.

Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin oder Thiophen, oder ein kondensierter (annellierter) aromatischer bzw. heteroaromatischer Polycyclus, beispielsweise Naphthalin, Phenanthren, Chinolin oder Carbazol verstanden. Ein kondensierter (annellierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Unter einer Aryloxygruppe gemäß der Definition der vorliegenden Erfindung wird eine Arylgruppe, wie oben definiert, verstanden, welche über ein Sauerstoffatom gebunden ist. Eine analoge Definition gilt für Heteroaryloxygruppen.

Ein aromatisches Ringsystem im Sinne dieser Erfindung enthält 6 bis 60 C-Atome im Ringsystem. Ein heteroaromatisches Ringsystem im Sinne dieser Erfindung enthält 5 bis 60 aromatische Ringatome, von denen mindestens eines ein Heteroatom darstellt. Die Heteroatome sind bevorzugt ausgewählt aus N, O und/oder S.

Unter einem aromatischen oder heteroaromatischen Ringsystem mit 5 - 60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Biphenylen, Terphenyl, Terphenylen, Quaterphenyl, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Kombinationen dieser Gruppen.

Im Rahmen der vorliegenden Erfindung werden unter einer geradkettigen Alkylgruppe mit 1 bis 40 C-Atomen bzw. einer verzweigten oder cyclischen Alkylgruppe mit 3 bis 40 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer Alkoxy- oder Thioalkylgruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung verknüpft sind. Dies wird durch das folgende Schema verdeutlicht:

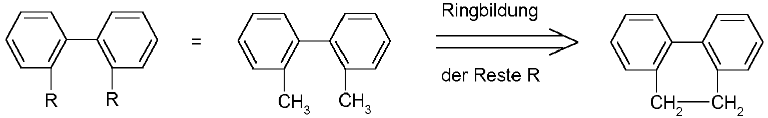

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

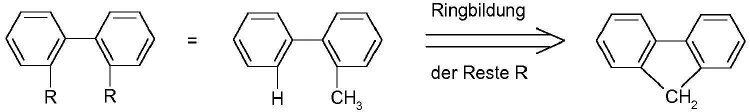

Eine weiterhin bevorzugte Ausführungsform der vorliegenden Erfindung ist die Verbindung der allgemeinen Formel (4), wobei die Symbole wie oben angegeben definiert sind.

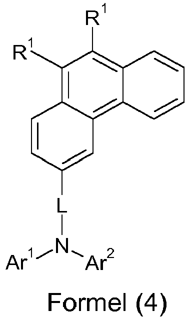

Weiterhin bevorzugt im Sinne der vorliegenden Erfindung ist die Verbindung der allgemeinen Formel (4a), wobei die Symbole wie oben angegeben definiert sind und die an anderer Stelle genannten bevorzugten Ausführungsformen auch für die Formel (4a) gelten. So gilt, beispielsweise, dass in einer besonders bevorzugten Ausführungsform der vorliegenden Erfindung L in der Verbindung der vorliegenden Formel eine Einfachbindung ist und die Gruppen Ar¹ und Ar² ganz besonders bevorzugt mindestens jeweils zwei Aryl- oder Heteroarylgruppen enthalten. 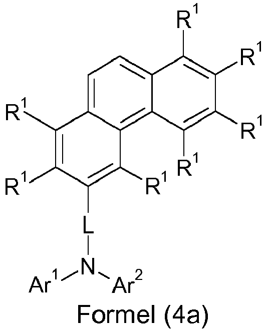 Wie oben bereits beschrieben, enthält die Verbindung nach Formel (1) neben dem Phenanthren keinen weiteren kondensierten aromatischen oder heteroaromatischen Ring mit mehr als 10 Ringatomen. Folglich stellen die Substituenten Ar¹ und Ar² bzw. die Ringe A und B keine kondensierte aromatische oder heteroaromatische Ringsysteme mit mehr als 10 Ringatomen dar.

Wenigstens einer der Reste Ar¹ und Ar² in Formel (1) enthält mehr als einen Ring, ganz bevorzugt ist, wenn beide Reste Ar¹ und Ar² wenigstens 2 oder mehr Ringe enthalten.

Weiterhin bevorzugt im Sinne der vorliegenden Erfindung ist eine Verbindung der allgemeinen Formel (1), dadurch charakterisiert, dass sie insgesamt wenigstens 26, ganz bevorzugt insgesamt wenigstens 32, ganz besonders bevorzugt insgesamt wenigstens 38 und insbesondere bevorzugt insgesamt wenigstens 44 Ringatome enthält.

In einer insbesonders bevorzugten Ausführungsform der vorliegenden Erfindung enthält die Verbindung gemäß den Formel (1) bis (4a) in keinen der direkt an das Phenanthren gebundenen Reste R¹ eine aromatische oder heteroaromatische Gruppe oder ein aromatisches oder heteroaromatisches Ringsystem. Die Reste R¹ am Phenanthren in Formel (1) enthalten keine weiteren Amingruppen.

Dies führt zu besonders gut geeigneten Verbindungen für die Verwendung in organischen elektronischen und insbesondere in organischen elektrolumineszierenden Vorrichtungen.

Die Gruppen Ar¹ und Ar² in Formel (1) sind ausgewählt aus den folgenden Formeln (8) bis (100): 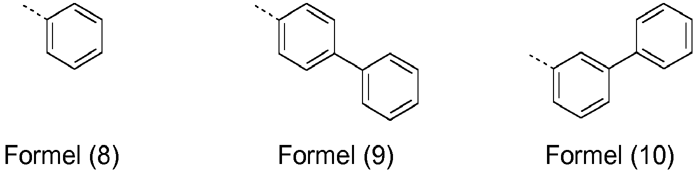 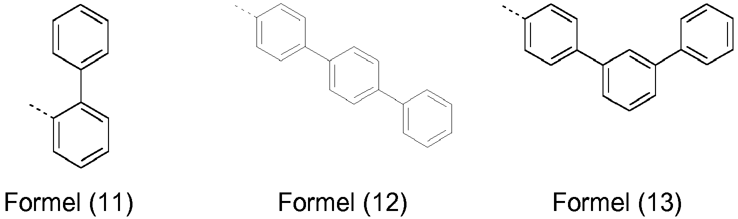 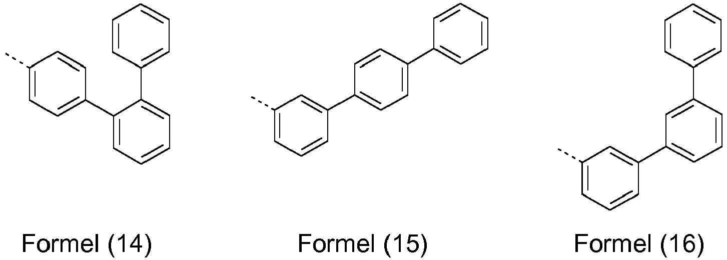 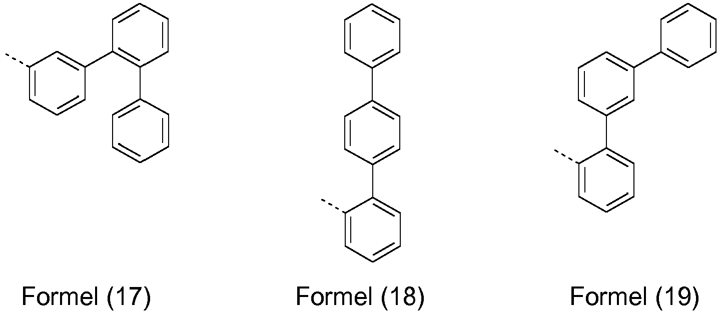 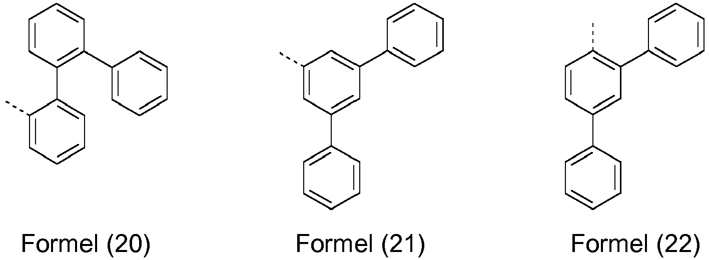 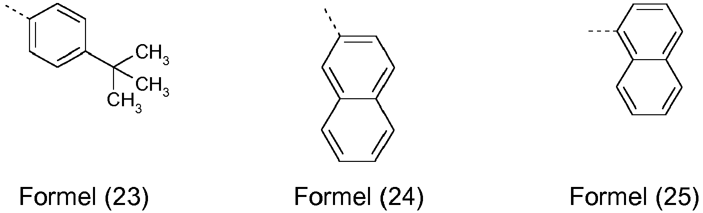 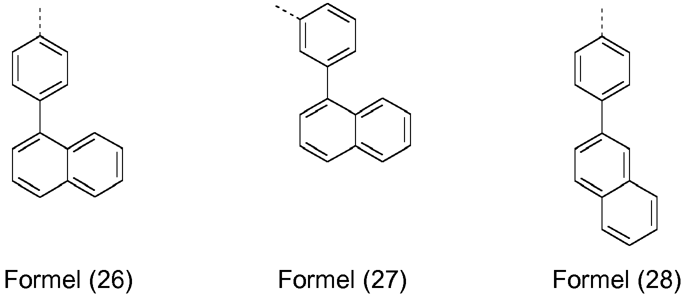 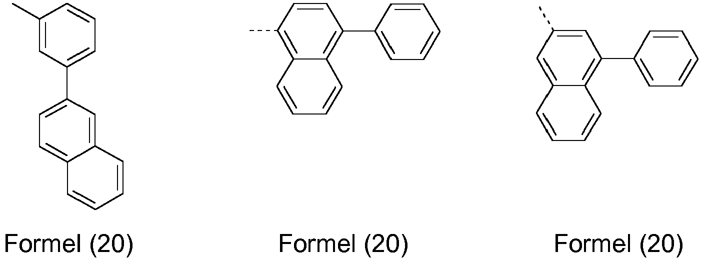 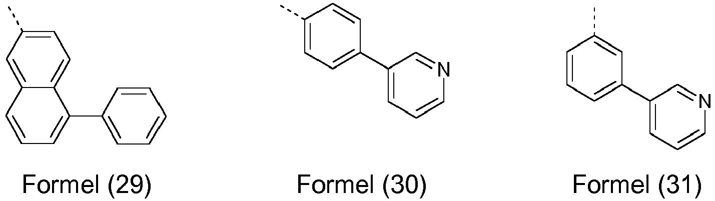 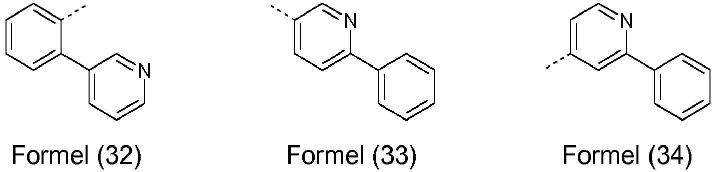 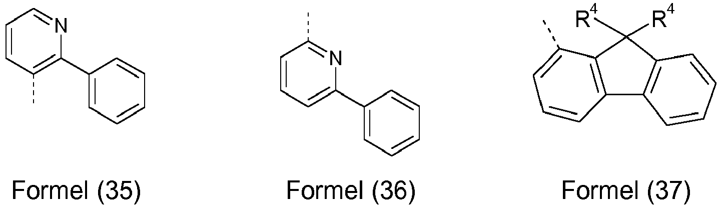 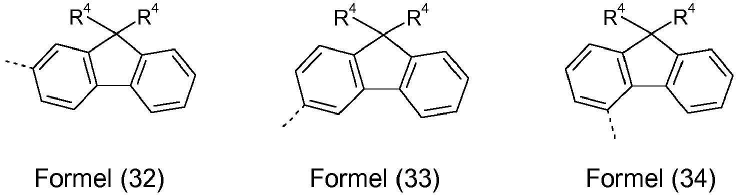 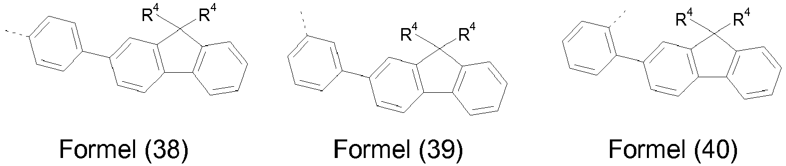 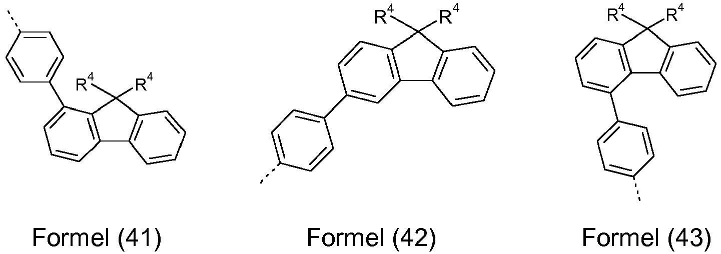 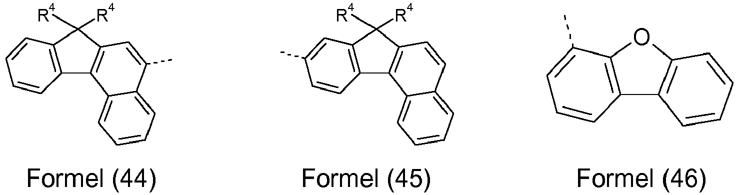 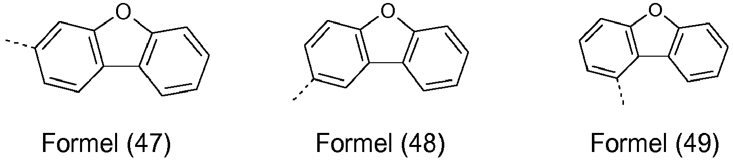 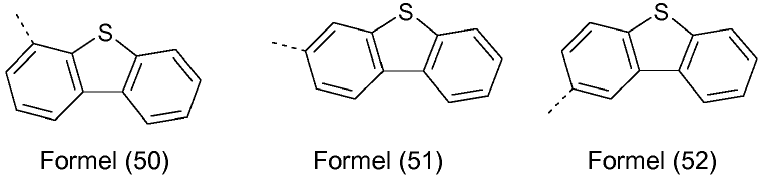 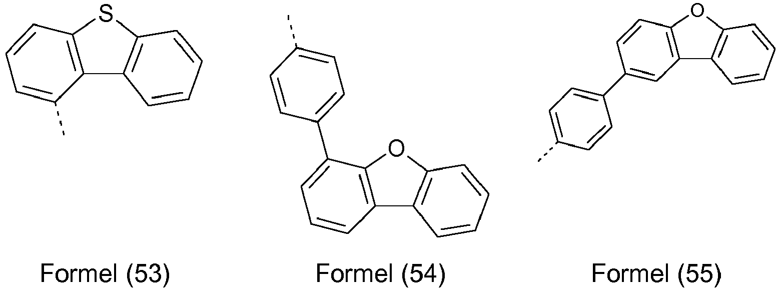 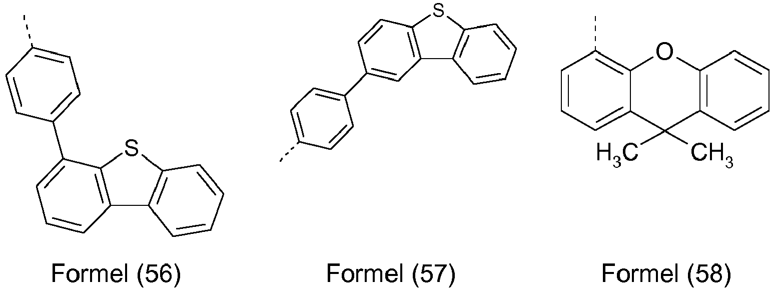 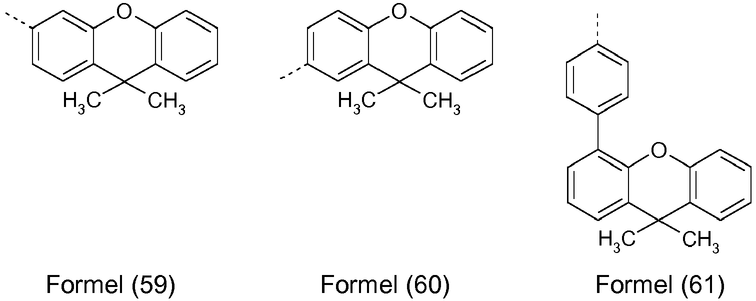 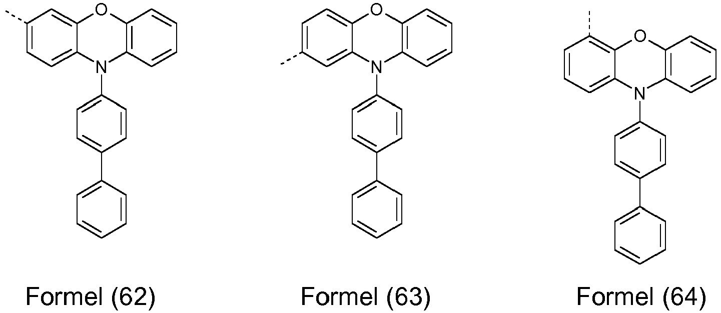 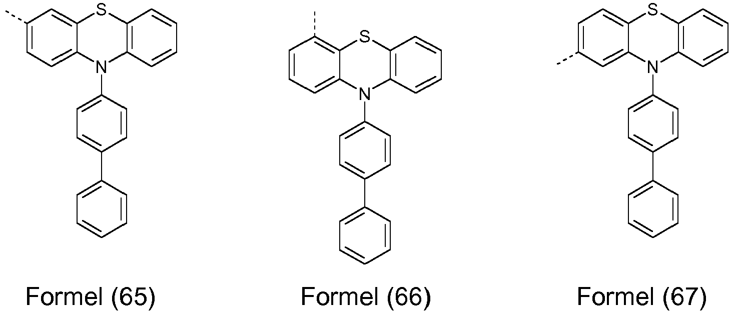 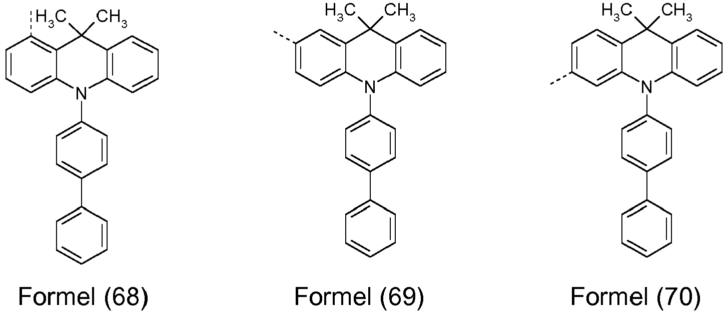 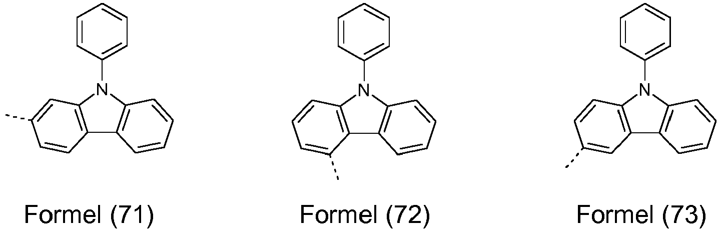 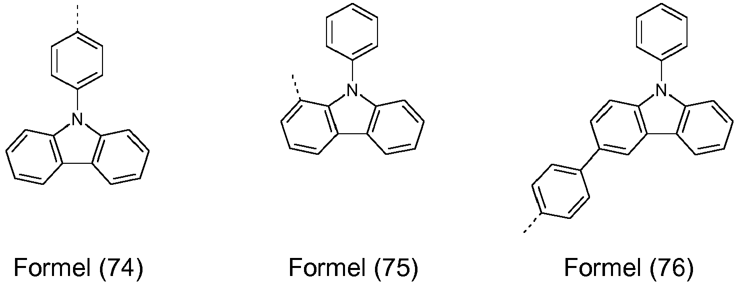 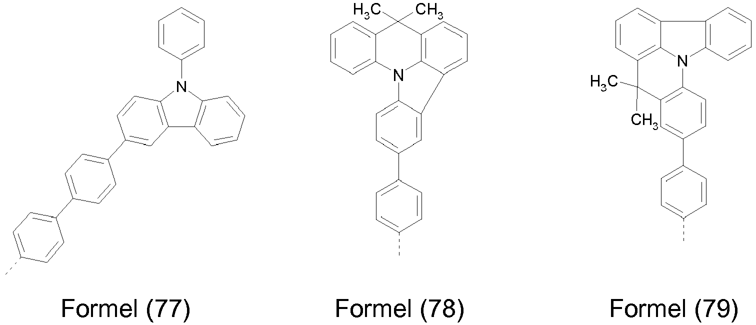 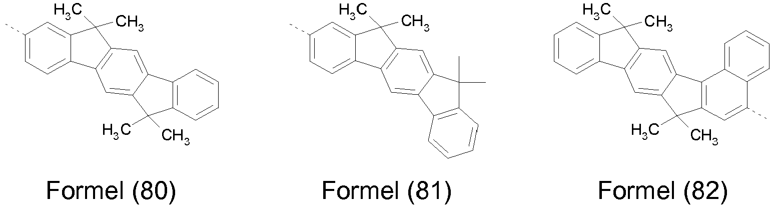 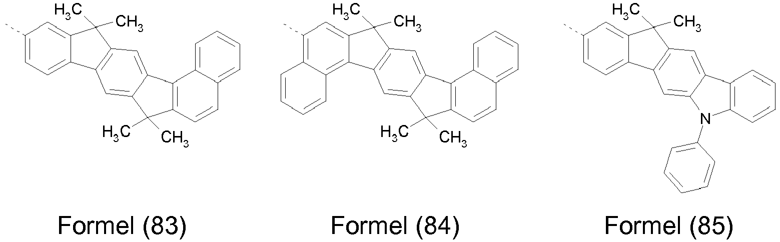 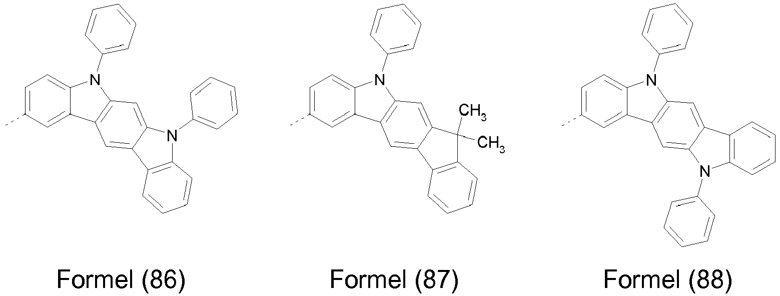 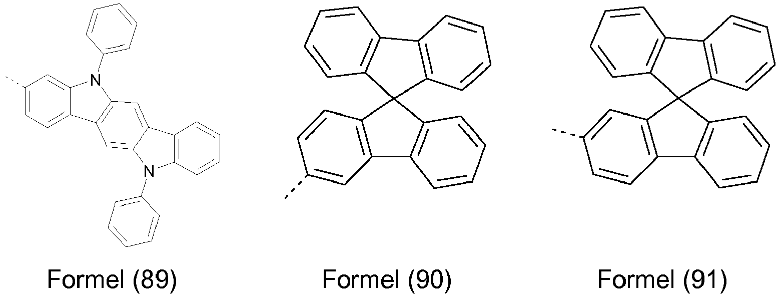 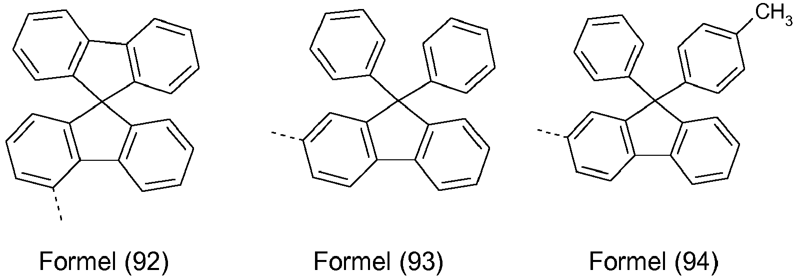 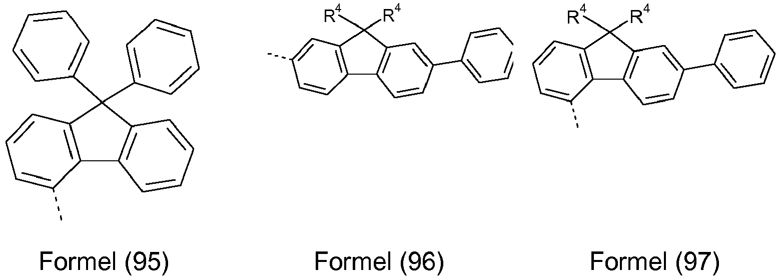 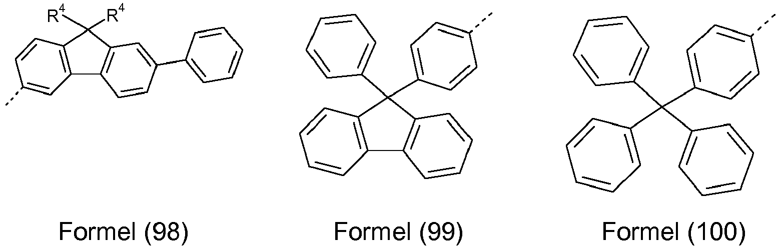 wobei die gestrichelte Linie die Bindungsposition darstellt und wobei die Strukturen mit einem oder mehreren Resten R⁴ substituiert sein können, und R⁴ definiert ist wie oben angegeben.

Die Gruppe L kann auch ein aromatisches Ringsystem sein, das ausgewählt ist aus der Gruppe bestehend aus Biphenylenen, Terphenylenen und den Verbindungen der folgenden Formel (101a) und (101b) 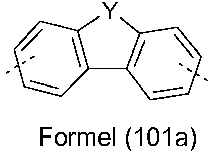 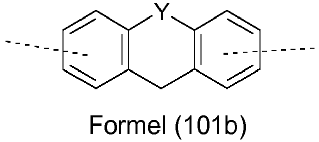 wobei Y gleich C(R²)₂, NR², O, Si(R²)₂ und S, bevorzugt C(R²)₂, NR², O und S, ganz bevorzugt C(R²)₂, NR² und O und insbesondere bevorzugt C(R²)₂ und NR² ist und wobei R² wie oben angegeben definiert ist.

Erfindungsgemäß ist L eine Einfachbindung, d.h., das Stickstoffatom ist direkt über eine Einfachbindung an das Phenanthren in Position 3 gebunden.

In einer insbesondere bevorzugten Ausführungsform betrifft die vorliegende Erfindungen eine Verbindung der Formel (1), dadurch charakterisiert, dass sie lediglich eine Amingruppe enthält, so dass es sich bei den Verbindungen nach Formel (1) folglich um Monoamine handelt.

Beispiele für erfindungsgemäße und nicht-erfindungsgemäße Verbindungen sind in der folgenden Tabelle wiedergegeben.

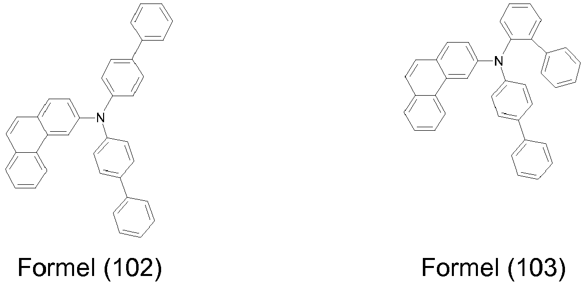

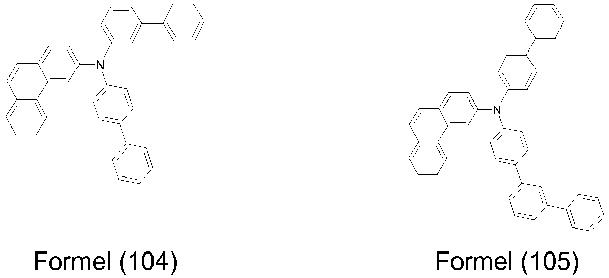

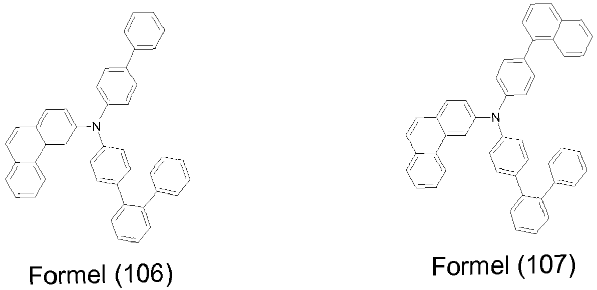

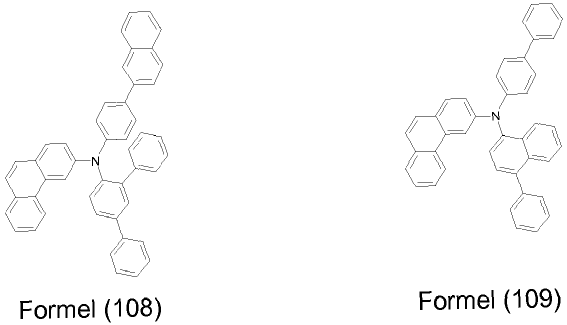

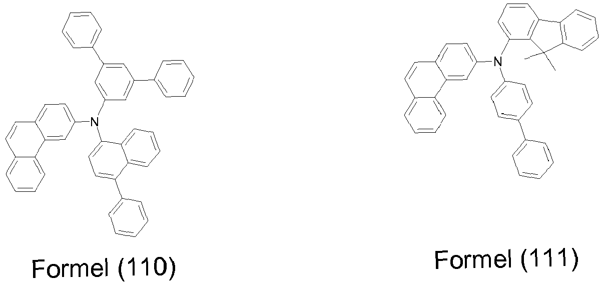

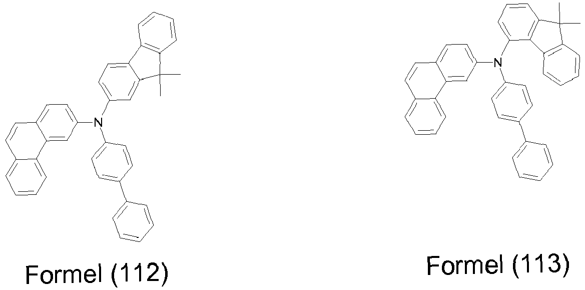

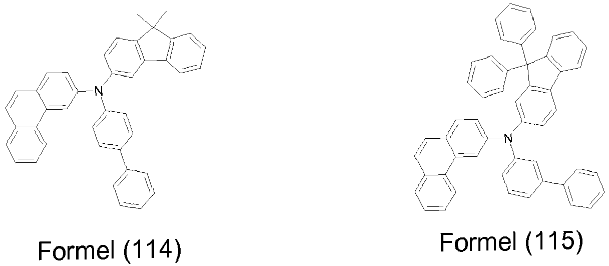

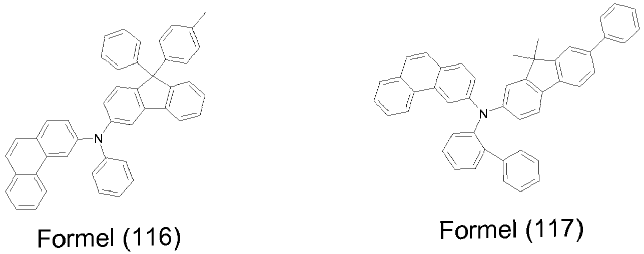

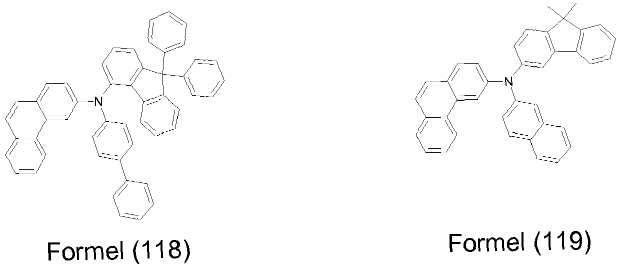

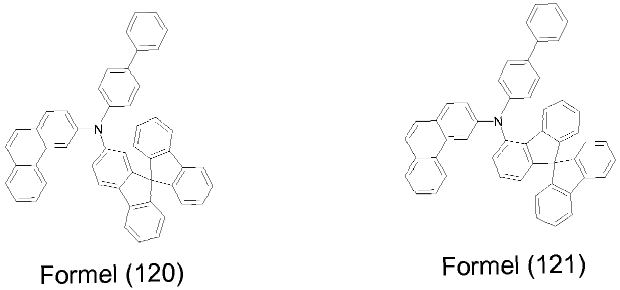

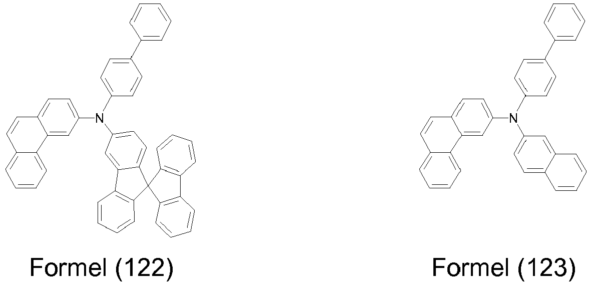

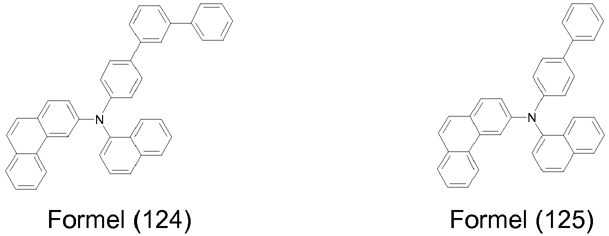

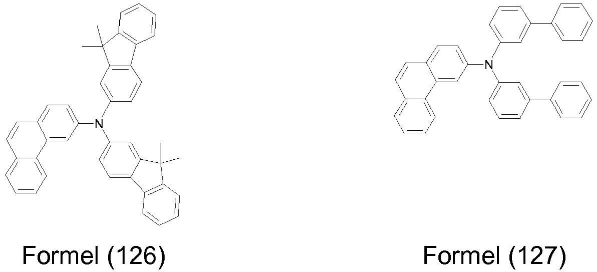

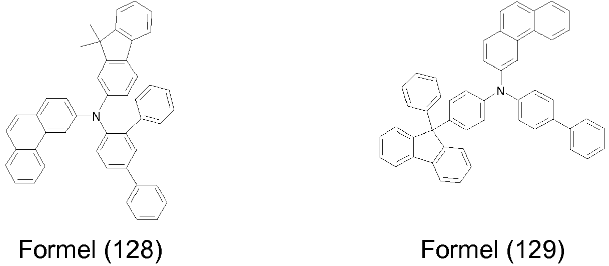

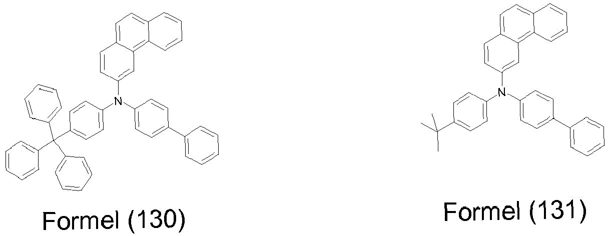

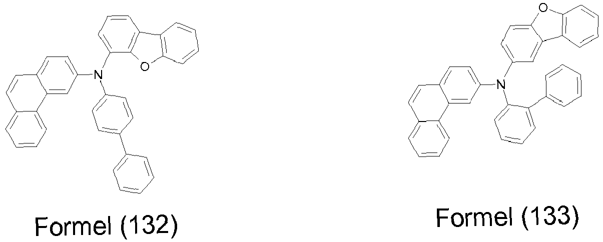

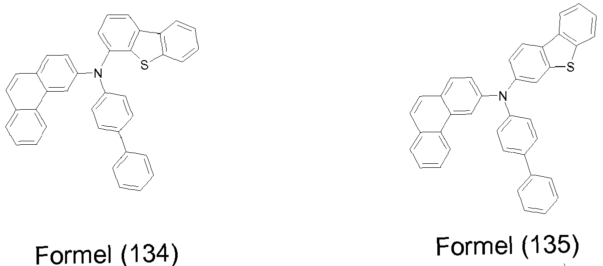

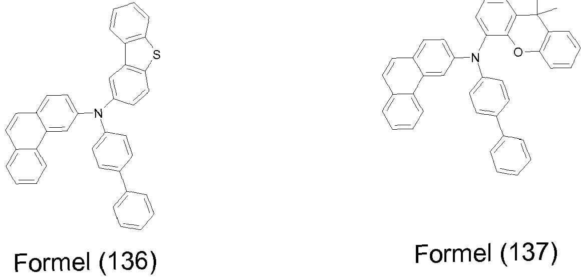

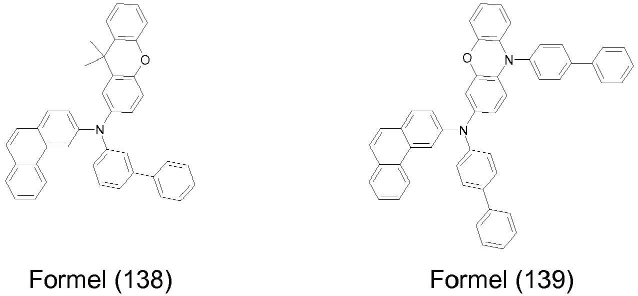

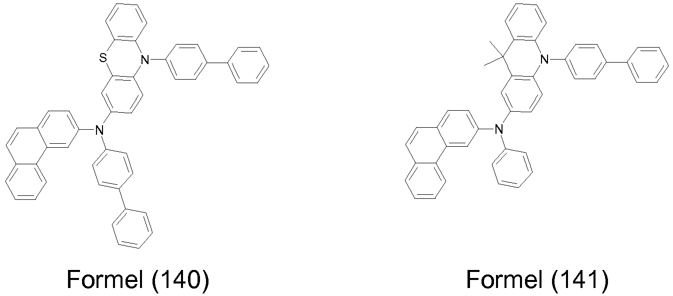

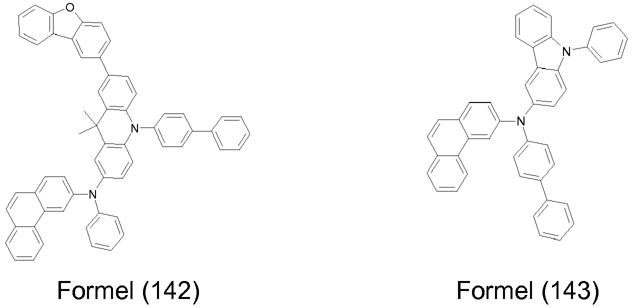

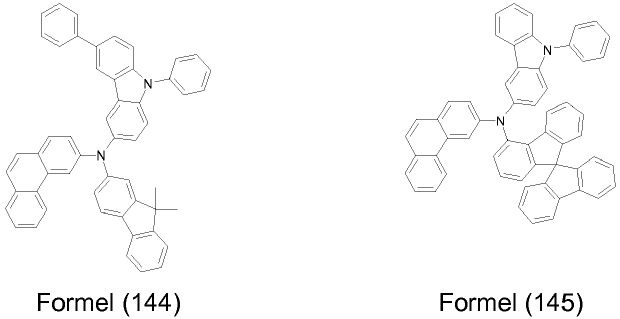

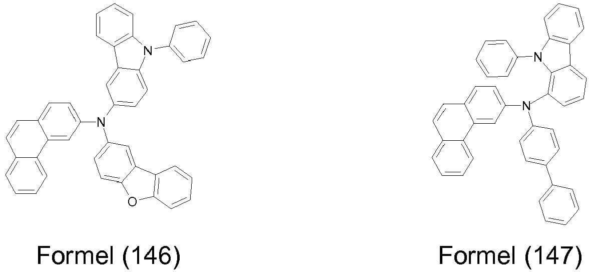

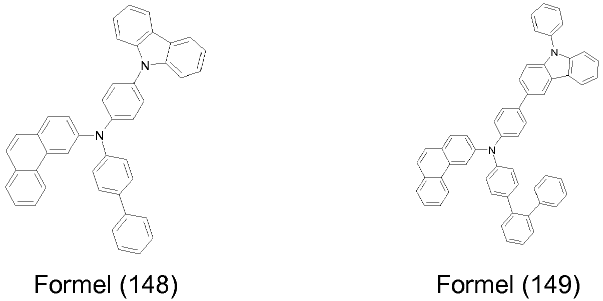

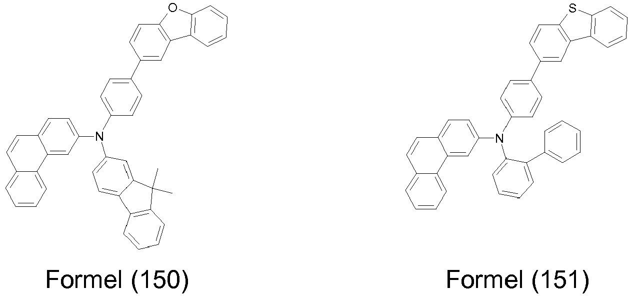

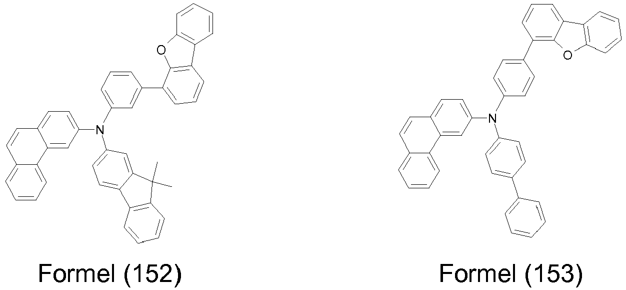

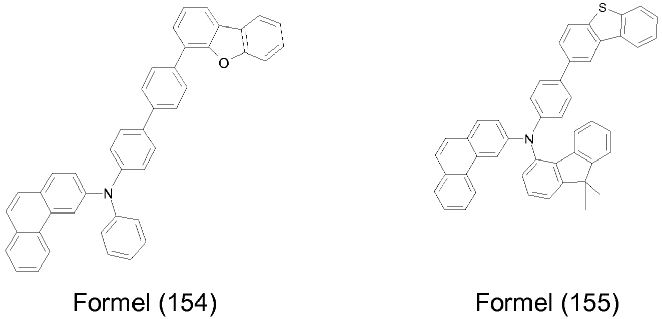

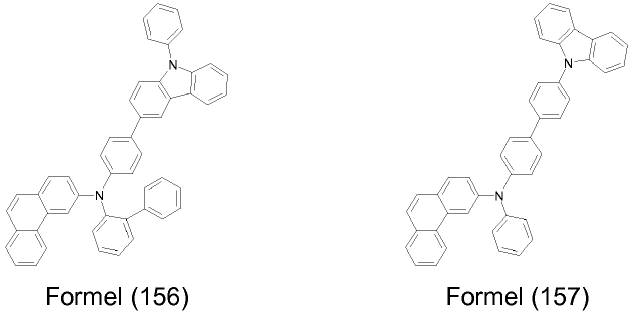

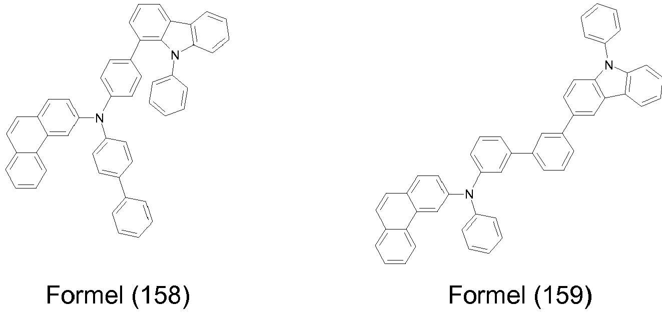

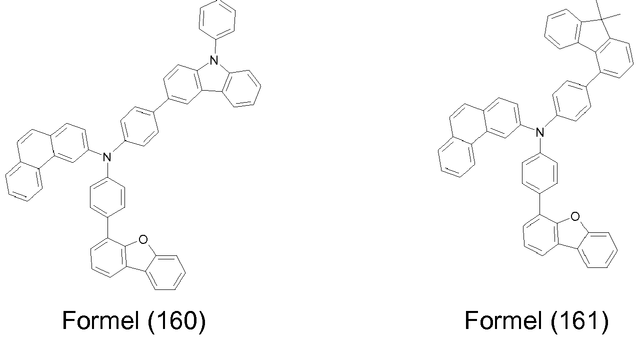

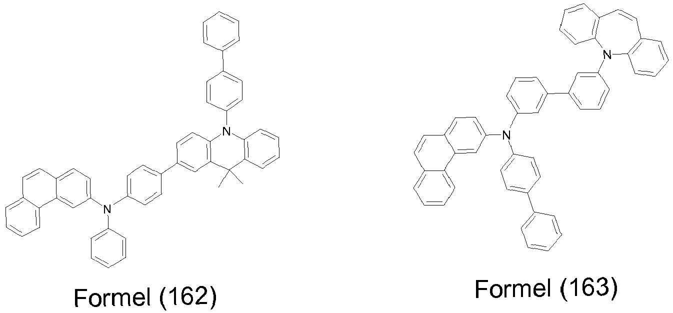

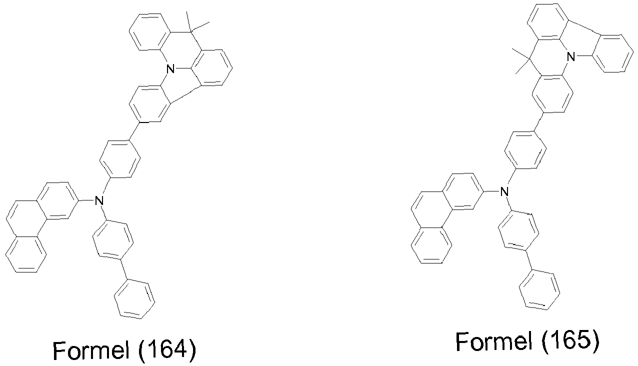

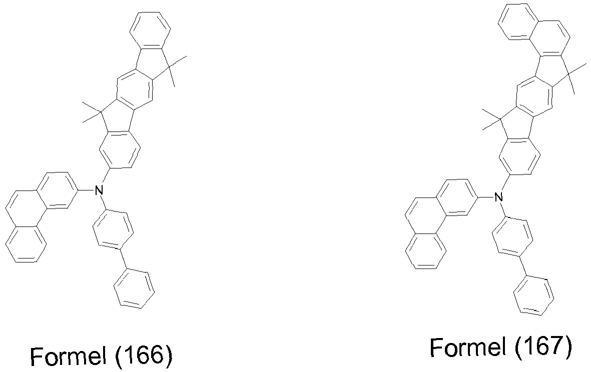

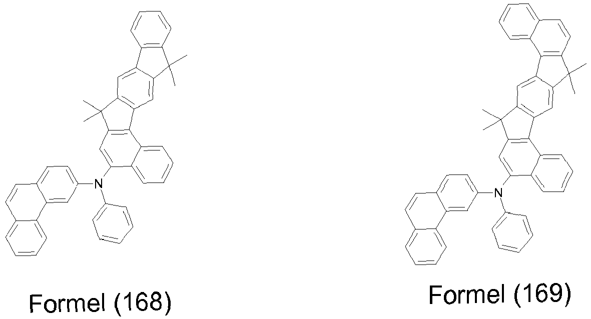

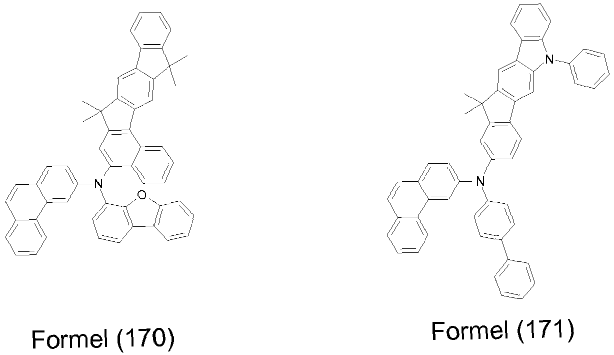

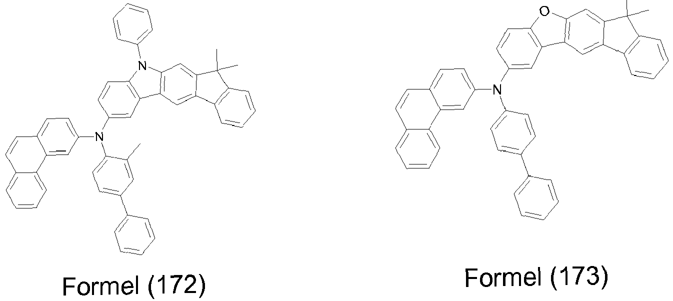

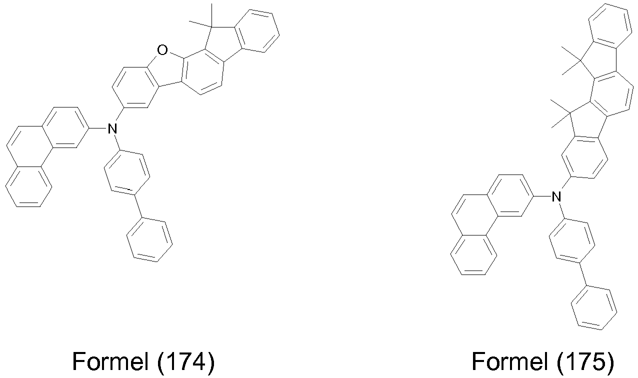

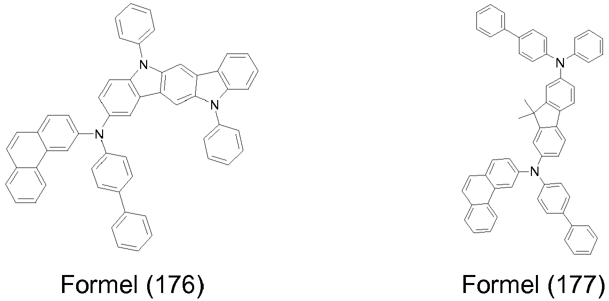

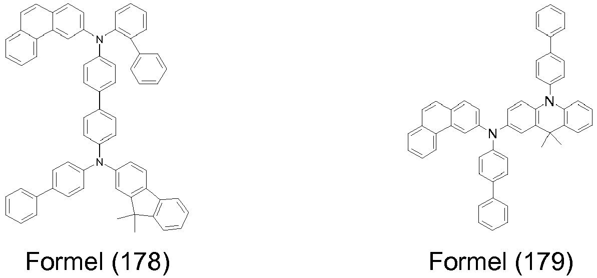

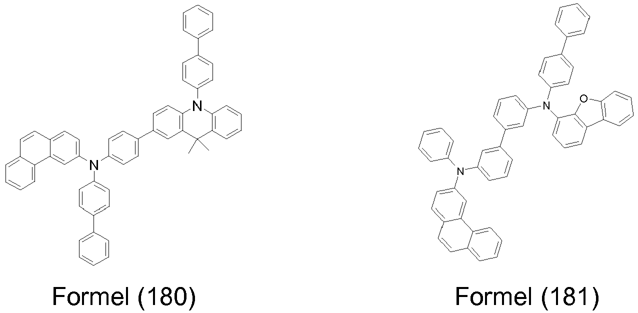

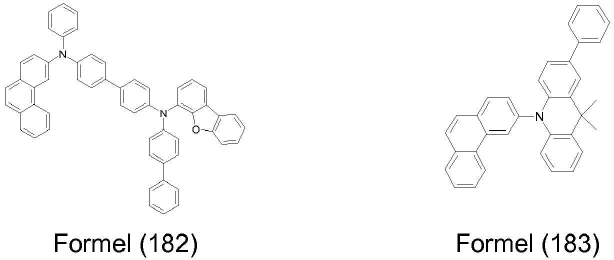

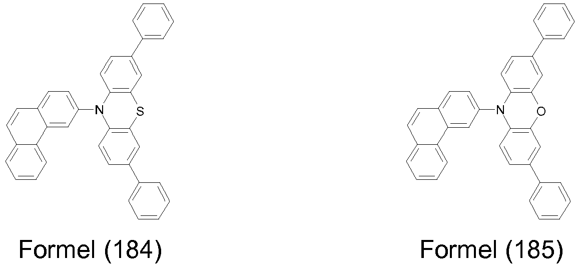

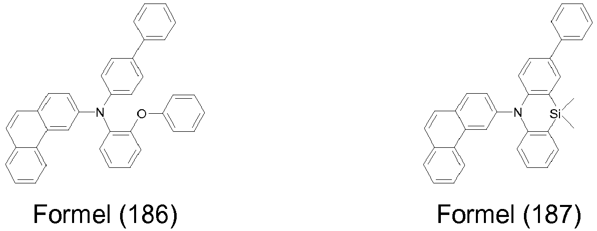

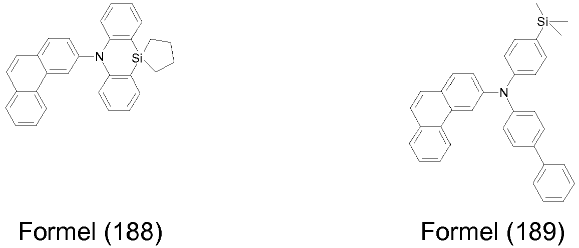

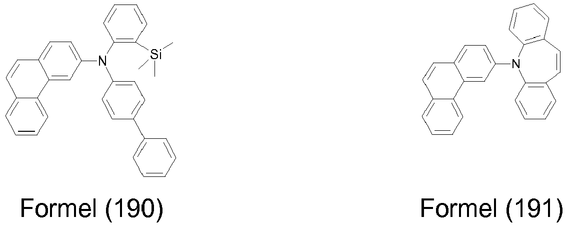

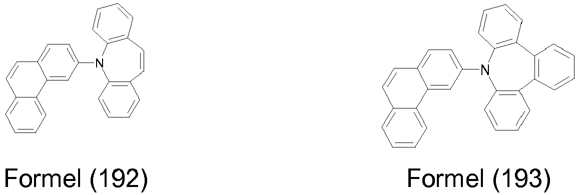

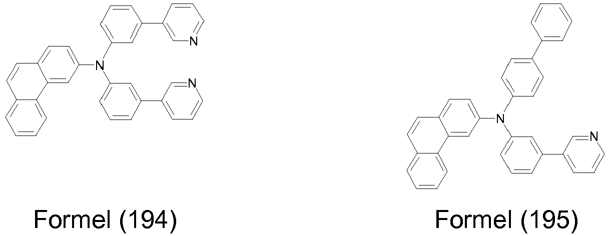

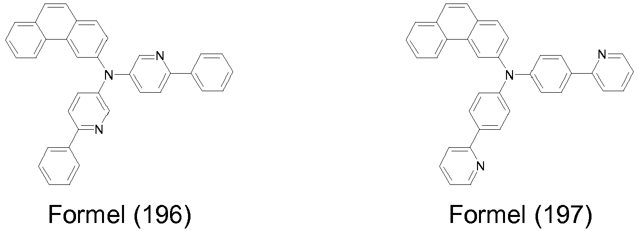

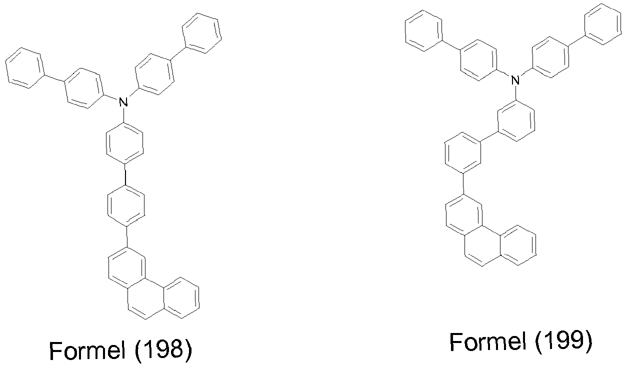

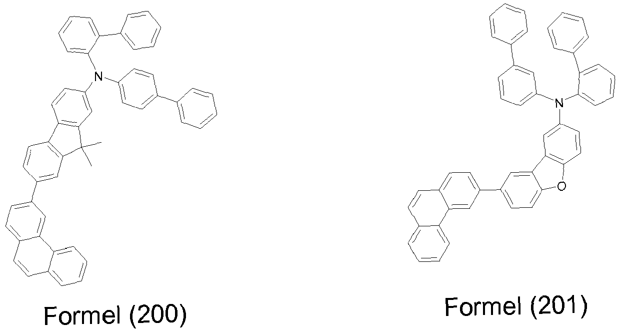

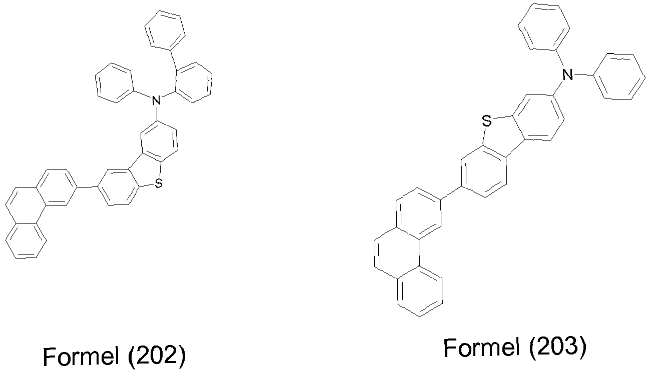

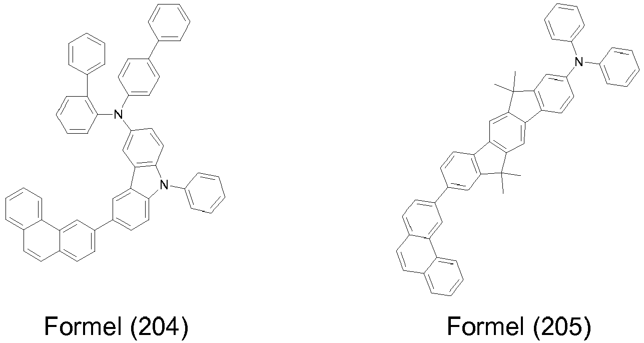

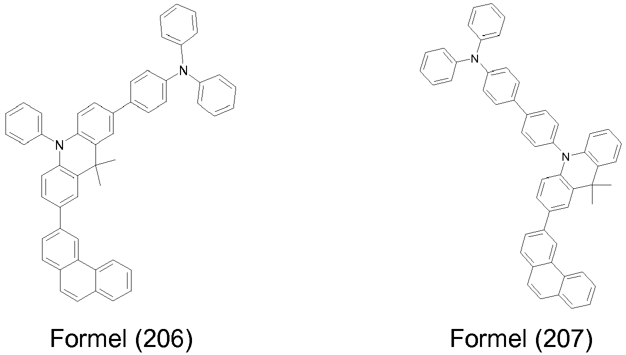

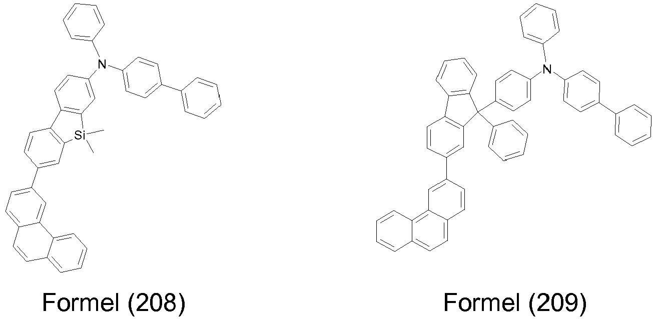

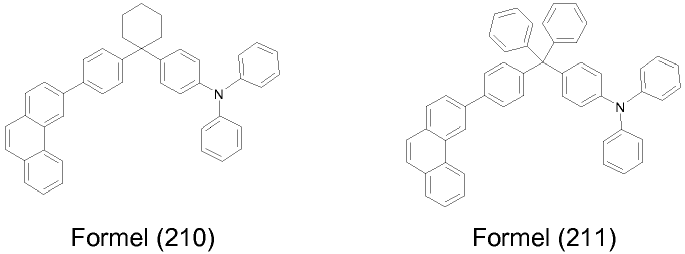

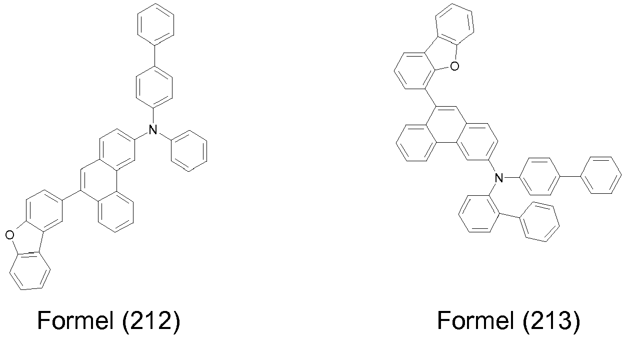

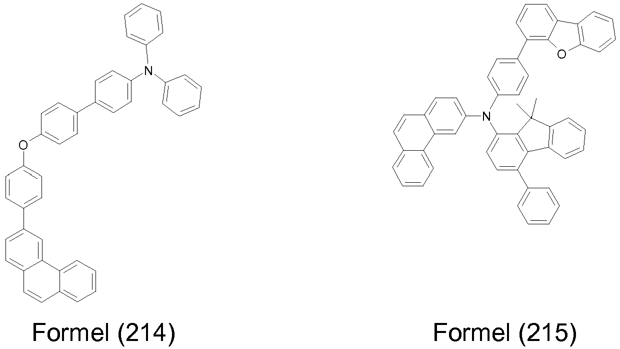

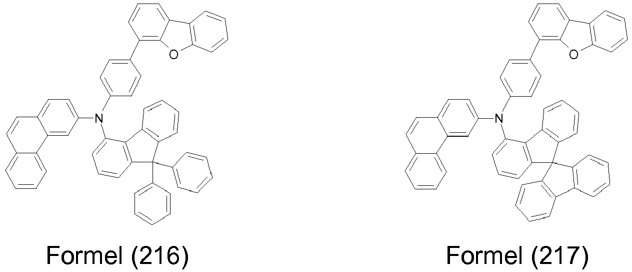

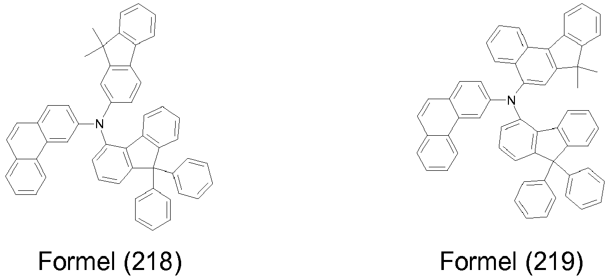

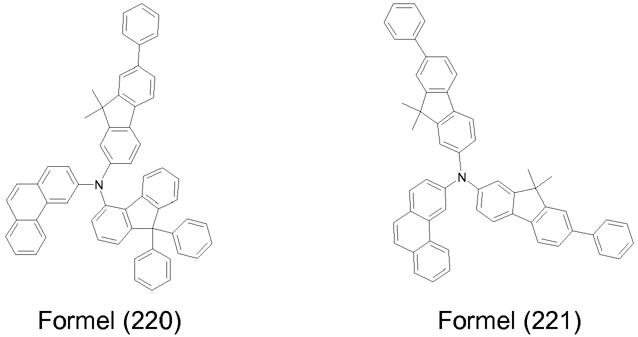

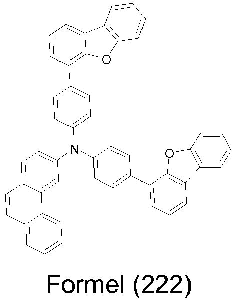

Die Synthese der erfindungsgemäßen Verbindungen kann gemäß im Stand der Technik bekannten Verfahren und Reaktionstypen, beispielsweise Halogenierung, Buchwald-Kupplung und Suzuki-Kupplung erfolgen.

Ein bevorzugtes Verfahren zur Herstellung der erfindungsgemäßen Verbindungen geht von den in Schema 1 als Edukte abgebildeten Grundstrukturen aus. Diese sind in einigen Fällen kommerziell erhältlich, in anderen Fällen können sie in wenigen Syntheseschritten aus einfachen, kommerziell erhältlichen Verbindungen hergestellt werden.

Das nachfolgende Schema 1 zeigt einen bevorzugten Syntheseweg zur Herstellung der erfindungsgemäßen Verbindungen. Zur Synthese der erfindungsgemäßen Verbindungen wird an das Phenanthren-Verbindung A in einer Buchwald-Kupplung mit einem Amin B der Formel Ar²-NH-Ar¹ umgesetzt

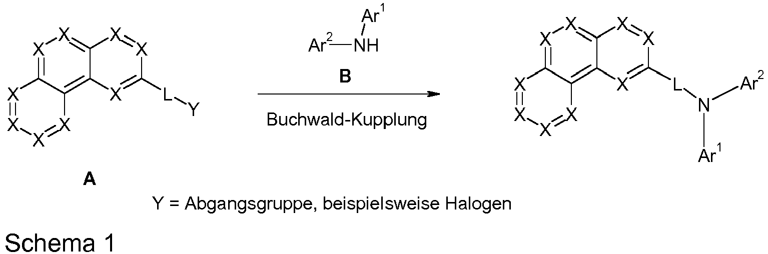

Ein anderer bevorzugter Syntheseweg zur Herstellung der erfindungsgemäßen Verbindungen wird im Schema 2 dargestellt. Der Syntheseweg umfasst zwei Kupplungsreaktionen: zunächst wird das das Phenanthren-Verbindung **A** in einer ersten Buchwald-Kupplung mit einem Amin C der Formel Ar²-NH₂ umgesetzt. Schließlich erfolgt eine zweite Buchwald-Kupplung mit einer Verbindung **D,** beispielsweise mit einer Bromaryl-Verbindung.

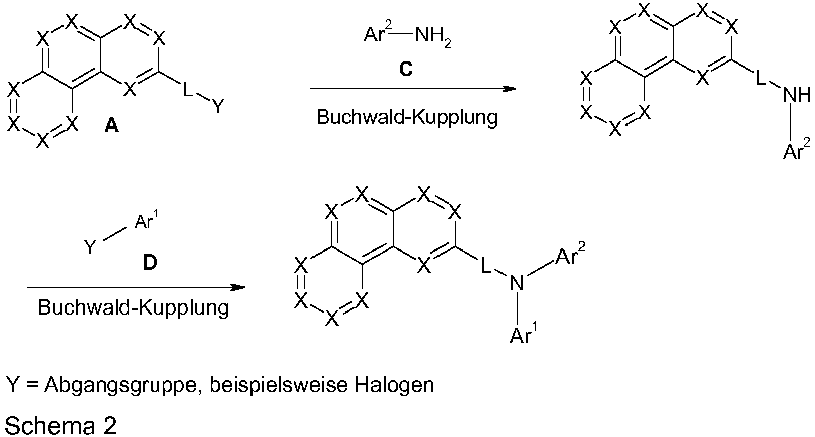

Die Kupplungsreaktionen stellen dabei bevorzugt Buchwald-Kupplungen dar.

Die Synthese der Ausgangsverbindungen (**A**) bereitet dem Fachmann keine Schwierigkeiten. Sie können, beispielsweise, durch Umsetzung von Acetyl-Verbindungen in Amine und anschließende Umsetzung in Halogenide mittels Sandmayer Reaktion hergestellt werden. Beispiele hierzu sind weiter unten offenbart.

Die oben beschriebenen erfindungsgemäßen Verbindungen, insbesondere Verbindungen, welche mit reaktiven Abgangsgruppen, wie Brom, Iod, Chlor, Boronsäure oder Boronsäureester, substituiert sind, können als Monomere zur Erzeugung entsprechender Oligomere, Dendrimere oder Polymere Verwendung finden. Die Oligomerisation bzw. Polymerisation erfolgt dabei bevorzugt über die Halogenfunktionalität bzw. die Boronsäurefunktionalität.

Einen weiteren Gegenstand der vorliegenden Erfindung stellt also ein Verfahren zur Herstellung von Verbindungen gemäß Formel (1) dar, welches dadurch gekennzeichnet ist, dass das Verfahren entweder nach Schema 1 oder nach Schema 2 erfolgt.

Die oben beschriebenen erfindungsgemäßen Verbindungen, insbesondere Verbindungen, welche mit reaktiven Abgangsgruppen, wie Brom, Iod, Chlor, Boronsäure oder Boronsäureester, substituiert sind, können als Monomere zur Erzeugung entsprechender Oligomere, Dendrimere oder Polymere Verwendung finden. Geeignete reaktive Abgangsgruppen sind beispielsweise Brom, Iod, Chlor, Boronsäuren, Boronsäureester, Amine, Alkenyl- oder Alkinylgruppen mit endständiger C-C-Doppelbindung bzw. C-C-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weiterer Gegenstand der Erfindung sind daher Oligomere, Polymere oder Dendrimere enthaltend eine oder mehrere Verbindungen gemäß Formel (1), wobei die Bindung(en) zum Polymer, Oligomer oder Dendrimer an beliebigen, in Formel (1) mit R¹ oder R² substituierten Positionen lokalisiert sein können. Je nach Verknüpfung der Verbindung gemäß Formel (1) ist die Verbindung Bestandteil einer Seitenkette des Oligomers oder Polymers oder Bestandteil der Hauptkette. Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist. Die erfindungsgemäßen Polymere, Oligomere oder Dendrimere können konjugiert, teilkonjugiert oder nichtkonjugiert sein. Die erfindungsgemäßen Oligomere oder Polymere können linear, verzweigt oder dendritisch sein. In den linear verknüpften Strukturen können die Einheiten gemäß Formel (1) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten und dendritischen Strukturen können beispielsweise drei oder mehrere Einheiten gemäß Formel (1) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein.

Für die Wiederholeinheiten gemäß Formel (1) in Oligomeren, Dendrimeren und Polymeren gelten dieselben Bevorzugungen wie oben für Verbindungen gemäß Formel (1) beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen (z. B. gemäß EP 842208 oder WO 2000/22026), Spirobifluorenen (z. B. gemäß EP 707020, EP 894107 oder WO 2006/061181), Paraphenylenen (z. B. gemäß WO 1992/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689 oder WO 2007/006383), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 2007/068325) oder phosphoreszierende Metallkomplexe (z. B. gemäß WO 2006/003000), und/oder Ladungstransporteinheiten, insbesondere solche basierend auf Triarylaminen.

Die erfindungsgemäßen Polymere, Oligomere und Dendrimere weisen vorteilhafte Eigenschaften, insbesondere hohe Lebensdauern, hohe Effizienzen und gute Farbkoordinaten auf.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (1) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind folgende:
(A) SUZUKI-Polymerisation;
(B) YAMAMOTO-Polymerisation;
(C) STILLE-Polymerisation; und
(D) HARTWIG-BUCHWALD-Polymerisation.

Wie die Polymerisation nach diesen Methoden durchgeführt werden kann und wie die Polymere dann vom Reaktionsmedium abgetrennt und aufgereinigt werden können, ist dem Fachmann bekannt und in der Literatur, beispielsweise in WO 2003/048225, WO 2004/037887 und WO 2004/037887, im Detail beschrieben.

Gegenstand der vorliegenden Erfindung ist somit auch ein Verfahren zur Herstellung der erfindungsgemäßen Polymere, Oligomere und Dendrimere, das dadurch gekennzeichnet ist, dass sie durch Polymerisation gemäß SUZUKI, Polymerisation gemäß YAMAMOTO, Polymerisation gemäß STILLE oder Polymerisation gemäß HARTWIG-BUCHWALD hergestellt werden. Die erfindungsgemäßen Dendrimere können gemäß dem Fachmann bekannten Verfahren oder in Analogie dazu hergestellt werden. Geeignete Verfahren sind in der Literatur beschrieben, wie z. B. in Frechet, Jean M. J.; Hawker, Craig J., "Hyperbranched polyphenylene and hyperbranched polyesters: new soluble, three-dimensional, reactive polymers", Reactive & Functional Polymers (1995), 26(1-3), 127-36; Janssen, H. M.; Meijer, E. W., "The synthesis and characterization of dendritic molecules", Materials Science and Technology (1999), 20 (Synthesis of Polymers), 403-458; Tomalia, Donald A., "Dendrimer molecules", Scientific American (1995), 272(5), 62-6; WO 2002/067343 A1 und WO 2005/026144 A1.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Miniemulsionen sein. Es kann bevorzugt sein, hierfür Mischungen aus zwei oder mehr Lösemitteln zu verwenden. Geeignete und bevorzugte Lösemittel sind beispielsweise Toluol, Phenoxytoluol, Anisol, o-, m- oder p-Xylol, Methylbenzoat, Dimethylanisol, Mesitylen, Tetralin, Veratrol, THF, Methyl-THF, THP, Chlorbenzol, Dioxan oder Mischungen dieser Lösemittel.

Gegenstand der Erfindung ist daher weiterhin eine Formulierung, insbesondere eine Lösung, Dispersion oder Miniemulsion, enthaltend mindestens eine Verbindung gemäß Formel (1) oder mindestens ein Polymer, Oligomer oder Dendrimer enthaltend mindestens eine Einheit gemäß Formel (1) sowie mindestens ein Lösungsmittel, bevorzugt ein organisches Lösungsmittel. Wie solche Lösungen hergestellt werden können, ist dem Fachmann bekannt und beispielsweise in WO 2002/072714, WO 2003/019694 und der darin zitierten Literatur beschrieben.

Die erfindungsgemäßen Verbindungen können mit anderen organisch funktionellen Materialien, die in elektronischen Vorrichtungen verwendet werden, als Zusammensetzungen eingesetzt werden. Dem Fachmann sind hierbei eine Vielzahl möglicher organisch funktioneller Materialien (oft auch organische Halbleiter genannt) bekannt. Die vorliegende Erfindung betrifft daher auch eine Zusammensetzung enthaltend eine oder meherer erfindungsgemäße Verbindung und wenigstens ein weiteres organisch funktionelles Material ausgewählt aus der Gruppe bestehend aus fluoreszierenden Emitter, phosphoreszierenden Emitter, Host Materialien, Matrix Materialien, Elektronentransportmaterialien, Elektroneninjektionsmaterialien, Lochleitermaterialien, Lochinjektionsmaterialien, Elektronenblockiermaterialien und Lochblockiermaterialien.

Die erfindungsgemäßen Verbindungen eignen sich für den Einsatz in elektronischen Vorrichtungen, insbesondere in organischen Elektrolumineszenzvorrichtungen (OLEDs). Abhängig von der Substitution werden die Verbindungen in unterschiedlichen Funktionen und Schichten eingesetzt.

Weitere Gegenstände der Erfindung sind daher die Verwendung der Verbindungen gemäß Formel (1) in elektronischen Vorrichtungen sowie elektronische Vorrichtungen selbst, welche eine oder mehrere Verbindungen gemäß Formel (1) enthalten. Dabei sind die elektronischen Vorrichtungen bevorzugt ausgewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und besonders bevorzugt organischen Elektrolumineszenzvorrichtungen (OLEDs).

Gegenstand der Erfindung sind, wie bereits oben ausgeführt, elektronische Vorrichtungen, enthaltend mindestens eine Verbindung gemäß Formel (1). Dabei sind die elektronischen Vorrichtungen bevorzugt ausgewählt aus den oben genannten Vorrichtungen. Besonders bevorzugt sind organische Elektrolumineszenzvorrichtungen (OLEDs), enthaltend Anode, Kathode und mindestens eine emittierende Schicht, dadurch gekennzeichnet, dass mindestens eine organische Schicht, die eine emittierende Schicht, eine Lochtransportschicht oder eine andere Schicht sein kann, mindestens eine Verbindung gemäß Formel (1) enthält.

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer) und/oder organischen oder anorganischen p/n-Übergängen. Es sei aber darauf hingewiesen, dass nicht notwendigerweise jede dieser Schichten vorhanden sein muss und die Wahl der Schichten immer von den verwendeten Verbindungen abhängt und insbesondere auch von der Tatsache, ob es sich um eine fluoreszierende oder phosphoreszierende Elektrolumineszenzvorrichtung handelt.

Die erfindungsgemäße organische Elektrolumineszenzvorrichtung kann mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues oder gelbes oder orangefarbenes oder rotes Licht emittieren. Insbesondere bevorzugt sind Dreischichtsysteme, also Systeme mit drei emittierenden Schichten, wobei die drei Schichten blaue, grüne und orange oder rote Emission zeigen (für den prinzipiellen Aufbau siehe z. B. WO 2005/011013). Die erfindungsgemäßen Verbindungen können in solchen Vorrichtungen in einer Lochtransportschicht, einer emittierenden Schicht und/oder in einer anderen Schicht vorhanden sein. Es soll angemerkt werden, dass sich für die Erzeugung von weißem Licht anstelle mehrerer farbig emittierender Emitterverbindungen auch eine einzeln verwendete Emitterverbindung eignen kann, welche in einem breiten Wellenlängenbereich emittiert.

Es ist erfindungsgemäß bevorzugt, wenn die Verbindung gemäß Formel (1) in einer elektronischen Vorrichtung enthaltend einen oder mehrere phosphoreszierende Dotanden eingesetzt wird. Dabei kann die Verbindung in unterschiedlichen Schichten, bevorzugt in einer Lochtransportschicht, einer Lochinjektionsschicht oder in einer emittierenden Schicht, verwendet werden. Die Verbindung gemäß Formel (1) kann aber auch erfindungsgemäß in einer elektronischen Vorrichtung enthaltend einen oder mehrere fluoreszierende Dotanden eingesetzt werden.

Wird die Verbindung gemäß Formel (1) als Lochtransportmaterial in einer Lochtransportschicht, einer Lochinjektionsschicht oder einer Elektronenblockierschicht eingesetzt, so kann die Verbindung als Reinmaterial, d.h. in einem Anteil von 100 %, in der Lochtransportschicht eingesetzt werden, oder sie kann in Kombination mit einer oder mehreren weiteren Verbindungen eingesetzt werden. Gemäß einer bevorzugten Ausführungsform enthält die organische Schicht enthaltend die Verbindung der Formel (1) dann zusätzlich einen oder mehrere p-Dotanden. Als p-Dotanden werden gemäß der vorliegenden Erfindung bevorzugt solche organischen Elektronenakzeptorverbindungen eingesetzt, die eine oder mehrere der anderen Verbindungen der Mischung oxidieren können.

Besonders bevorzugte Ausführungsformen von p-Dotanden sind die in WO 2011/073149, EP 1968131, EP 2276085, EP 2213662, EP 1722602, EP 2045848, DE 102007031220, US 8044390, US 8057712, WO 2009/003455, WO 2010/094378, WO 2011/120709, US 2010/0096600 und WO 2012/095143 offenbarten Verbindungen.

In einer weiteren bevorzugten Ausführungsform der Erfindung wird die Verbindung gemäß Formel (1) als Lochtransportmaterial in Kombination mit einem Hexaazatriphenylenderivat, wie in US 2007/0092755 beschrieben, verwendet. Besonders bevorzugt wird das Hexaazatriphenylenderivat dabei in einer separaten Schicht eingesetzt. Besonders bevorzugt als p-Dotanden sind Chinodimethanverbindungen, Azaindenofluorendione, Azaphenalene, Azatriphenylene, I₂, Metallhalogenide, bevorzugt Übergangsmetallhalogenide, Metalloxide, bevorzugt Metalloxide enthaltend mindestens ein Übergangsmetall oder ein Metall der 3. Hauptgruppe, und Übergangsmetallkomplexe, bevorzugt Komplexe von Cu, Co, Ni, Pd und Pt mit Liganden enthaltend mindestens ein Sauerstoffatom als Bindungsstelle. Bevorzugt sind weiterhin Übergangsmetalloxide als Dotanden, bevorzugt Oxide von Rhenium, Molybdän und Wolfram, besonders bevorzugt Re₂O₇, MoO₃, WO₃ und ReO₃.

Bevorzugt sind als p-Dotanden weiterhin die folgenden Verbindungen:

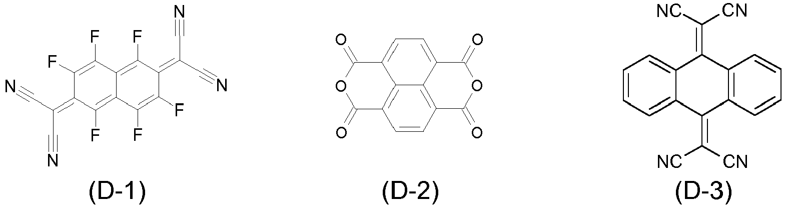

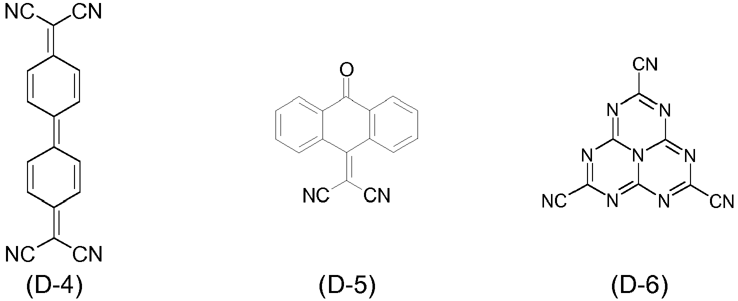

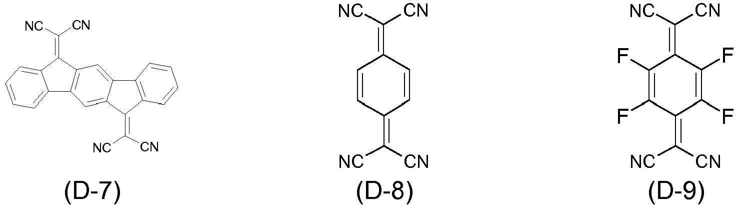

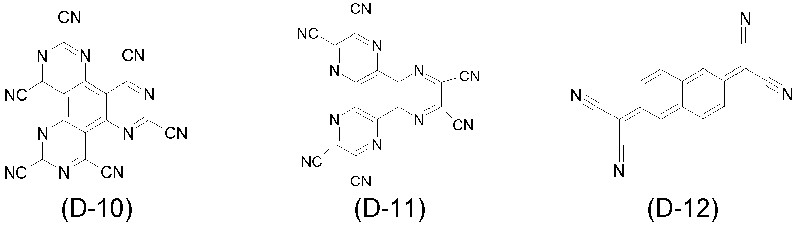

Der p-Dotand in Lochtransportschicht liegt bevorzugt in einer Konzentration von 0.1 bis 20 Vol-%, ganz bevorzugt 0.5 bis 12 Vol-%, besonders bevorzugt 1 bis 8 Vol-% und ganz besonders bevorzugt 2 bis 6 Vol-% vor.

Die Lochtransportschicht weist bevorzugt eine Dicke von 5 bis 50 nm, besonders bevorzugt 10 bis 40 nm, auf.

Vom Begriff phosphoreszierende Dotanden sind typischerweise Verbindungen umfasst, bei denen die Lichtemission durch einen spinverbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Dotanden (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Erfindung alle lumineszierenden Iridium-, Platin- oder Kupferkomplexe als phosphoreszierende Verbindungen angesehen.

Beispiele der oben beschriebenen Emitter können den Anmeldungen WO 00/70655, WO 01/41512, WO 02/02714, WO 02/15645, EP 1191613, EP 1191612, EP 1191614, WO 2005/033244, WO 2005/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind. Auch kann der Fachmann ohne erfinderisches Zutun weitere phosphoreszierende Komplexe in Kombination mit den Verbindungen gemäß Formel (1) in organischen Elektrolumineszenzvorrichtungen einsetzen.

Explizite Beispiele für geeignete phosphoreszierende Emitterverbindungen können weiterhin der folgenden Tabelle entnommen werden.

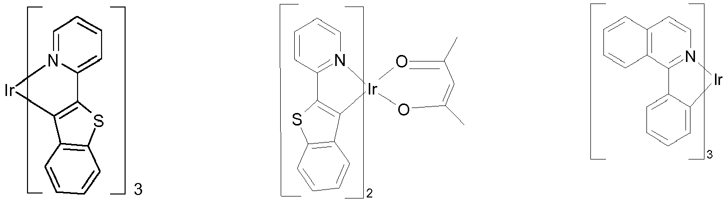

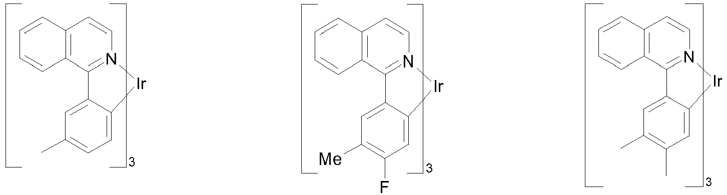

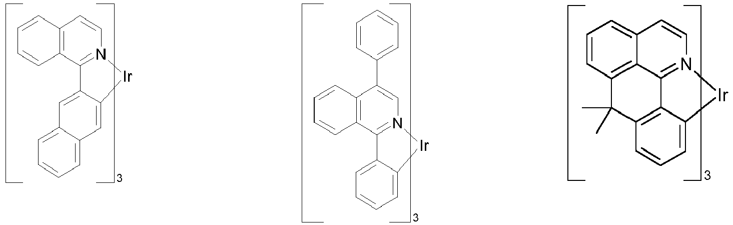

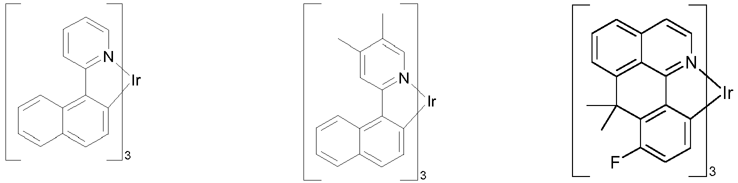

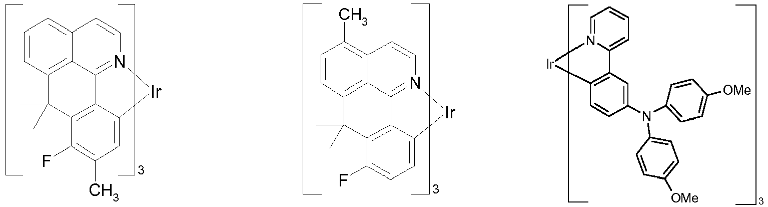

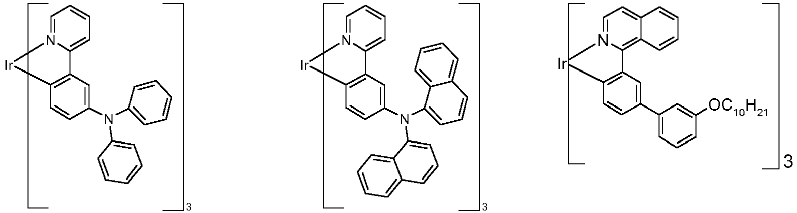

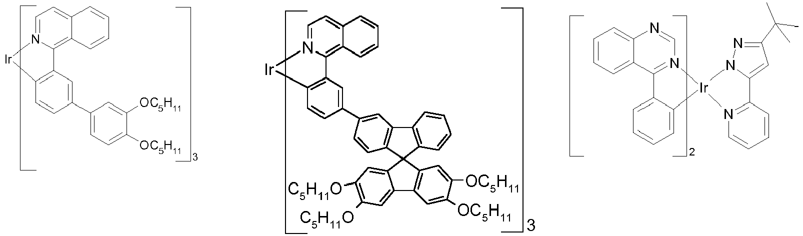

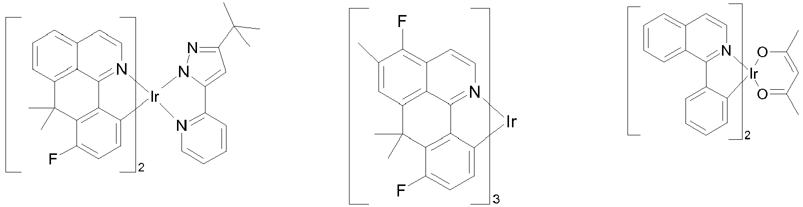

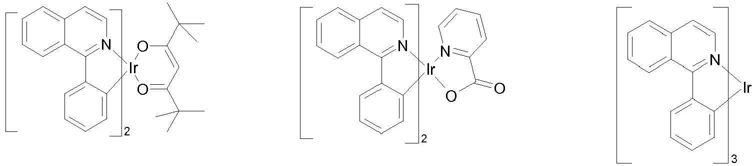

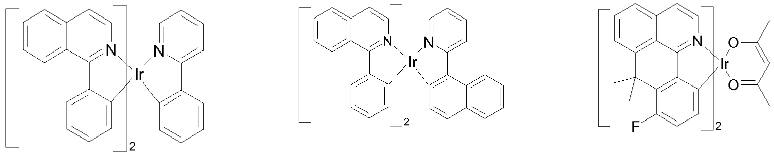

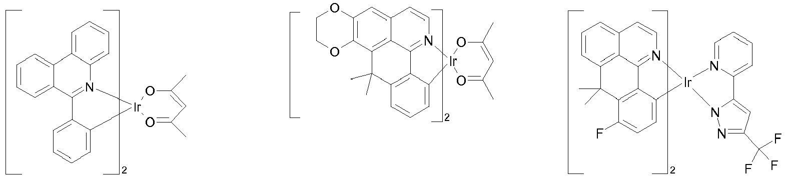

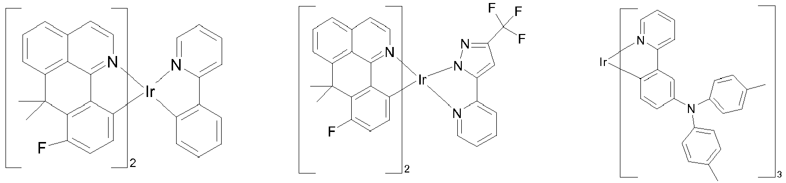

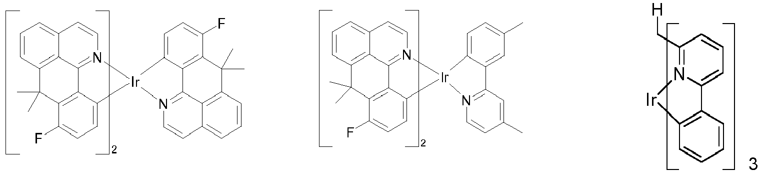

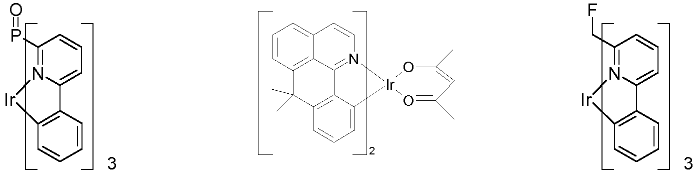

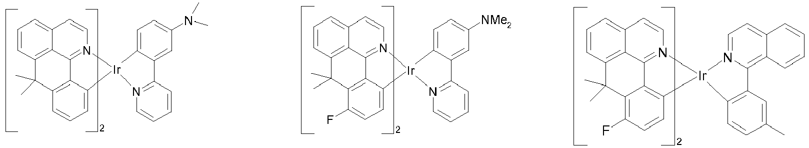

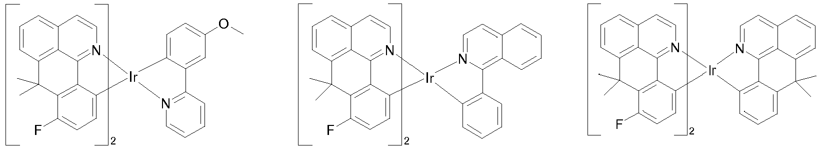

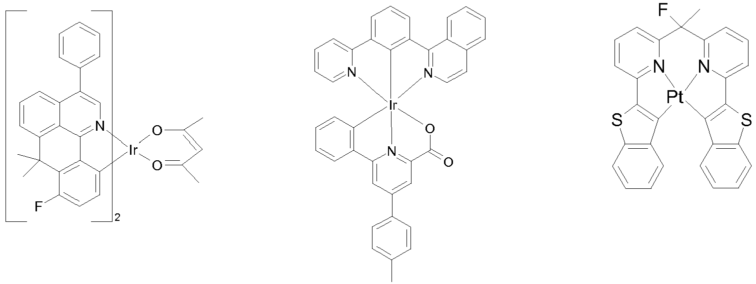

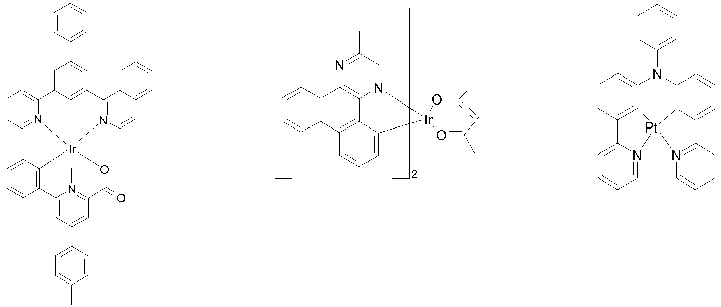

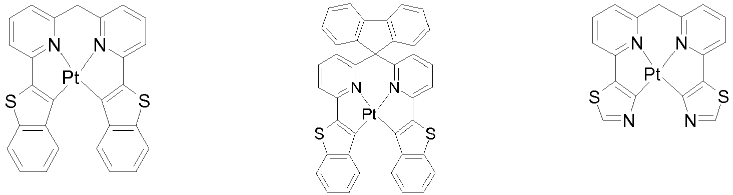

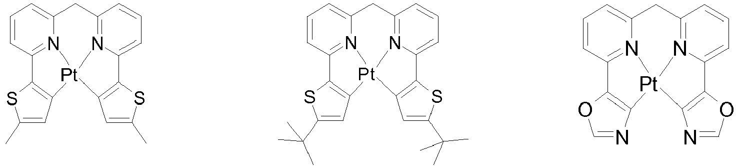

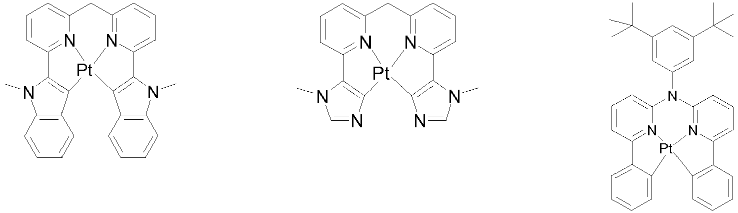

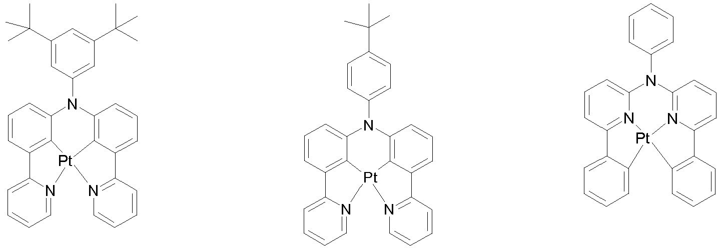

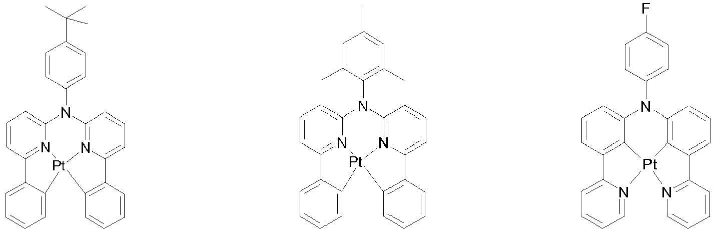

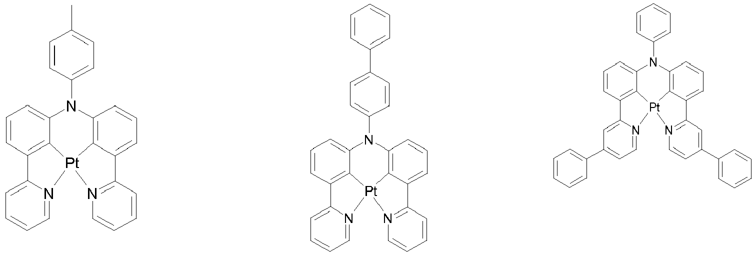

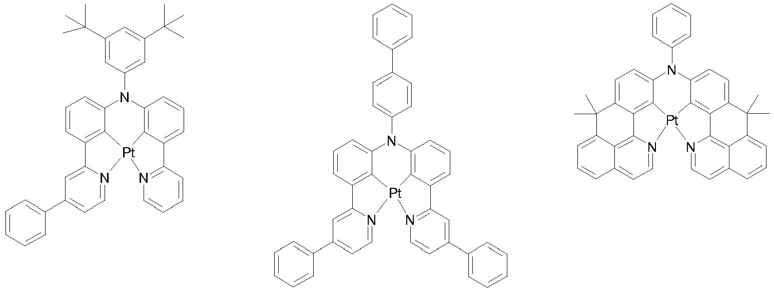

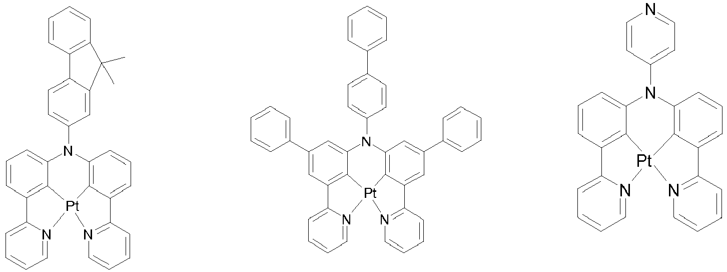

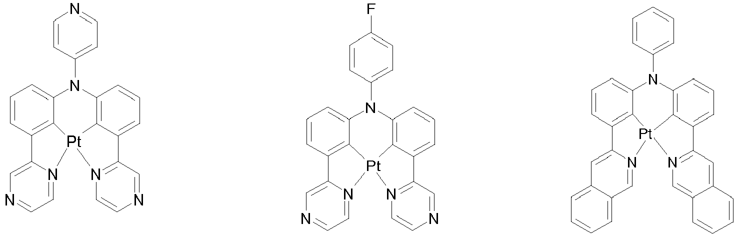

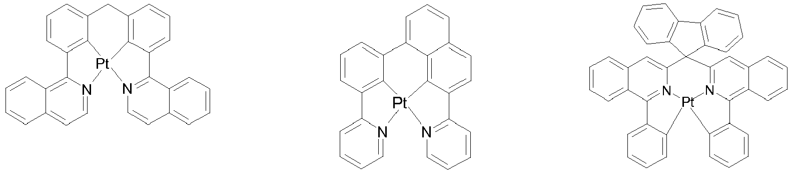

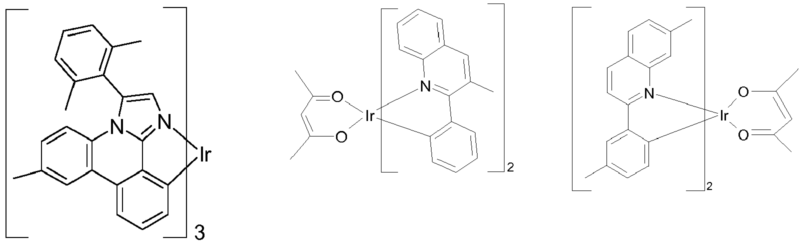

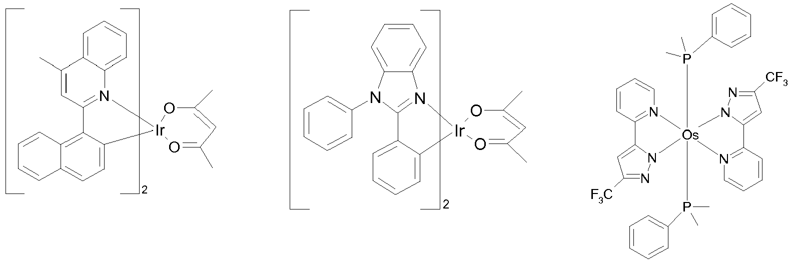

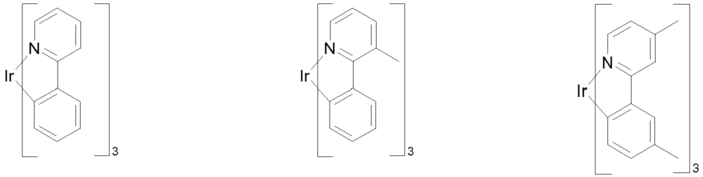

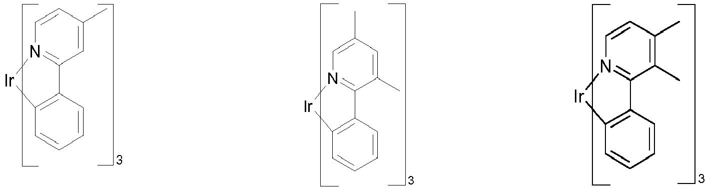

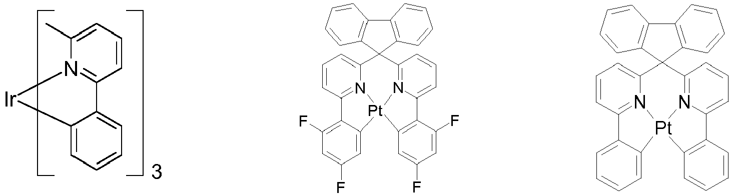

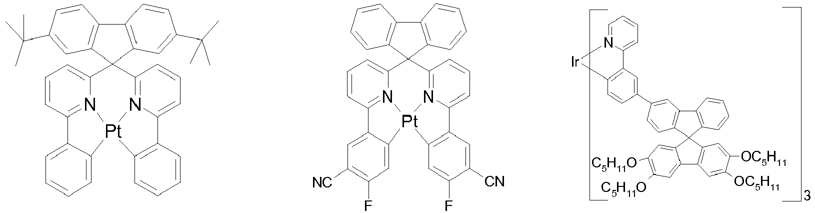

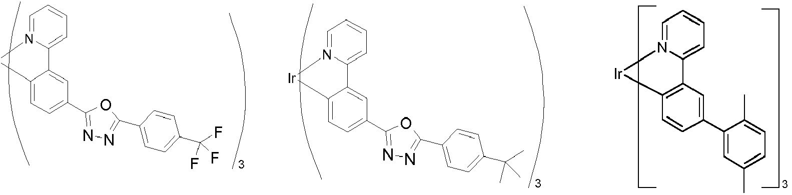

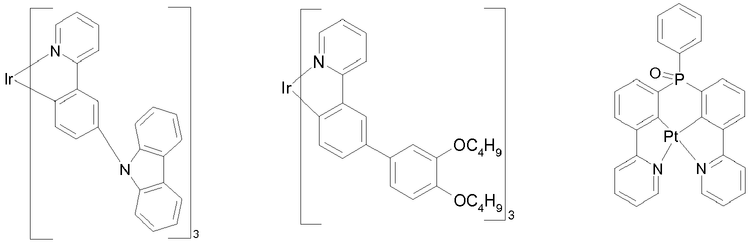

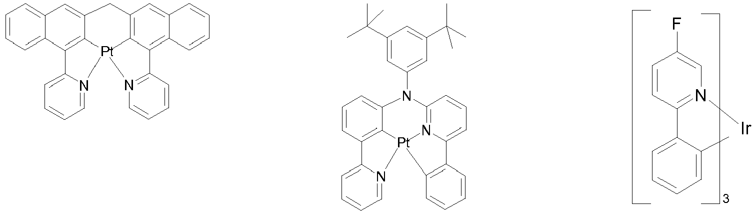

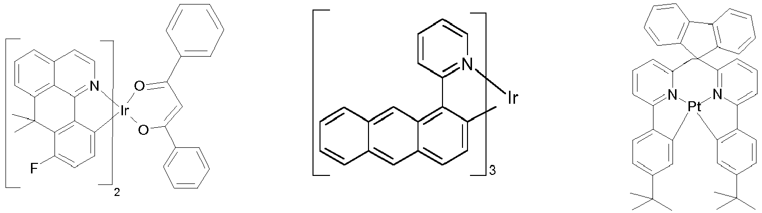

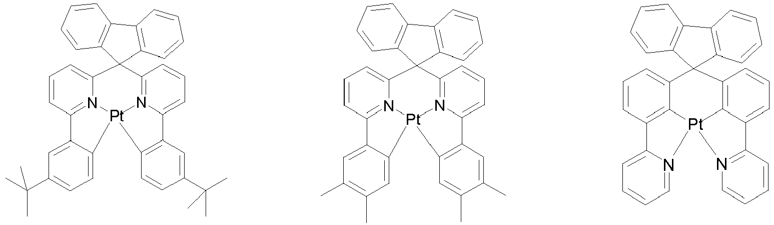

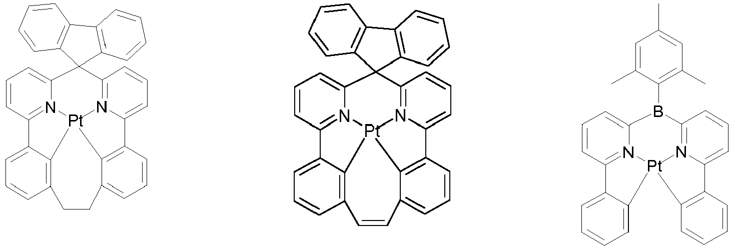

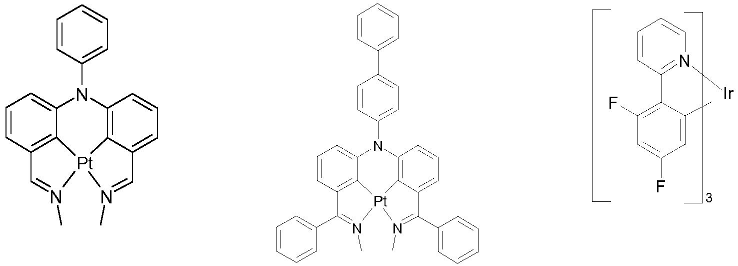

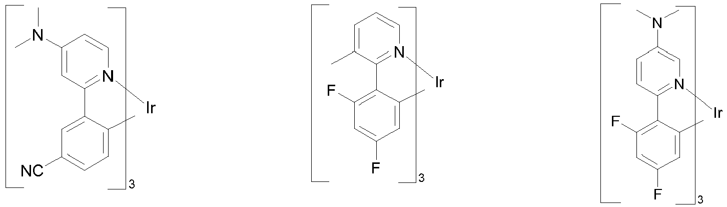

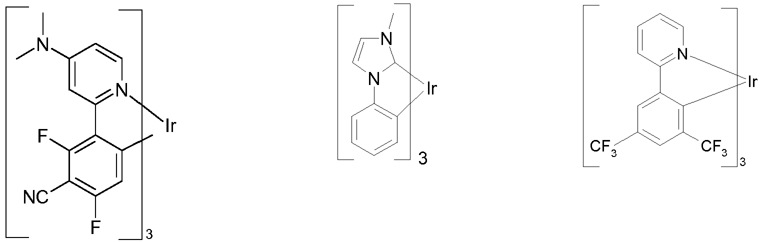

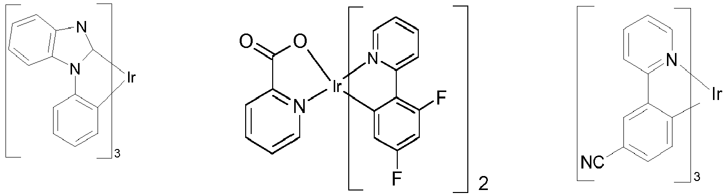

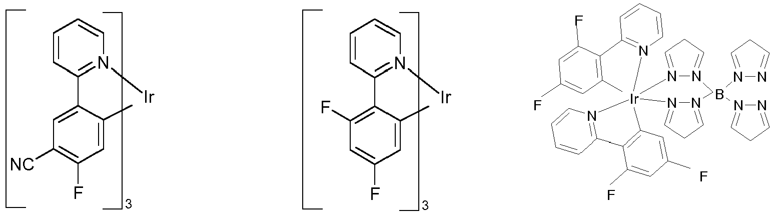

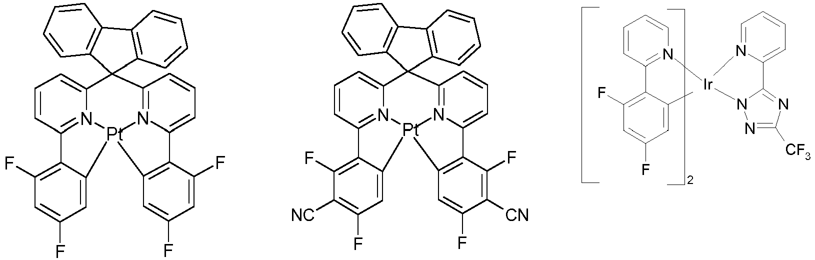

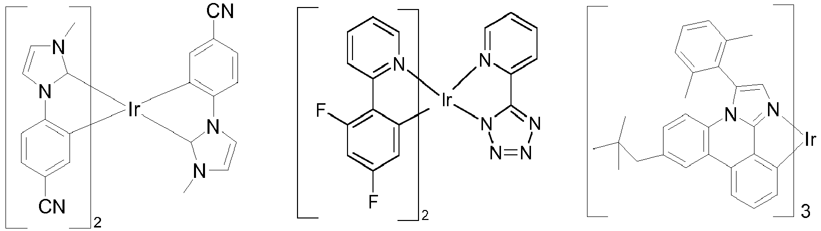

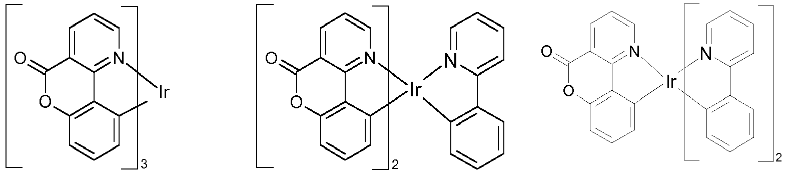

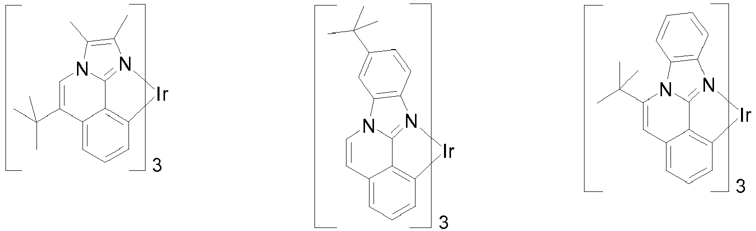

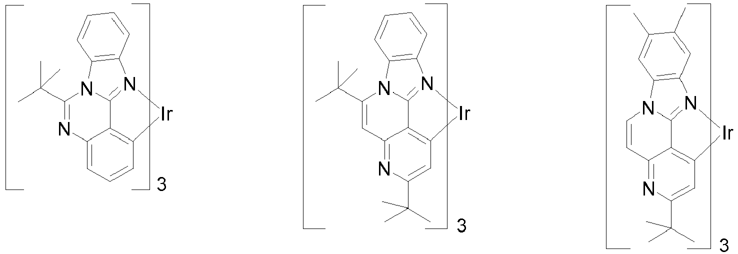

In einer bevorzugten Ausführungsform der Erfindung werden die Verbindungen gemäß Formel (1) als Lochtransportmaterial eingesetzt. Die Verbindungen werden dann bevorzugt in einer Lochtransportschicht und/oder in einer Lochinjektionsschicht eingesetzt. Eine Lochinjektionsschicht im Sinne dieser Erfindung ist eine Schicht, die direkt an die Anode angrenzt. Eine Lochtransportschicht im Sinne dieser Erfindung ist eine Schicht, die zwischen der Lochinjektionsschicht und der Emissionsschicht liegt. Die Lochtransportschicht kann direkt an die Emissionschicht angrenzen. Wenn die Verbindungen gemäß Formel (1) als Lochtransportmaterial oder als Lochinjektionsmaterial verwendet werden, kann es bevorzugt sein, wenn sie mit Elektronenakzeptor-Verbindungen dotiert sind, beispielsweise mit F₄-TCNQ oder mit Verbindungen, wie sie in EP 1476881 oder EP 1596445 beschrieben werden. In einer weiteren bevorzugten Ausführungsform der Erfindung wird eine Verbindung gemäß Formel (I) als Lochtransportmaterial in Kombination mit einem Hexaazatriphenylenderivat, wie in US 2007/0092755 beschrieben, verwendet. Besonders bevorzugt wird das Hexaazatriphenylenderivat dabei in einer separaten Schicht eingesetzt.

Wird die Verbindung gemäß Formel (1) als Lochtransportmaterial in einer Lochtransportschicht eingesetzt, so kann die Verbindung als Reinmaterial, d.h. in einem Anteil von 100 %, in der Lochtransportschicht eingesetzt werden, oder sie kann in Kombination mit einer oder mehreren weiteren Verbindungen in der Lochtransportschicht eingesetzt werden.

In einer weiteren Ausführungsform der vorliegenden Erfindung werden die Verbindungen der Formel (1) als emittierende Materialien eingesetzt. Die Verbindungen werde hierzu bevorzugt in einer Emissionsschicht eingesetzt. Die Emissionsschicht enthält neben wenigstens einer der Verbindungen nach Formel (1) weiterhin wenigstens ein Host Material. Dabei kann der Fachmann aus den bekannten Hostmaterialien ohne Schwierigkeiten und ohne erfinderisch zu sein auswählen.

In einer weiteren Ausführungsform der vorliegenden Erfindung werden die Verbindungen der Formel (1) als Matrixmaterial in Kombination mit einem oder mehreren Dotanden, vorzugsweise phosphoreszierenden Dotanden, eingesetzt.

Unter einem Dotanden wird in einem System enthaltend ein Matrixmaterial und einen Dotanden diejenige Komponente verstanden, deren Anteil in der Mischung der kleinere ist. Entsprechend wird unter einem Matrixmaterial in einem System enthaltend ein Matrixmaterial und einen Dotanden diejenige Komponente verstanden, deren Anteil in der Mischung der größere ist.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%.

Entsprechend beträgt der Anteil des Dotanden zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere Dotanden enthalten. Auch in diesem Fall sind die Dotanden im Allgemeinen diejenigen Materialien, deren Anteil im System der kleinere ist und die Matrixmaterialien sind diejenigen Materialien, deren Anteil im System der größere ist. In Einzelfällen kann jedoch der Anteil eines einzelnen Matrixmaterials im System kleiner sein als der Anteil eines einzelnen Dotanden.

In einer weiteren bevorzugten Ausführungsform der Erfindung werden die Verbindungen gemäß Formel (1) als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Die Mixed-Matrix-Systeme können einen oder mehrere Dotanden umfassen, bevorzugt einen oder mehrere phosphoreszierende Dotanden. Allgemein werden Mixed-Matrix-Systeme bevorzugt in phosphoreszierenden organischen Elektrolumineszenzvorrichtungen eingesetzt.

Besonders geeignete Matrixmaterialien, welche in Kombination mit den erfindungsgemäßen Verbindungen als Matrixkomponenten eines Mixed-Matrix-Systems verwendet werden können, sind ausgewählt aus den unten angegebenen bevorzugten Matrixmaterialien für phosphoreszierende Dotanden oder den bevorzugten Matrixmaterialien für fluoreszierende Dotanden, je nachdem welche Art von Dotand im mixed-Matrix-System eingesetzt wird.

Bevorzugte phosphoreszierende Dotanden zur Verwendung in Mixed-Matrix-Systemen sind die in der obenstehenden Tabelle aufgeführten phosphoreszierenden Dotanden.

Im Folgenden werden die in den erfindungsgemäßen Vorrichtungen in den betreffenden Funktionen bevorzugt eingesetzten Materialien aufgeführt.

Bevorzugte fluoreszierende Dotanden sind ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind.

Als Matrixmaterialien, bevorzugt für fluoreszierende Dotanden, kommen neben den erfindungsgemäßen Verbindungen Materialien verschiedener Stoffklassen in Frage. Bevorzugte Matrixmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind.

Bevorzugte Matrixmaterialien für phosphoreszierende Dotanden sind neben den erfindungsgemäßen Verbindungen aromatische Amine, insbesondere Triarylamine, z. B. gemäß US 2005/0069729, Carbazolderivate (z. B. CBP, N,N-Biscarbazolylbiphenyl) oder Verbindungen gemäß WO 2005/039246, US 2005/0069729, JP 2004/288381, EP 1205527 oder WO 2008/086851, verbrückte Carbazolderivate, z. B. gemäß WO 2011/088877 und WO 2011/128017, Indenocarbazolderivate, z. B. gemäß WO 2010/136109 und WO 2011/000455, Azacarbazolderivate, z. B. gemäß EP 1617710, EP 1617711, EP 1731584, JP 2005/347160, Indolocarbazolderivate, z. B. gemäß WO 2007/063754 oder WO 2008/056746, Ketone, z. B. gemäß WO 2004/093207 oder WO 2010/006680, Phosphinoxide, Sulfoxide und Sulfone, z. B. gemäß WO 2005/003253, Oligophenylene, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Aluminiumkomplexe, z. B. BAlq, Diazasilol- und Tetraazasilol-Derivate, z. B. gemäß WO 2010/054729, Diazaphosphol-Derivate, z. B. gemäß WO 2010/054730 und Aluminiumkomplexe, z. B. BAlQ.

Geeignete Ladungstransportmaterialien, wie sie in der Lochinjektions- bzw. Lochtransportschicht oder in der Elektronentransportschicht der erfindungsgemäßen organischen Elektrolumineszenzvorrichtung verwendet werden können, sind neben den erfindungsgemäßen Verbindungen beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Als Kathode der organischen Elektrolumineszenzvorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere.

Die Vorrichtung wird entsprechend (je nach Anwendung) strukturiert, kontaktiert und schließlich versiegelt, da sich die Lebensdauer der erfindungsgemäßen Vorrichtungen bei Anwesenheit von Wasser und/oder Luft verkürzt.

In einer bevorzugten Ausführungsform ist die erfindungsgemäße organische Elektrolumineszenzvorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (I) nötig. Hohe Löslichkeit lässt sich durch geeignete Substitution der Verbindungen erreichen.

Weiterhin bevorzugt ist es, dass zur Herstellung einer erfindungsgemäßen organischen Elektrolumineszenzvorrichtung eine oder mehrere Schichten aus Lösung und eine oder mehrere Schichten durch ein Sublimationsverfahren aufgetragen werden.

Erfindungsgemäß können die elektronischen Vorrichtungen enthaltend eine oder mehrere Verbindungen gemäß Formel (I) in Displays, als Lichtquellen in Beleuchtungsanwendungen sowie als Lichtquellen in medizinischen und/oder kosmetischen Anwendungen (z.B. Lichttherapie) eingesetzt werden.

Vorrichtungen enthaltend die Verbindungen nach Formel (1) können sehr vielseitig eingesetzt werden. So können, beispielsweise, elektrolumineszierende Vorrichtuungen enthaltend eine oder mehrere Verbindungen nach Formel (1) in Displays für Fernseher, Mobiltelefone, Computer und Kameras eingesetzt werden. Die Vorrichtungen können aber auch in Beleuchtungsanwendungen verwendet werden. Weiterhin können elektrolumineszierende Vorrichtungen, z. B. in OLEDs oder OLECs, enthaltend wenigstens eine der Verbindung nach Formel (1) in der Medizin oder Kosmetik zu Phototherapie genutzt werden. Somit kann eine Vielzahl von Erkrankungen (Psoriasis, atopische Dermatitis, Inflammation, Akne, Hautkrebs etc.) oder die Vermeidung sowie Reduktion von Hautfaltenbildung, Hautrötung und Hautalterung behandelt werden. Weiterhin können die lichtemittierenden Vorrichtungen dazu genutzt werden, um Getränke, Speisen oder Lebensmittel frisch zu halten oder um Geräte (bspw. medizinische Geräte) zu sterilisieren.

Gegenstand der vorliegenden Erfindung ist daher auch eine elektronische Vorrichtung, bevorzugt eine organische elektrolumineszierende Vorrichtung, ganz bevorzugt eine OLED oder OLEC, enthaltend wenigstens eine erfindungsgemäße Verbindung zur phototherapeutischen Verwendung in der Medizin, bevorzugt zur Verwendung zur Behandlung von Hautkrankheiten, ganz bevorzugt zur Verwendung zur Behandlung von Psoriasis, atopischer Dermatitis, Neurodermitis, Hautkrebs, Entzündungen der Haut, Gelbsucht (Ikterus) und Neugeborenengelbsucht.

Ein weiterer Gegenstand der vorliegenden Erfindung betrifft die Verwendung einer elektronischen Vorrichtung, bevorzugt einen organischen elektrolumineszierenden Vorrichtung, ganz bevorzugt eine OLED oder OLEC, enthaltend wenigstens eine erfindungsgemäße Verbindnung in der Kosmetik, bevorzugt zur Behandlung von Akne, Hautrötung, zur Behandlung von Hautalterung (Anti-Ageing), zur Reduktion oder Verringerung von Hautfalten und zur Behandlung von Zellulithe.

Die erfindungsgemäßen Verbindungen bzw. die erfindungsgemäßen organischen Elektrolumineszenzvorrichtungen zeichnen sich durch folgende überraschende Vorteile gegenüber dem Stand der Technik aus:
1. Die erfindungsgemäßen Verbindungen eignen sich sehr gut für den Einsatz in einer Lochtransportschicht oder einer Lochinjektionsschicht in elektronischen Vorrichtungen, wie beispielsweise in organischen Elektrolumineszenzvorrichtungen, insbesondere aufgrund ihrer hohen Lochbeweglichkeit.
2. Die erfindungsgemäßen Verbindungen weisen eine relativ niedrige Sublimationstemperatur, eine hohe Temperaturstabilität sowie eine hohe Oxidationsstabilität und eine hohe Glasübergangtemperatur auf, was sowohl für die Prozessierbarkeit, beispielweise aus Lösung oder aus der Gasphase, als auch für die Verbendung in elektronischen Vorrichtungen vorteilhaft ist.
3. Die Verwendung der erfindungsgemäßen Verbindungen in elektronischen Vorrichtungen, insbesondere eingesetzt als Lochtransport- oder Lochinjektionsmaterial, aber auch als lichtemittierendes Material, führen zu hohen Effizienzen, geringen Betriebspannungen sowie zu langen Lebensdauern.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Es sei ferner darauf hingewiesen, dass viele der Merkmale, und insbesondere die der bevorzugten Ausführungsformen der vorliegenden Erfindung selbst erfinderisch und nicht lediglich als Teil der Ausführungsformen der vorliegenden Erfindung zu betrachten sind. Für diese Merkmale kann ein unabhängiger Schutz zusätzlich oder alternativ zu jeder gegenwärtig beanspruchten Erfindung begehrt werden.

Die mit der vorliegenden Erfindung offengelegte Lehre zum technischen Handeln kann abstrahiert und mit anderen Beispielen kombiniert werden.

Die Erfindung wird durch die nachfolgenden Beispiele näher erläutert, ohne sie dadurch einschränken zu wollen.

### Beispiele

### Materialien

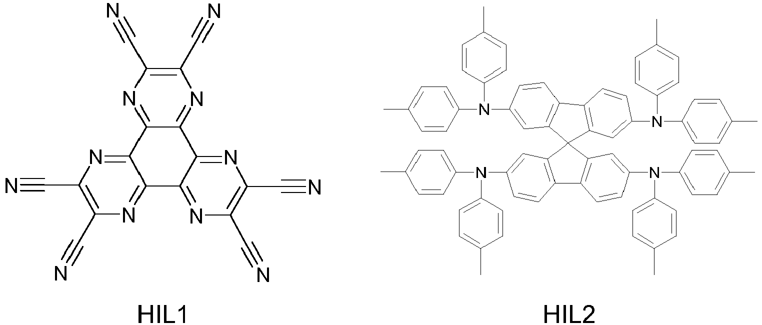

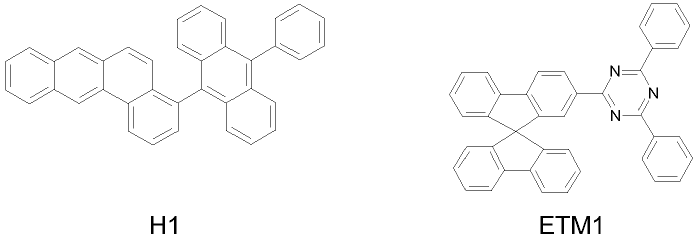

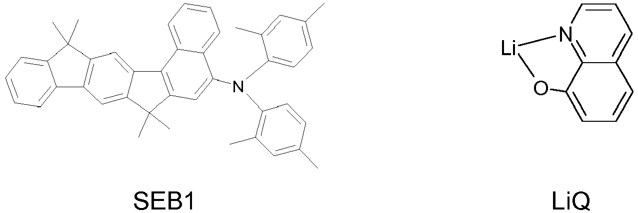

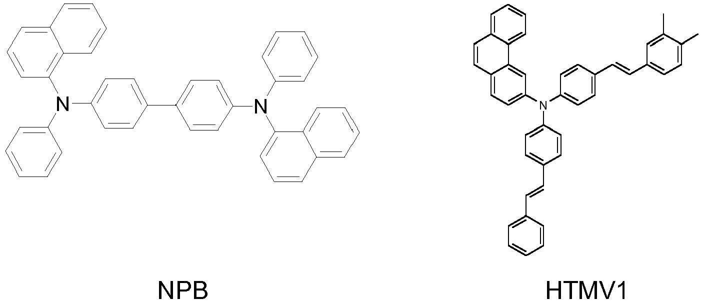

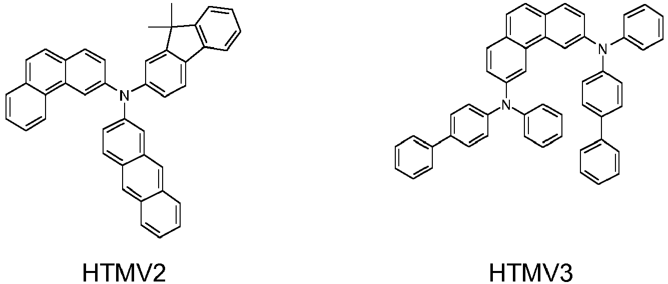

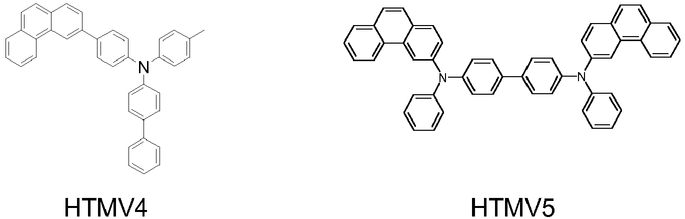

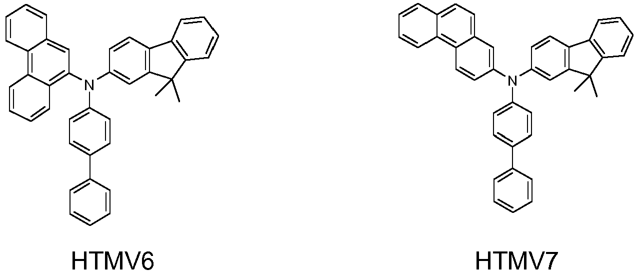

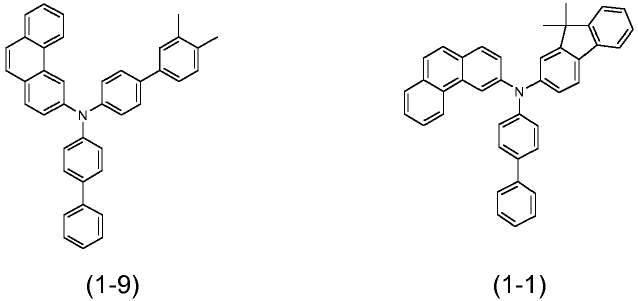

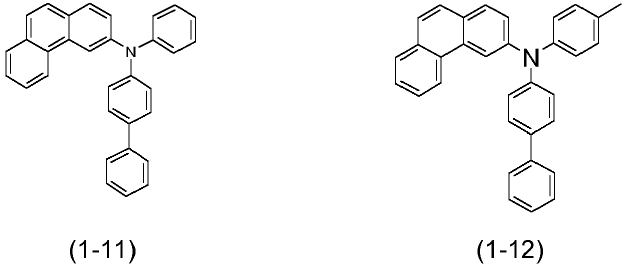

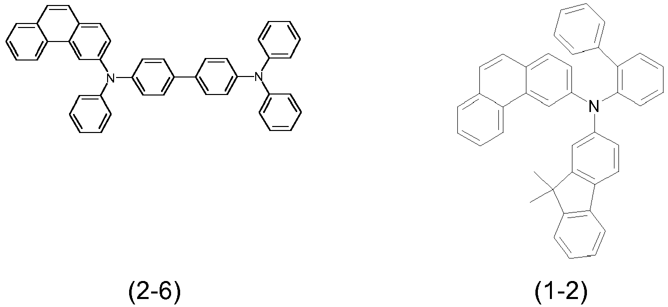

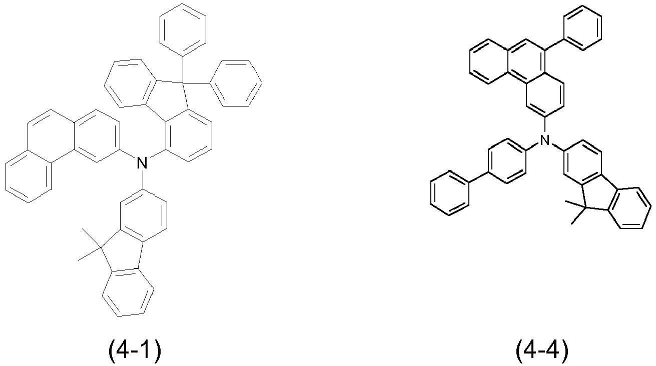

Die Materialien HIL1, HIL2 (EP 0676461), H1 (WO 2008/145239), ETM1 (WO 2005/053055), SEB1 (WO 2008/006449), LiQ und NPB sind dem Fachmann wohl bekannt. Deren Eigenschaften und Synthesen sind aus dem Stand der Technik bekannt. Die Verbindungen (1-9), (1-1), (1-11), (1-12), (2-6) (1-2) und (4-1) sind erfindungsgemäß.

### Beispiel 1

### Synthese von Biphenyl-4-yl-(9,9-dimethyl-9H-fluoren-2-yl)-phenanthren-3-yl-amin (1-1)

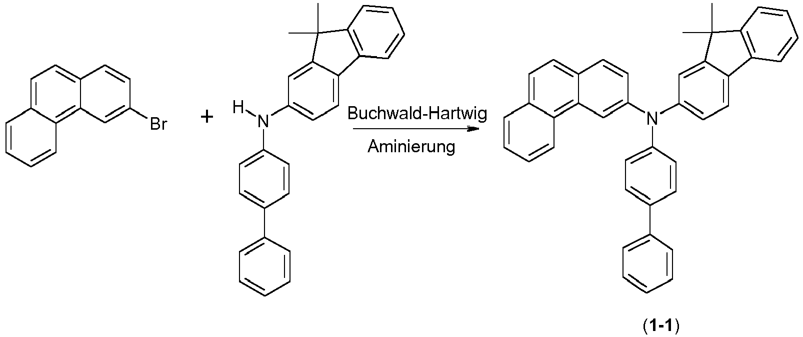

### Synthese von Ausgangmaterial 3-Brom-Phenanthren

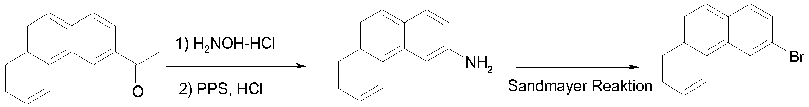

### Synthese von 3-Amino-Phenanthren

50 g (227 mmol) 3-Acetyl-Phenenthren und 63,8 mL Pyridin(790 mmol) und 42 g (592 mmol) Hydroxylammoniumchlorid werden in 300 mL EtOH gelöst. Der Ansatz wird auf 75°C erhitzt. Nach 1 h Reaktion wird der Ansatz abgekühlt. Das Gemisch wird im Anschluss zwischen Ethylacetat und Wasser verteilt, die organische Phase dreimal mit Wasser gewaschen und über Na₂SO₄ getrocknet und einrotiert. Die einrotierte Lösung wird vorsichtig mit 300 ml Polyphosphorsäure versetzt und 1 h auf 75°C erhitzt. Danach wird der Ansatz auf Raumtemperatur abgekühlt, und vorsichtig mit Eiswasser gegossen (300 mL). Der ausgefallene Feststoff wird abgesaugt und mit Methanol nachgewaschen. Abschließend wird der Feststoff mit 800 mL MeOH und 70 ml konz. HCl versetzt. Die Reaktionsmischung wird 8 h zum Sieden erhitzt. Das Gemisch wird im Anschluss mit Natronlaugen neutralisiert, zwischen Etylacetat und Wasser verteilt, die organische Phase dreimal mit Wasser gewaschen und über Na₂SO₄ getrocknet und einrotiert. Der Rückstand wurde im Vakuum bei 40°C getrocknet. Ausbeute 35,5 g (184 mmol) (81% der Theorie)

### Synthese von 3-Brom-Phenanthren

30 g (155 mmol) 3-Amino-Phenanthren und 36,7 g CuBr₂ (155 mmol) werden in 300 mL getrocknetem Acetonitril gelöst. Bei 0°C werden 40,4 mL tert-Butylnitrit (535 mmol) portionsweise zugegeben. Die Suspension wird 1 h weiter gerührt und dann auf 400 mL Eiswasser gegossen und ca. 20 min gerührt. Der ausgefallene Feststoff wird abgesaugt, in Dichlormethan gelost und mir Wasser mehrere male gewaschen. Die organische Phase wird einrotiert und aus Toluol/Heptan umkristallisiert. Ausbeute: 21,9 g (85 mmol) (55% d. Theorie)

Analog dazu werden können andere halogenierten Phenanthrene als Ausgangsverbindungen hergestellt werden.

| **Edukt 1** | **Edukt 2** | **Produkt** | **Ausbeute** |
|---|---|---|---|
| 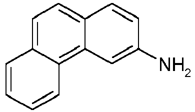 | CuI₂ | 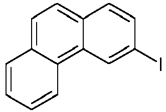 | 45 % |
| 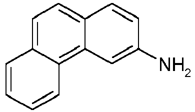 | CuCl₂ | 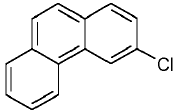 | 50% |

### Synthese der Verbindung (1-1)

28,1 g (78 mmol) Biphenyl-4-yl-(9,9-dimethyl-9H-fluoren-2-yl)-amine, 20,0 g (78 mol) 3-Bromo-phenanthrene werden in 600 mL Toluol gelöst: Die Lösung wird entgast und mit N₂ gesättigt. Danach wird sie mit 3,1 mL (3,11 mmol) einer Tri-tert-Butylphosphin Lösung und 0,35 g (1,56 mmol) Palladium(II)acetat versetzt und anschließend werden 11,6 g Natrium-tert-butylat (116,7 mmol) zugegeben. Die Reaktionsmischung wird 5 h unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert. Nach Filtration des Rohproduktes über Kieselgel mit Toluol wird der verbleibende Rückstand aus Heptan/Toluol umkristallisiert und abschließend im Hochvakuum sublimiert, Reinheit beträgt 99.9%(HPLC). Die Ausbeute von Verbindung (**1-1**) beträgt 29,6 g (71% der Theorie).

### Beispiele 2 - 12

### Synthese der Verbindungen (1-2) bis (1-12)

Analog zu der in Beispiel 1 beschriebenen Synthese von Verbindung (**1-1**) werden auch die folgenden Verbindungen (**1-2**) bis (**1-12**) hergestellt.

| | **Edukt 1** | **Edukt 2** | **Produkt** | **Ausbeute** |
|---|---|---|---|---|
| **1-2** | 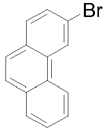 | 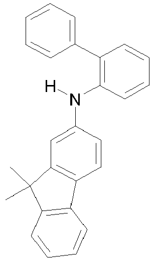 | 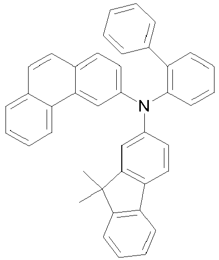 | 78 % |
| **1-3** | 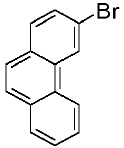 | 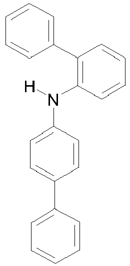 | 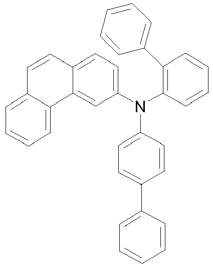 | 82% |
| **1-4** | 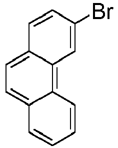 | 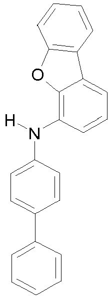 | 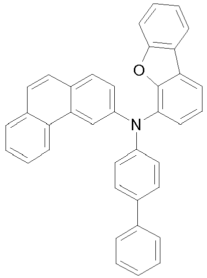 | 88% |
| **1-5** | 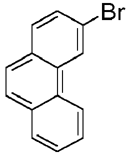 | 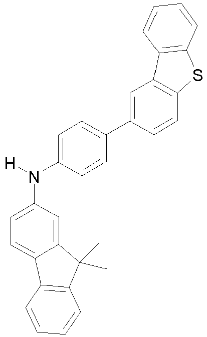 | 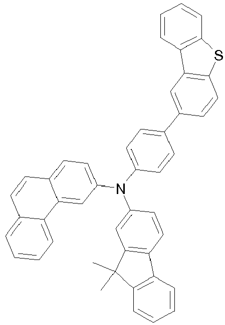 | 67% |
| **1-6** | 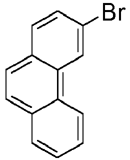 | 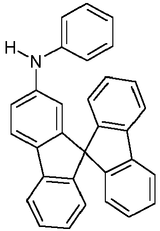 | 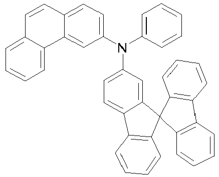 | 76% |
| **1-7** | 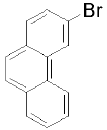 | 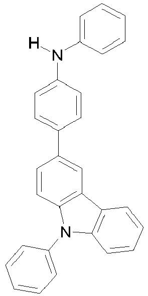 | 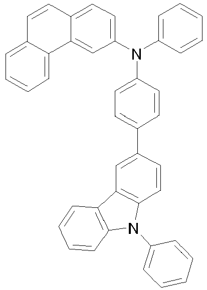 | 80% |
| **1-8** | 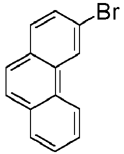 | 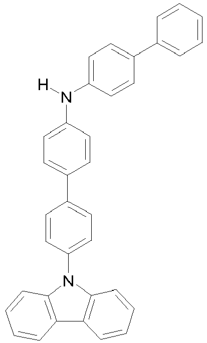 | 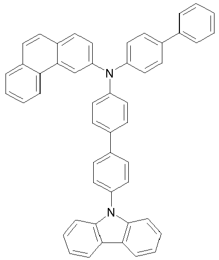 | 75% |
| **1-9** | 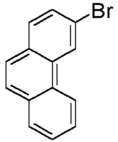 | 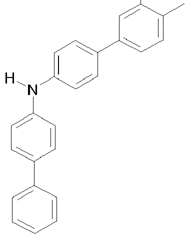 | 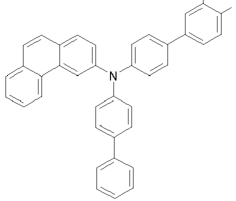 | 70% |
| **1-10** | 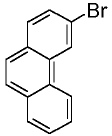 | 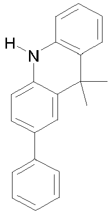 | 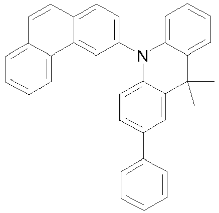 | 67% |
| **1-11** | 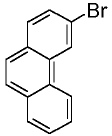 | 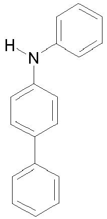 | 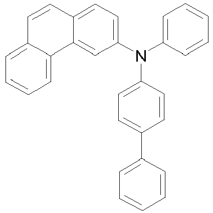 | 72% |
| **1-12** | 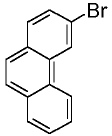 | 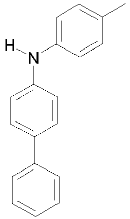 | 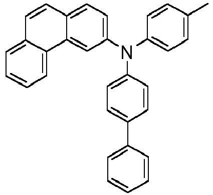 | 65% |

Analog zu der in Beispiel 1 beschriebenen Synthese von Verbindung (1-1) werden auch die folgenden Vergleichsverbindungen (**HTMV1**) bis (**HTMV3**) und (**HTMV6**) bis (**HTMV7**) hergestellt.

| | **Edukt 1** | **Edukt 2** | **Produkt** |
|---|---|---|---|
| **HTMV1** | 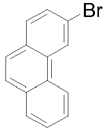 | 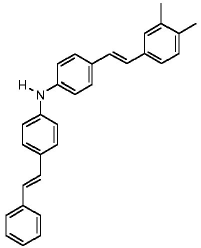 | 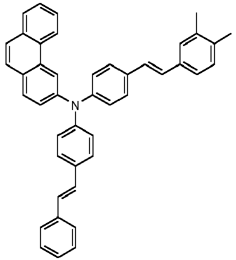 |
| **HTMV2** | 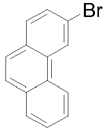 | 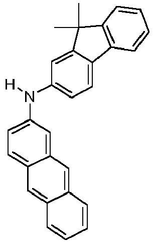 | 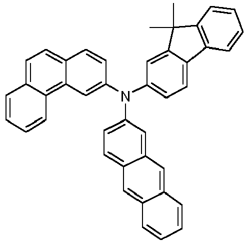 |
| **HTMV3** | 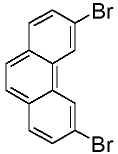 | 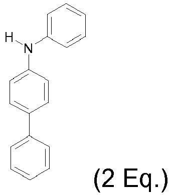 | 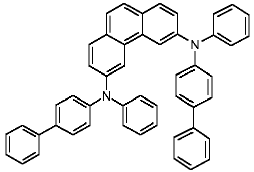 |
| **HTMV6** | 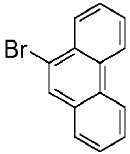 | 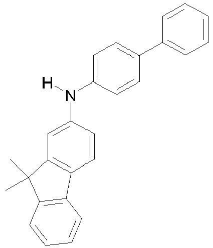 | 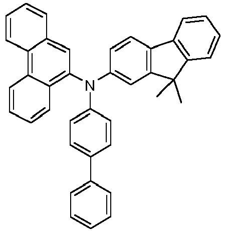 |
| **HTMV7** | 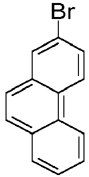 | 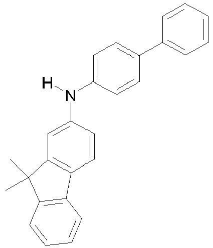 | 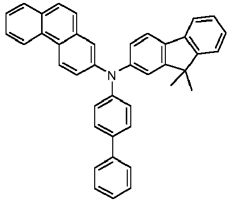 |

### Beispiele 13

### Synthese der Verbindung N*4'*-Biphenyl-4-yl-N*4'*-dibenzofuran-4-yl-N*4*-phenanthren-3-yl-N*4*-phenyl-biphenyl-4,4'-diamin (2-1)

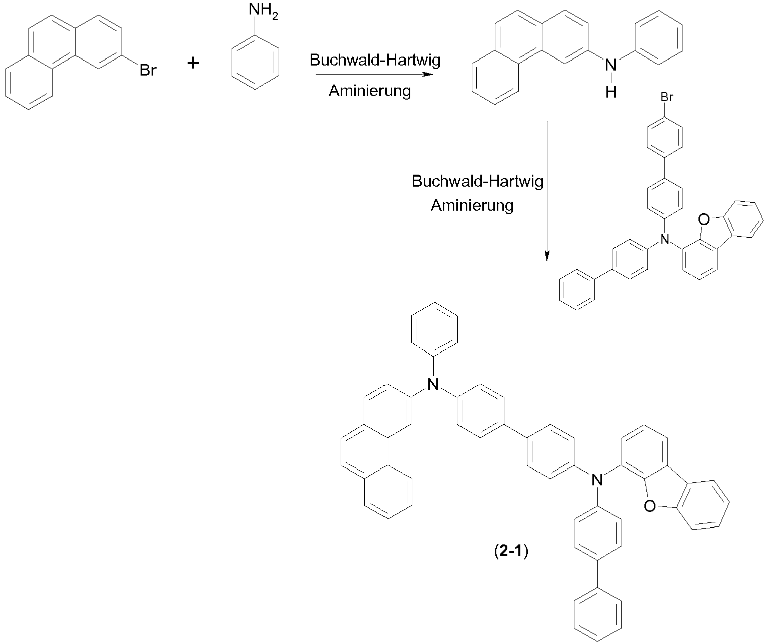

10 g Phenanthren-3-yl-phenyl-amine (37 mmol), 21 g. Biphenyl-4-yl-(4'-bromo-biphenyl-4-yl)-dibenzofuran-4-yl-amine (37 mol) werden in 500 mL Toluol gelöst: Die Lösung wird entgast und mit N₂ gesättigt. Danach wird sie mit 1,5 mL (1,5 mmol) einer Tri-tert-Butylphosphin-Lösung und 0,17 g (0,74 mmol) Palladium(II)acetat versetzt und anschließend werden 5,6 g Natrium-tert-butylat (56 mmol) zugegeben. Die Reaktionsmischung wird 3 h unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert. Nach Filtration des Rohproduktes über Kieselgel mit Toluol wird der verbleibende Rückstand aus Heptan/Toluol umkristallisiert und abschließend im Hochvakuum sublimiert, Reinheit beträgt 99.9 % (HPLC). Die Ausbeute beträgt 16,8 g (60% der Theorie).

### Beispiele 14- 18

### Synthese der Verbindungen (2-2) bis (2-6)

Analog zu der in Beispiel 13 beschriebenen Synthese von Verbindung (**2-1**) werden auch die folgenden Verbindungen (**2-2**) bis (**2-6**) hergestellt.

| | **Edukt 1** | **Edukt 2** | **Produkt** | **Ausbeute** |
|---|---|---|---|---|
| **2-2** | 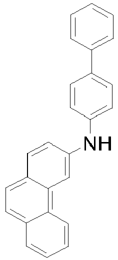 | 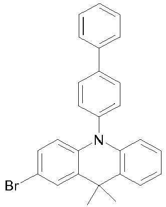 | 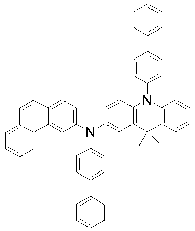 | 55% |
| **2-3** | 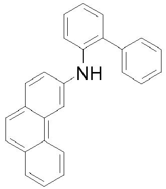 | 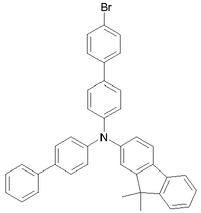 | 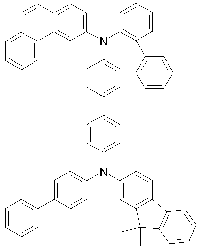 | 62% |
| **2-4** | 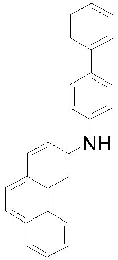 | 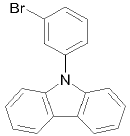 | 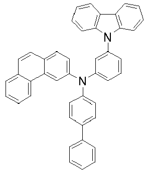 | 65% |
| **2-5** | 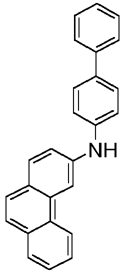 | 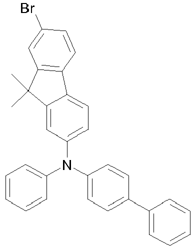 | 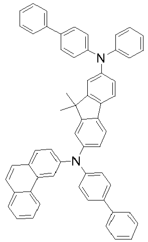 | 65% |
| **2-6** | 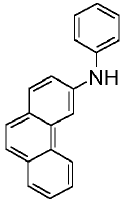 | 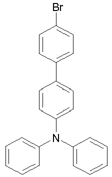 | 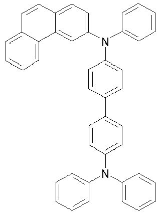 | 60% |

Analog zu der in Beispiel 13 beschriebenen Synthese von Verbindung (**2-1**) wird auch die Vergleichsverbindung (**HTMV5**) hergestellt.

| | **Edukt 1** | **Edukt 2** | **Produkt** |
|---|---|---|---|
| **HTMV5** | 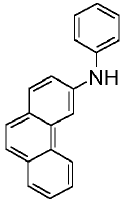 | 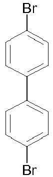 | 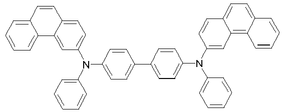 |
| | (2 Eq) | | |

### Beispiele 19

### Synthese der Verbindung Biphenyl-4-yl-biphenyl-2-yl-(9,9-dimethyl-7-phenanthren-3-yl-9H-fluoren-2-yl)-amin (3-1)

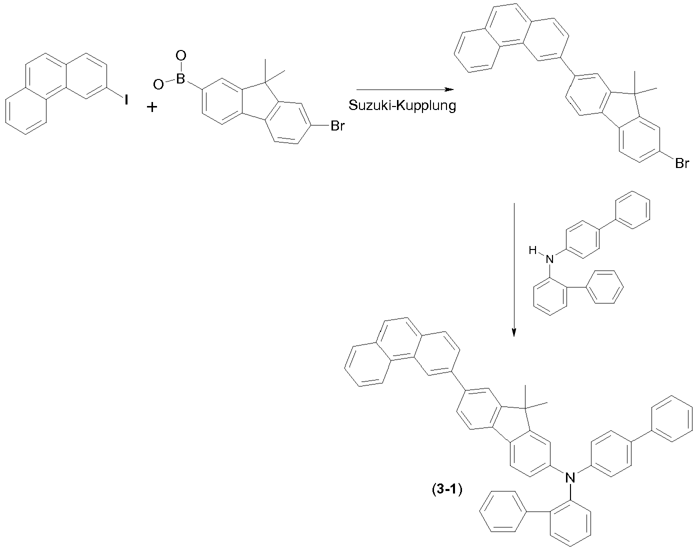

### 3-(7-Bromo-9,9-dimethyl-9H-fluoren-2-yl)-phenanthren

52 g (164 mmol) 7-Brom-(9,9-dimethyllfluoren-2-yl)boronsäure (CAS-Nr.: 1213768-48-9), 50 g (164 mmol) 3-lod-Phenanthren unda 205 mL einer 2 M NaHCO₃ wässrige-Lösung (327 mmol) werden in 800 mL Dimethoxyethan suspendiert. Zu dieser Suspension werden 3,8 g (3,3 mmol) Tetrakis-(triphenyl)phosphin-palladium(0) gegeben, und die Reaktionsmischung wird 16 h unter Rückfluss erhitzt. Nach Erkalten wird die organische Phase abgetrennt, über Kieselgel filtriert, dreimal mit 300 mL Wasser gewaschen und anschließend zur Trockene eingeengt. Nach Filtration des Rohproduktes über Kieselgel mit Heptan/Essigsäureester (20:1) erhielt man 55 g (75%) 3-(7-Bromo-9,9-dimethyl-9H-fluoren-2-yl)-phenanthrene.

### Biphenyl-4-yl-biphenyl-2-yl-(9,9-dimethyl-7-phenanthren-3-yl-9H-fluoren-2-yl)-amine

19,2 g Biphenyl-4-Biphenyl-2-yl-amin (60 mmol), 26,9 g 3-(7-Bromo-9,9-dimethyl-9H-fluoren-2-yl)-phenanthrene (60 mol) werden in 500 mL Toluol gelöst: Die Lösung wird entgast und mit N₂ gesättigt. Danach wird sie mit 3,2 g (3,9 mmol) einer Tri-tert-Butylphosphin-Lösung und 0,27 g (1,2 mmol) Palladium(II)acetat versetzt und anschließend werden 8,9 g Natrium-tert-butylat (90 mmol) zugegeben. Die Reaktionsmischung wird 4 h unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert. Nach Filtration des Rohproduktes über Kieselgel mit Toluol wird der verbleibende Rückstand aus Heptan/Toluol umkristallisiert und abschließend im Hochvakuum sublimiert, Reinheit beträgt 99.9%. Die Ausbeute beträgt 28 g (68% der Theorie.

### Beispiele 20- 22

### Synthese der Verbindungen (3-2) bis (3-4)

Die Ausgangsverbindungen werden analog zu der in Beispiel 18 beschriebenen Synthese hergestellt.

| **Edukt 1** | **Edukt 2** | **Produkt** | **Ausbeute** |
|---|---|---|---|
| 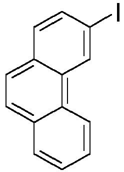 | 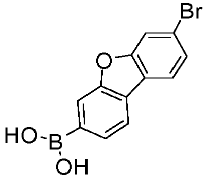 | 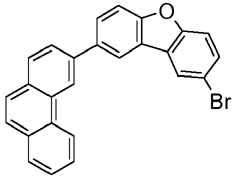 | 78% |
| | CAS-Nr.: 1177264-88-8 | | |
| 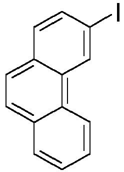 | 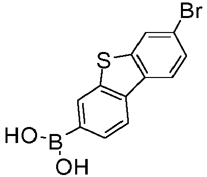 | 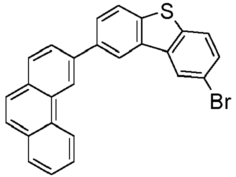 | 70% |
| | CAS-Nr.: 1030620-82-6 | | |

Die Verbindungen (**3-2**) bis (**3-4**) werden analog zu der in Beispiel 19 beschriebenen Synthese von Verbindung (**3-1**) hergestellt.

| | **Edukt 1** | **Edukt 2** | **Produkt** | **Ausbeute** |
|---|---|---|---|---|
| **3-2** | 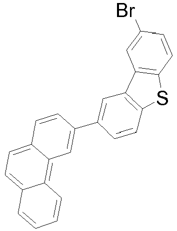 | 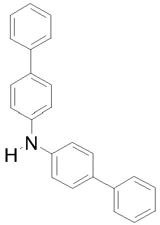 | 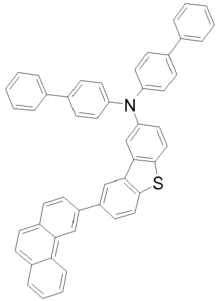 | 65% |
| **3-3** | 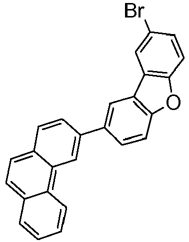 | 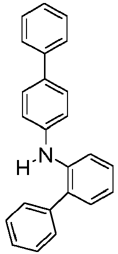 | 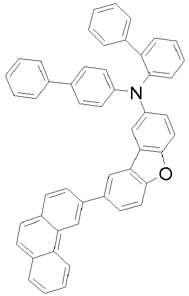 | 75% |
| **3-4** | 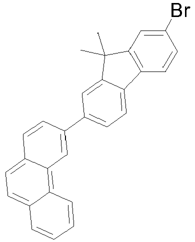 | 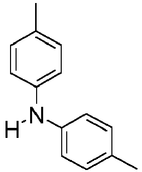 | 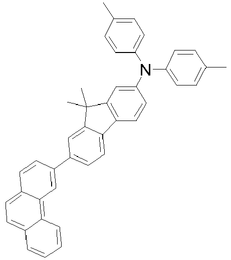 | 78% |

Analog zu der in Beispiel 19 beschriebenen Synthese von Verbindung (**3-1**) wird auch die Vergleichverbindung (**V4**) hergestellt.

| | **Edukt 1** | **Edukt 2** | **Produkt** |
|---|---|---|---|
| **HTMV4** | 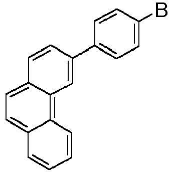 | 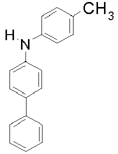 | 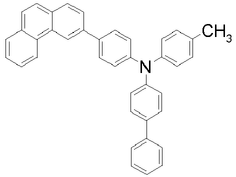 |

### Beispiele 23 -25

### Synthese der Verbindungen (9,9-Dimethyl-9H-fluoren-2-yl)-(9,9-diphenyl-9H-fluoren-4-yl)-phenanthren-3-yl-amin (4-1), (4-2 und (4-3)

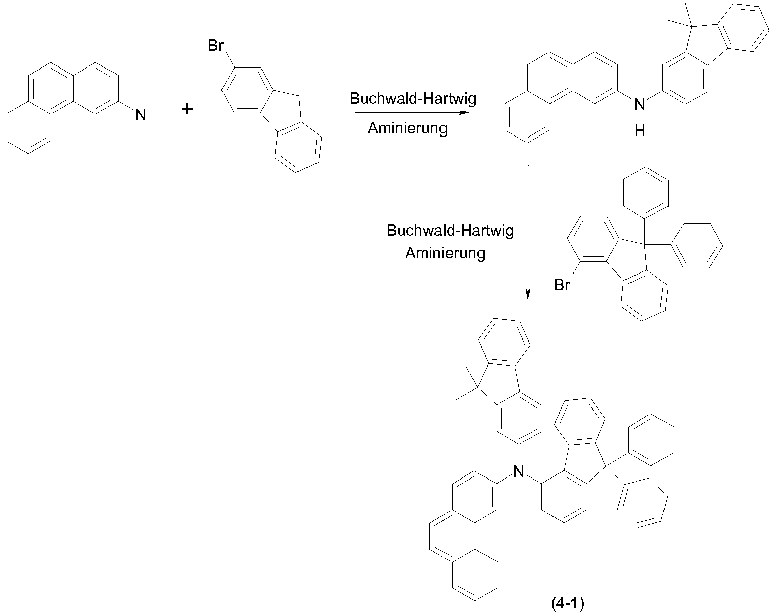

### (9,9-Dimethyl-9H-fluoren-2-yl)-phenanthren-3-yl-amin

18,4 g (95,29 mmol) 3-Aminophenanthren,26 g (95,4 mmol) 2-Brom-Fluoren und 18,3 g (190 mmol) Natriumtertbutylat werden in 350 ml Toluol, suspendiert. Zu dieser Suspension werden 1,07 g (4,76 mmol) Palladiumacetat und 2,64 g 1,1-Bis(diphenylphosphino)ferrocen (4,76 mmol) gegeben. Die Reaktionsmischung wird 16 h unter Rückfluss erhitzt. Nach dem Erkalten wird die organische Phase abgetrennt dreimal mit 200 mL Wasser gewaschen und anschließend zur Trockene eingeengt. Der Rückstand wird aus Toluol umkristallisiert (31 g, 85% Ausbeute).

### (9,9-Dimethyl-9H-fluoren-2-yl)-(9,9-diphenyl-9H-fluoren-4-yl)-phenanthren-3-yl-amin (4-1)

13,1 g (9,9-Dimethyl-9H-fluoren-2-yl)-phenanthren-3-yl-amin (34 mmol), 13,5 g 4-Bromo-9,9-diphenyl-9H-fluoren (34 mol) werden in 300 mL Toluol gelöst: Die Lösung wird entgast und mit N₂ gesättigt. Danach wird sie mit 1,4 mL (0,68 mmol) einer 1 M Tri-tert-Butylphosphin-Lösung und 0,153 g (0,68 mmol) Palladium(II)acetat versetzt und anschließend werden 6,5 g Natrium-tert-butylat (68 mmol) zugegeben. Die Reaktionsmischung wird 4 h unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert. Nach Filtration des Rohproduktes über Kieselgel mit Toluol wird der verbleibende Rückstand aus Heptan/Toluol umkristallisiert und abschließend im Hochvakuum sublimiert, Reinheit beträgt 99.9%. Die Ausbeute beträgt 14,3 g (60% der Theorie).

Analog dazu werden folgende Verbindungen hergestellt:

| | **Edukt 1** | **Edukt 2** | **Produkt** | **Ausbeute** |
|---|---|---|---|---|
| 4-2 | 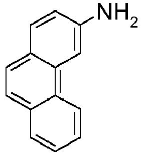 | 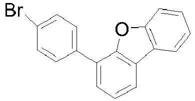 | 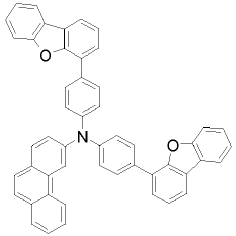 | 65% |
| | | 2Eq | | |
| 4-3 | 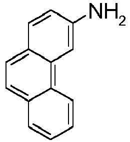 | 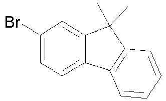 | 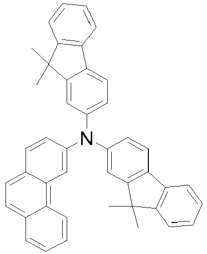 | 58% |
| | | 2 Eq | | |

### Beispiel 26

### Charakterisierung der Verbindungen

Die Herstellung von erfindungsgemäßen OLEDs sowie OLEDs nach dem Stand der Technik erfolgt nach einem allgemeinen Verfahren gemäß WO 2004/058911, das auf die hier beschriebenen Gegebenheiten (Schichtdickenvariation, Materialien etc.) angepasst wird.

In den folgenden Beispielen (siehe Tabellen 1 und 2) werden die Daten verschiedener OLEDs gezeigt. Als Substrate werden Glasplättchen verwendet, die mit strukturiertem ITO (Indium Zinn Oxid) der Dicke 50 nm beschichtet sind. Die OLEDs haben prinzipiell folgenden Schichtaufbau: Substrat / Lochinjektionsschicht (HIL1) / Lochtransportschicht (HIL2) / Lochinjektionsschicht (HIL3) / Elektronenblockerschicht (EBL) / Emissionsschicht (EML) / Elektronentransportschicht (ETL) / Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind oben gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie H1:SEB1 (95%:5%) bedeutet hierbei, dass das Material H1 in einem Volumenanteil von 95% und SEB1 in einem Anteil von 5% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe EQE @ 1000 cd/m² bezeichnet die externe Quanteneffizienz bei einer Betriebsleuchtdichte von 1000 cd/m². LD80 @ 6000 cd/m2 ist die Lebensdauer bis das OLED bei einer Helligkeit von 6000 cd/m² auf 80% der Anfangsintensität, also auf 4800 cd/m² abgefallen ist. Die Daten der verschiedenen OLEDs sind in Tabelle 2 zusammengefasst.

### Verwendung von erfindungsgemäßen Verbindungen als Matrixmaterialien in fluorszierenden OLEDs

Insbesondere eignen sich erfindungsgemäße Verbindungen als HIL, HTL oder EBL in OLEDs. Sie eignen sich als Einzelschicht, aber auch als Mixed Komponente als HIL, HTL, EBL oder innerhalb der EML.

Verglichen mit NPB Referenz Bauteilen (V1) zeigen alle Proben mit den erfindungsmässigen Verbindungen sowohl höhere Effizienzen, als auch deutlich verbesserte Lebensdauern in Singulett blau.

Im Vergleich zu den Referenzmaterial HTMV1 - HTMV5 (V2-V6) haben die erfindungsmässige Verbindung (1-9), (1-1) und (1-11) bessere Effizienzen und verbesserte Lebensdauern. Im Vergleich zu HTMV6 und HTMV7 (V7 und V8) hat die erfindungsmässige Verbindung (1-1) eine deutlich verbesserte Lebensdauer.

| **Tabelle 1: Aufbau der OLEDs** | | | |
|---|---|---|---|
| **IL (5 nm HIL1) / HTL (150 nm HIL2) / IL(5 nm HIL1) /EBL / EML / ETL** | | | |
| ***Bsp.*** | ***EBL*** | ***EML*** | ***ETL*** |
| | *Dicke* / *nm* | *Dicke* / *nm* | *Dicke* / *nm* |
| *V1* | *NPB 20 nm* | *H1 (95%):SEB1(5%) 20 nm* | *ETM1 (50%):LiQ(50%) 30 nm* |
| *V2* | *HTMV1 20 nm* | *H1 (95%):SEB1(5%) 20 nm* | *ETM1 (50%):LiQ(50%) 30 nm* |
| *V3* | *HTMV2 20 nm* | *H1 (95%):SEB1(5%) 20 nm* | *ETM1 (50%):LiQ(50%) 30 nm* |
| *V4* | *HTMV3 20 nm* | *H1 (95%):SEB1(5%) 20 nm* | *ETM1 (50%):LiQ(50%) 30 nm* |
| *V5* | *HTMV4 20 nm* | *H1 (95%):SEB1(5%) 20 nm* | *ETM1 (50%):LiQ(50%) 30 nm* |
| *V6* | *HTMV5 20 nm* | *H1 (95%):SEB1(5%) 20 nm* | *ETM1 (50%):LiQ(50%) 30 nm* |
| *V7* | *HTMV6 20 nm* | *H1(95%):SEB1(5%) 20 nm* | *ETM1 (50%):LiQ(50%) 30 nm* |
| *V8* | *HTMV7 20 nm* | *H1 (95%):SEB1(5%) 20 nm* | *ETM1 (50%):LiQ(50%) 30 nm* |
| *E0* | *HTMV8 20 nm* | *H1 (95%):SEB1(5%) 20 nm* | *ETM1 (50%):LiQ(50%) 30 nm* |
| *E1* | *(1-9) 20 nm* | *H1 (95%):SEB1(5%) 20 nm* | *ETM1 (50%):LiQ(50%) 30 nm* |
| *E2* | *(1-1) 20 nm* | *H1(95%):SE81(5%) 20 nm* | *ETM1 (50%):LiQ(50%) 30 nm* |
| *E3* | *(1-11) 20 nm* | *H1 (95%):SEB1(5%) 20 nm* | *ETM1 (50%):LiQ(50%) 30 nm* |
| *E4* | *(1-12) 20 nm* | *H1(95%):SEB1(5%) 20 nm* | *ETM1 (50%):LiQ(50%) 30 nm* |
| *E5* | *(2-6) 20 nm* | *H1 (95%):SEB1(5%) 20 nm* | *ETM1 (50%):LiQ(50%) 30 nm* |
| *E6* | *(1-2) 20 nm* | *H1 (95%):SEB1(5%) 20 nm* | *ETM1 (50%):LiQ(50%) 30 nm* |
| *E7* | *(4-1) 20 nm* | *H1(95%):SEB1(5%) 20 nm* | *ETM1 (50%):LiQ(50%) 30 nm* |

| **Tabelle 2: Daten der OLEDs** | | | | |
|---|---|---|---|---|
| ***Bsp.*** | ***EQE* @ *1000 cd*/*m2*** | ***LD80* @ *6000 cd*/*m²*** | ***CIE*** | |
| | *%* | *[h]* | *x* | y |
| *V1* | *4.8* | *70* | 0.14 | 0.17 |
| *V2* | *6.4* | *20* | 0.13 | 0.16 |
| *V3* | *5.4* | *25* | 0.13 | 0.15 |
| *V4* | *6.6* | *65* | 0.13 | 0.15 |
| *V5* | *5.6* | *80* | 0.13 | 0.15 |
| *V6* | *5.0* | *60* | 0.13 | 0.16 |
| *V7* | *6.8* | *100* | 0.14 | 0.14 |
| *V8* | *6.9* | *110* | 0.14 | 0.15 |
| *E0* | *6.8* | *115* | 0.13 | 0.16 |
| *E1* | *6.7* | *125* | 0.13 | 0.15 |
| *E2* | *7.0* | *155* | 0.13 | 0.15 |
| *E3* | *6.9* | *135* | 0.13 | 0.15 |
| *E4* | *6.8* | *120* | 0.13 | 0.15 |
| *E5* | *5.0* | *80* | 0.13 | 0.16 |
| *E6* | *7.1* | *125* | 0.13 | 0.15 |
| *E7* | *7.1* | *120* | 0.13 | 0.15 |

Beispiel E0 zeigt einen deutlich verbesserten LD50 Wert gegenüber den Vergleichsbeispielen V1 bis V8. Weitere, deutliche Verbesserungen gegenüber sowohl den Vergleichsbeispielen als auch gegenüber E0 können erreicht werden, indem das Phenanthren, abgesehen von Position 3, keine weitere aromatische und/oder heteroaromatische Substitution aufweist (E1 bis E7). Verbindung (2-6) (E5) ist direkt mit NPB (V1) zu vergleichen. Es zeigt sich, dass Verbindung (2-6) zu Vorrichtungen mit deutlich verbesserten EQE- und insbesondere zu verbesserten LD80-Werten führt.

## Patentansprüche

1. Verbindung der allgemeinen Formel (1) 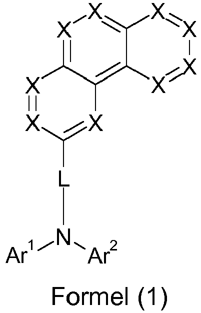 wobei für die auftretenden Symbole und Indices gilt:
X ist bei jedem Auftreten CR¹;
L ist eine Einfachbindung
Ar¹, Ar²
ist bei jedem Auftreten gleich oder verschieden ausgewählt aus den folgenden Gruppen, wobei wenigstens einer der Reste Ar¹ und Ar² mehr als einen Ring enthält 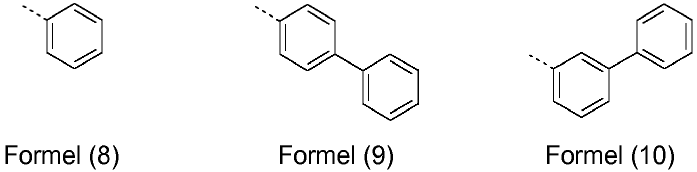 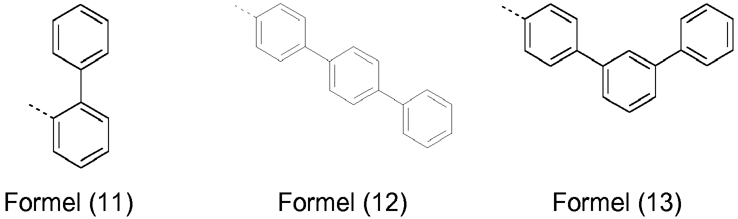 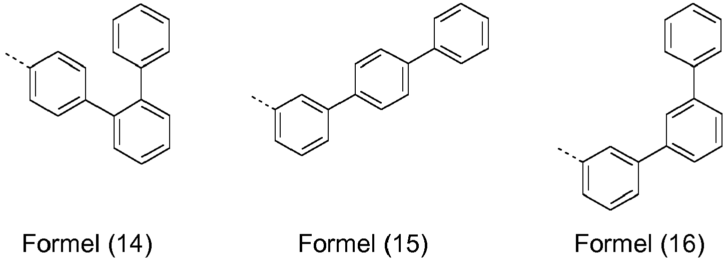 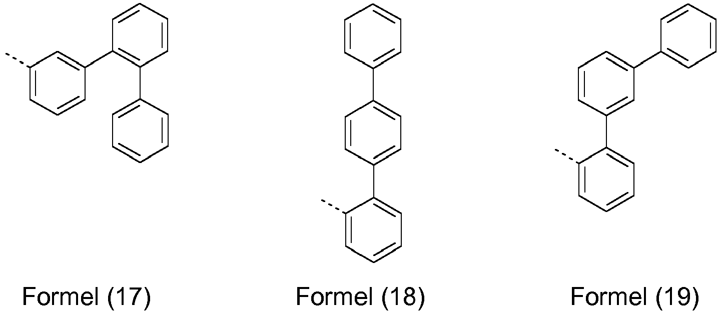 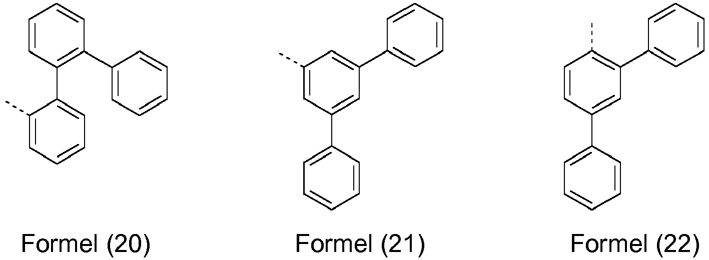 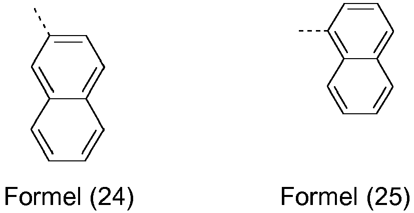 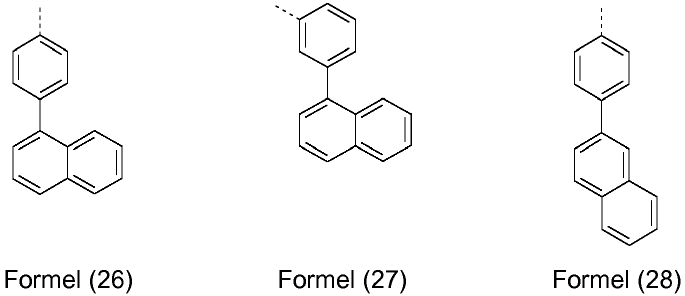 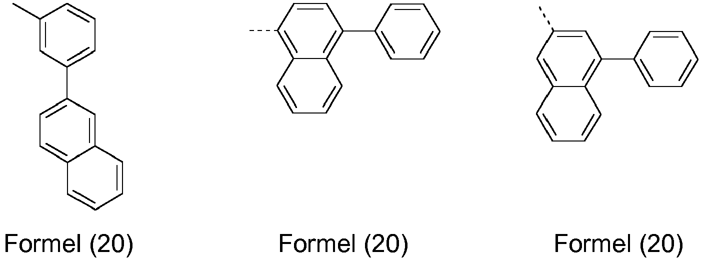 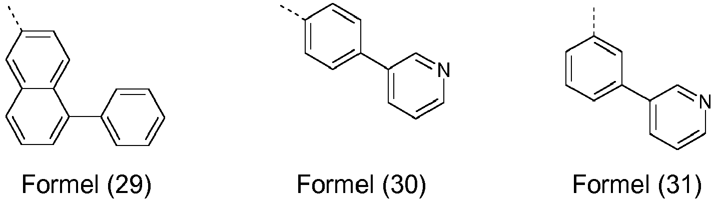 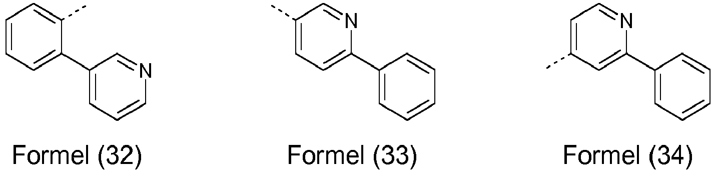 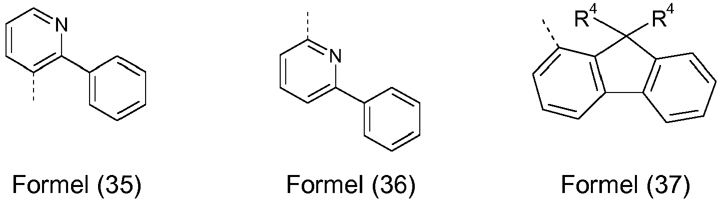 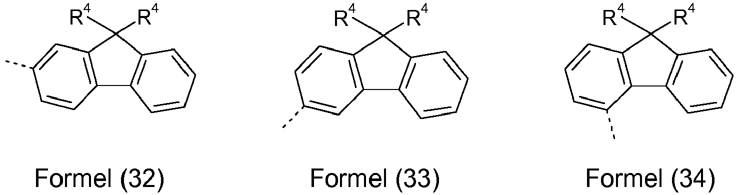 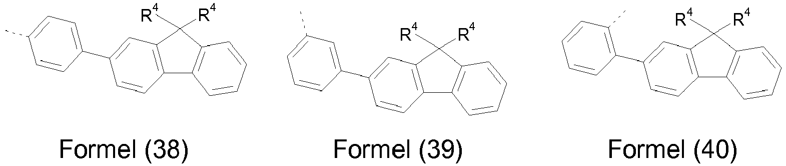 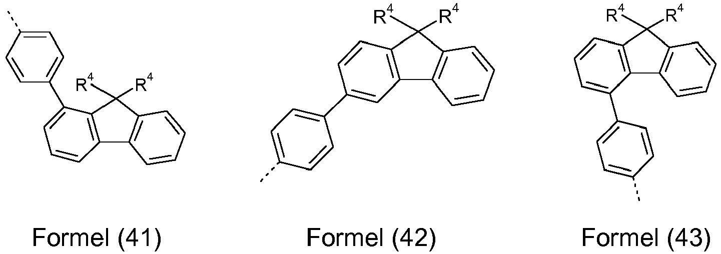 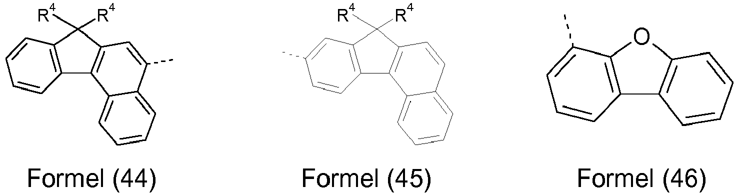 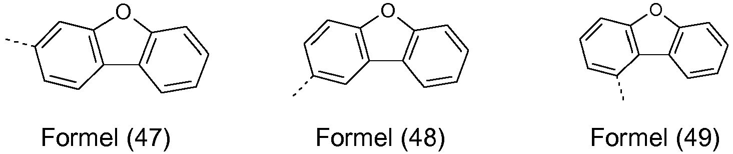 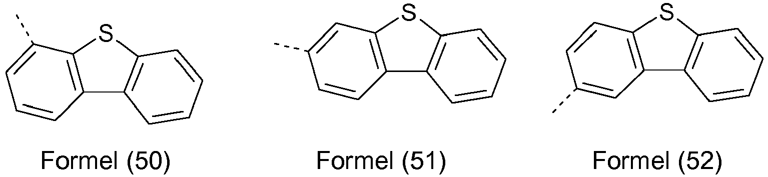 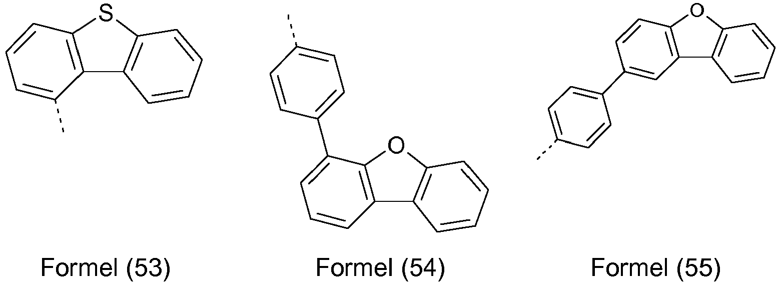 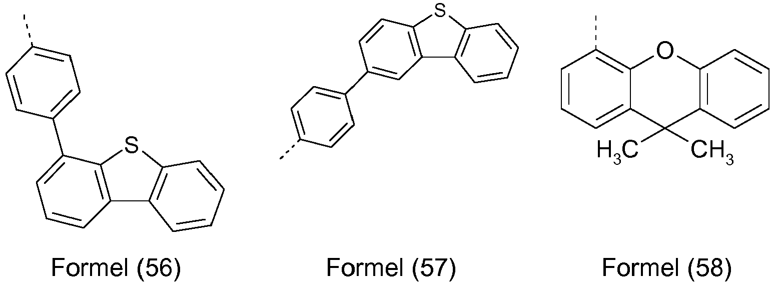 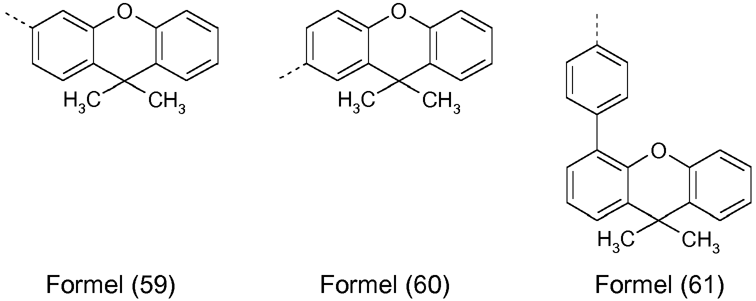 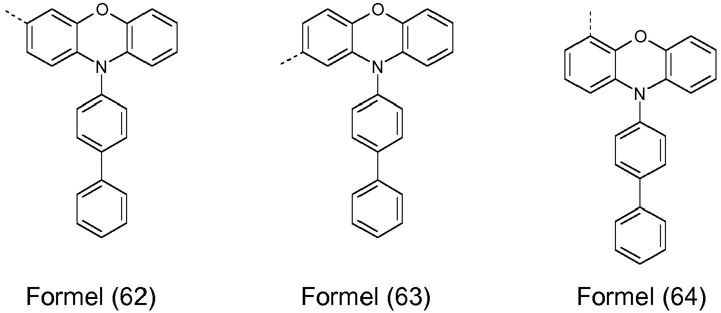 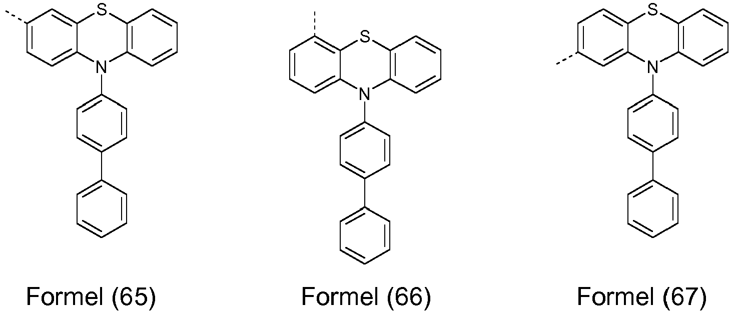 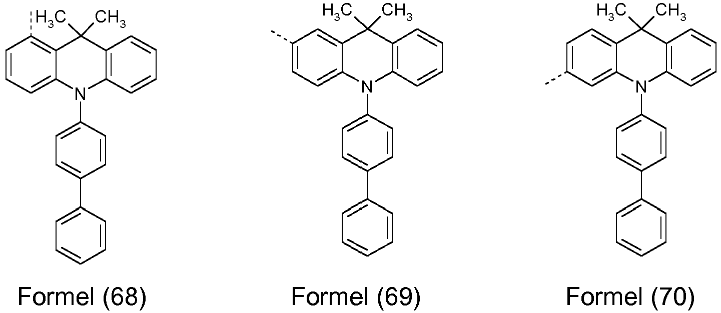 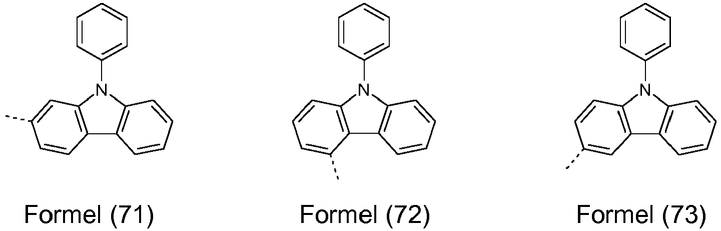 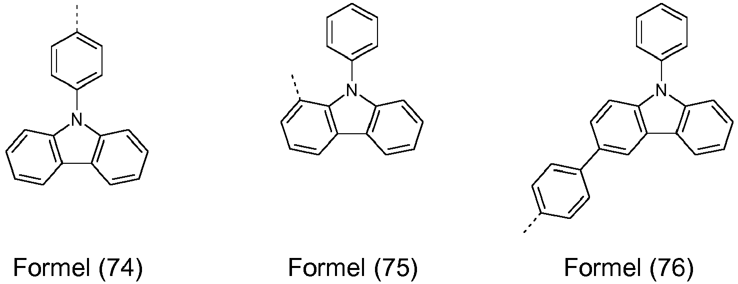 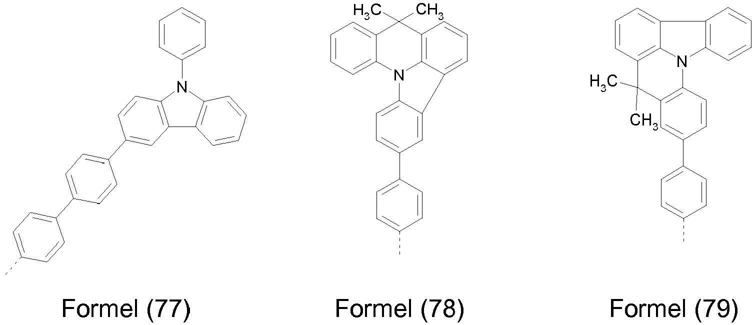 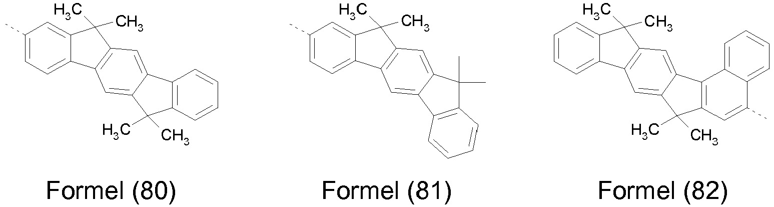 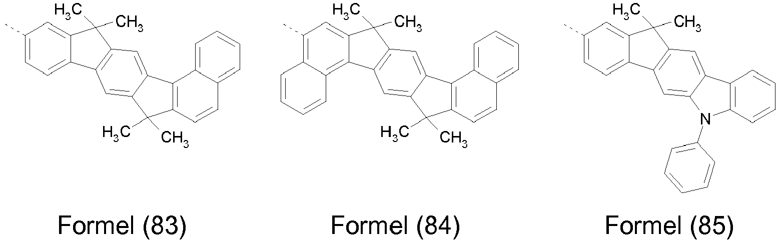 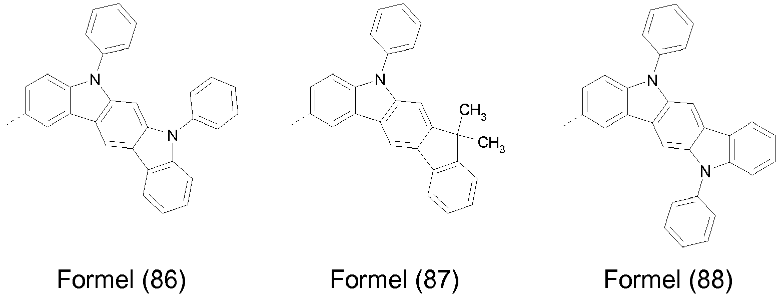 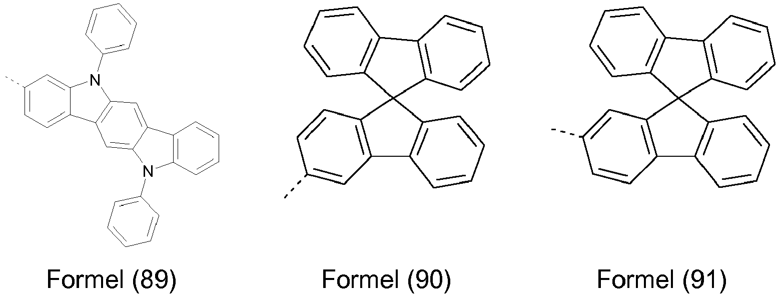 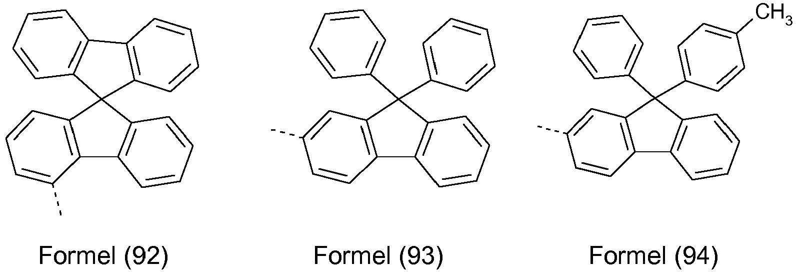 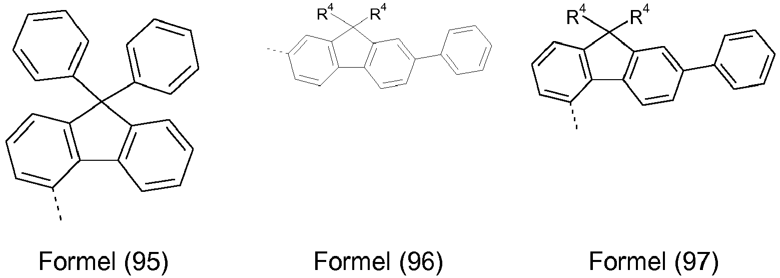 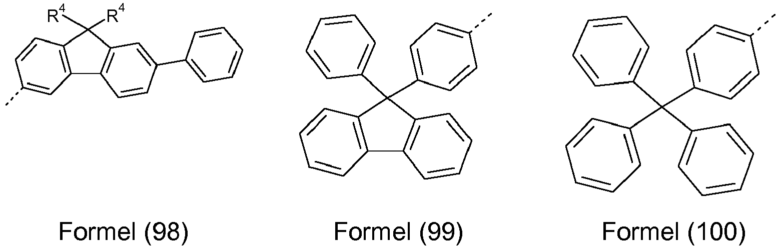
wobei die gestrichelte Linie die Bindungsposition darstellt und wobei die Strukturen mit einem oder mehreren Resten R⁴ substituiert sein können;
R¹ ist bei jedem Auftreten gleich oder verschieden H, D, F, Cl, Br, I, C(=O)R², CN, Si(R²)₃, NO₂, P(=O)(R²)₂, S(=O)R², S(=O)₂R², eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R² substituiert sein können und wobei eine oder mehrere CH₂-Gruppen in den oben genannten Gruppen durch -R²C=CR²-, -C≡C-, Si(R²)₂, C=O, C=S, C=NR², -C(=O)O-, -C(=O)NR²⁻, P(=O)(R²), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches Ringsystem mit 6 bis 30 aromatischen Ringatomen, das jeweils durch einen oder mehrere Reste R² substituiert sein kann, wobei zwei oder mehr Reste R¹ miteinander verknüpft sein können und einen Ring bilden können;
R⁴ ist bei jedem Auftreten gleich oder verschieden H, D, F, Cl, Br, I, C(=O)R², CN, Si(R²)₃, NR², NO₂, P(=O)(R²)₂, S(=O)R², S(=O)₂R², eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen, wobei die oben genannten Gruppen jeweils mit einem oder mehreren Resten R² substituiert sein können und wobei eine oder mehrere CH₂-Gruppen in den oben genannten Gruppen durch Si(R²)₂, C=O, C=S, C=NR², -C(=O)O-, -C(=O)NR²⁻, P(=O)(R²), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches Ringsystem mit 6 bis 30 aromatischen Ringatomen, das jeweils durch einen oder mehrere Reste R² substituiert sein kann, wobei zwei oder mehr Reste R⁴ miteinander verknüpft sein können und einen Ring bilden können;
R² ist bei jedem Auftreten gleich oder verschieden H, D, F, Cl, Br, I, C(=O)R³, CN, Si(R³)₃, NO₂, P(=O)(R³)₂, S(=O)R³, S(=O)₂R³, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R³ substituiert sein können und wobei eine oder mehrere CH₂-Gruppen in den oben genannten Gruppen durch -R³C=CR³⁻, -C≡C-, Si(R³)₂, C=O, C=S, C=NR³, -C(=O)O-, -C(=O)NR³-, P(=O)(R³), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 30 aromatischen Ringatomen, das jeweils durch einen oder mehrere Reste R³ substituiert sein kann, oder eine Aryloxy- oder Heteroaryloxygruppe mit 5 bis 30 aromatischen Ringatomen, die durch einen oder mehrere Reste R³ substituiert sein kann, wobei zwei oder mehr Reste R² miteinander verknüpft sein können und einen Ring bilden können;
R³ ist bei jedem Auftreten gleich oder verschieden H, D, F oder ein aliphatischer, aromatischer oder heteroaromatischer organischer Rest mit 1 bis 20 C-Atomen, in dem auch ein oder mehrere H-Atome durch D oder F ersetzt sein können; dabei können zwei oder mehr Substituenten R³ miteinander verknüpft sein und einen Ring bilden;
wobei die Verbindung nach Formel (1) neben dem Phenanthren keine weiteren kondensierten aromatische oder heteroaromatische Ring mit mehr als 10 Ringatomen enthält und
wobei die Reste R¹ am Phenanthren in Formel (1) keine weiteren Amingruppen enthalten.

2. Verbindung nach Anspruch 1, **dadurch gekennzeichnet, dass** R¹ in den Positionen 1, 2 und 4-8 des Phenanthrens in der Verbindung der Formel (1) gleich H ist.

3. Verbindung nach einem oder mehreren der Ansprüche 1 bis 3, **dadurch gekennzeichnet, dass** R¹ in den Positionen 1, 2 und 4-10 des Phenanthrens in der Verbindung der Formel (1) gleich H ist.

4. Verbindung nach einem oder mehreren der Ansprüche 1 bis 3, **dadurch gekennzeichnet, dass** die Verbindung insgesamt wenigstens 26, bevorzugt 32, ganz bevorzugt 38 und ganz besonders bevorzugt 42 Ringatome enthält.

5. Verbindung nach einem oder meherern der Ansprüche 1 bis 4, **dadurch gekennzeichnet, dass** sie nur eine Amingruppe enthält.

6. Verfahren zur Herstellung einer Verbindung nach einem oder mehreren der Ansprüche 1 bis 5 mittels einstufiger Buchwald Kupplung durch Umsetzung eines Phenanthrenderivats, das eine Abgangsgruppe enthält, mit Ar²-NH-Ar¹.

7. Verfahren zur Herstellung einer Verbindung nach einem oder mehreren der Ansprüche 1 bis 5 mittels zweistufiger Buchwald Kupplung durch stufenweise Umsetzung eines Phenanthrenderivats, das eine Abgangsgruppe enthält, mit (1) Ar²-NH₂ und (2) D-Ar¹, wobei D eine Abgangsgruppe darstellt.

8. Oligomer, Polymer oder Dendrimer enthaltend eine oder mehrere Verbindungen nach einem oder mehreren der Ansprüche 1 bis 5, wobei die Bindung(en) zum Polymer, Oligomer oder Dendrimer an beliebigen, in Formel (1) mit R¹, R⁴ oder R² substituierten Positionen lokalisiert sein können.

9. Zusammensetzung enthaltend eine oder meherer Verbindung nach einem oder mehreren der Ansprüche 1 bis 5 und wenigstens ein weiteres organisch funktionelles Material ausgewählt aus der Gruppe bestehend aus fluoreszierenden Emitter, phosphoreszierenden Emitter, Host Materialien, Matrix Materialien, Elektronentransportmaterialien, Elektroneninjektionsmaterialien, Lochleitermaterialien, Lochinjektionsmaterialien, Elektronenblockiermaterialien und Lochblockiermaterial ien.

10. Formulierung enthaltend mindestens eine Verbindung nach einem oder mehreren der Ansprüche 1 bis 5 oder mindestens ein Polymer, Oligomer oder Dendrimer gemäß Anspruch 8 oder mindestens eine Zusammensetzung nach Anspruch 9 und mindestens ein Lösungsmittel.

11. Elektronische Vorrichtung, enthaltend mindestens eine Verbindung gemäß einem oder mehreren der Ansprüche 1 bis 5 oder mindestens ein Polymer, Oligomer oder Dendrimer gemäß Anspruch 8 oder mindestens eine Zusammensetzung nach Anspruch 9.

12. Elektronische Vorrichtung gemäß Anspruch 11, **dadurch gekennzeichnet, dass** sie ausgewählt ist aus organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und organischen Elektrolumineszenzvorrichtungen (OLEDs).

13. Elektronische Vorrichtung gemäß Anspruch 11, welche ausgewählt ist aus der Gruppe der organischen Elektrolumineszenzvorrichtungen, insbesondere eine organische lichtemittierende Diode (OLED), **dadurch gekennzeichnet, dass** die Verbindung gemäß einem oder mehreren der Ansprüche 1 bis 5 oder das Polymer, Oligomer oder Dendrimer gemäß Anspruch 8 oder eine Zusammensetzung nach Anspruch 9 in einer oder mehreren der folgenden Funktionen eingesetzt wird:
- als Lochtransportmaterial in einer Lochtransport- oder Lochinjektionsschicht,
- als Matrixmaterial in einer emittierenden Schicht,
- als Elektronenblockiermaterial,
- als Excitonenblockiermaterial.

## Claims

1. Compound of the general formula (1) 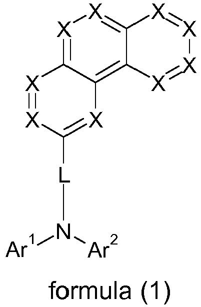 where the following applies to the symbols and indices occurring:
X is on each occurrence CR¹;
L is a single bond;
Ar¹, Ar² are selected on each occurrence, identically or differently, from the following groups, where at least one of the radicals Ar¹ and Ar² contains more than one ring 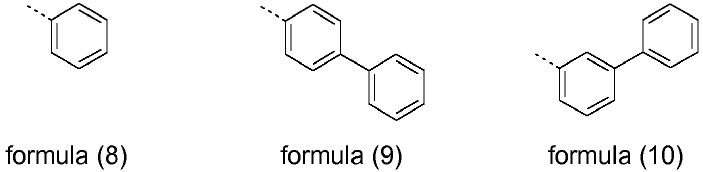 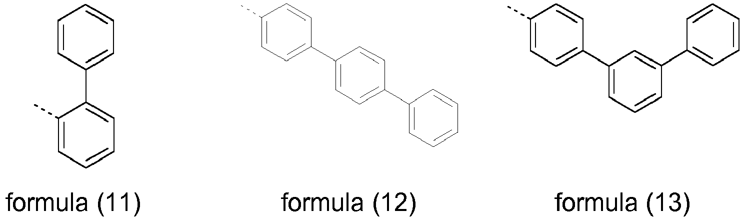 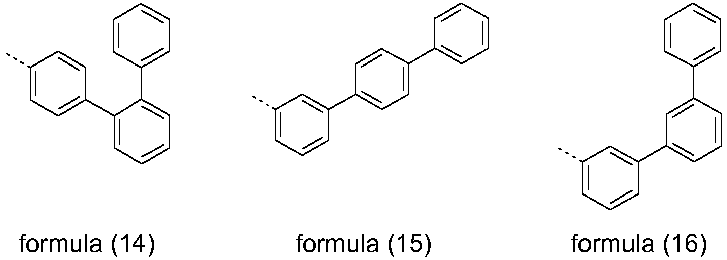 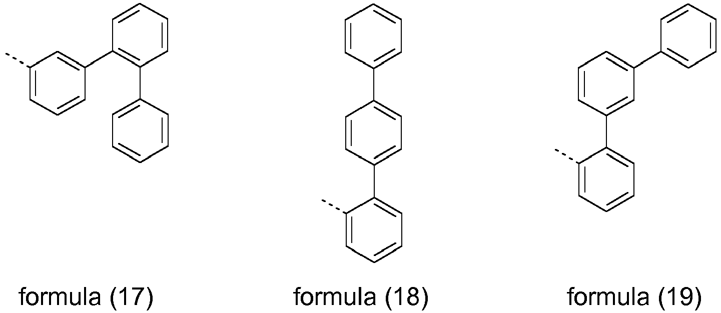 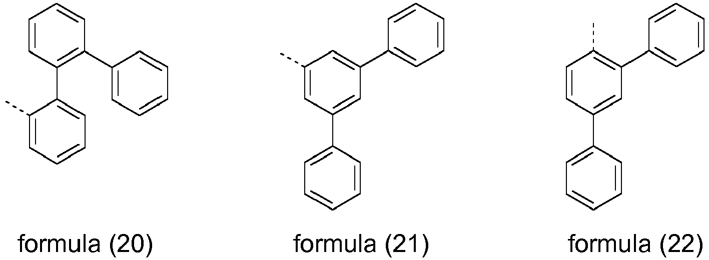 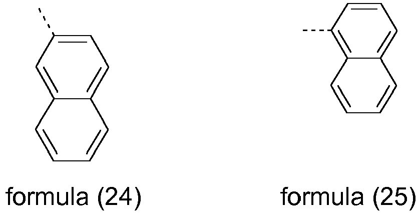 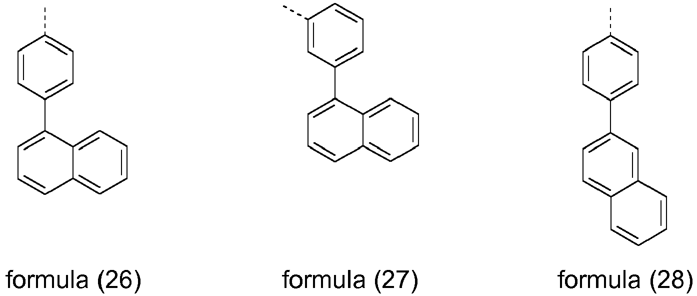 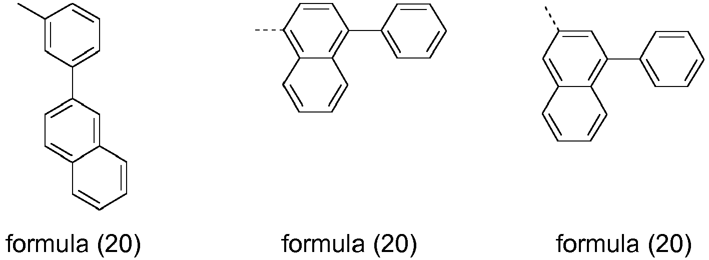 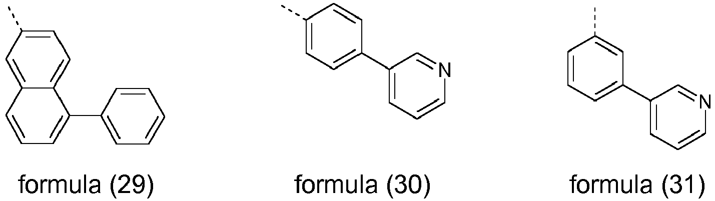 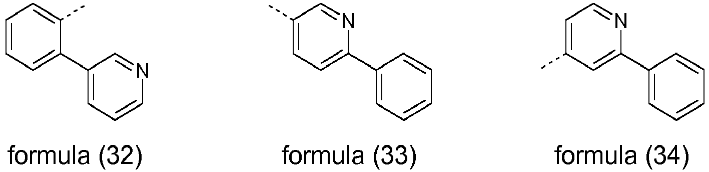 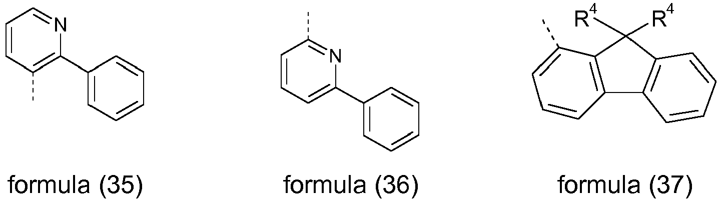 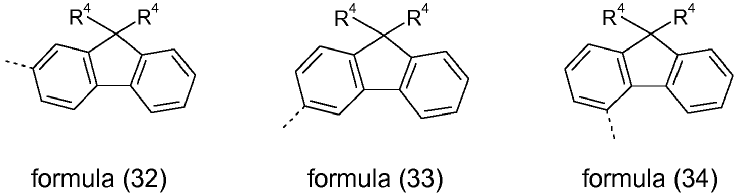 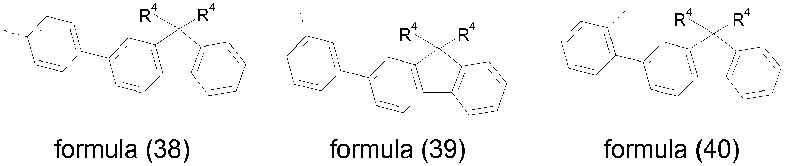 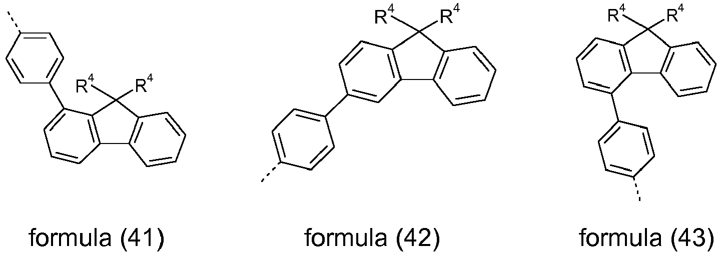 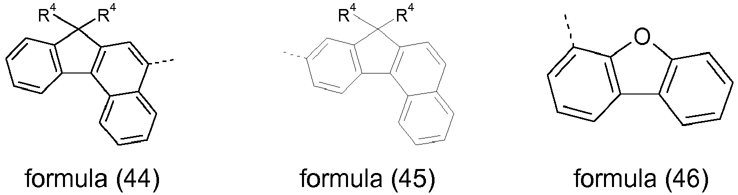 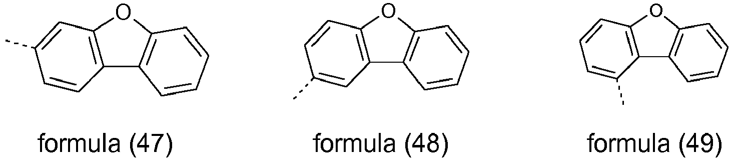 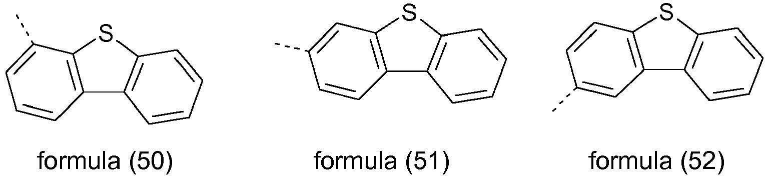 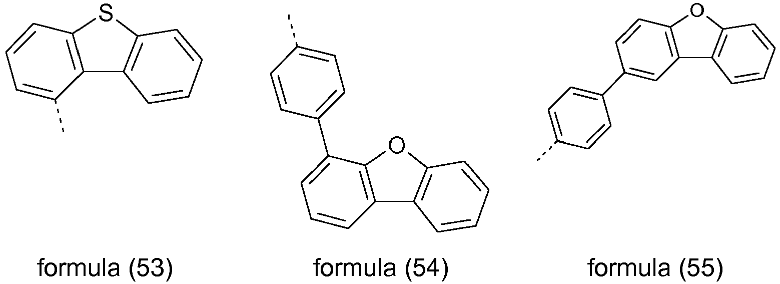 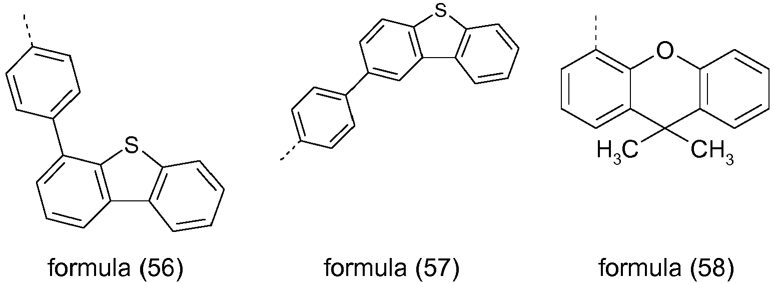 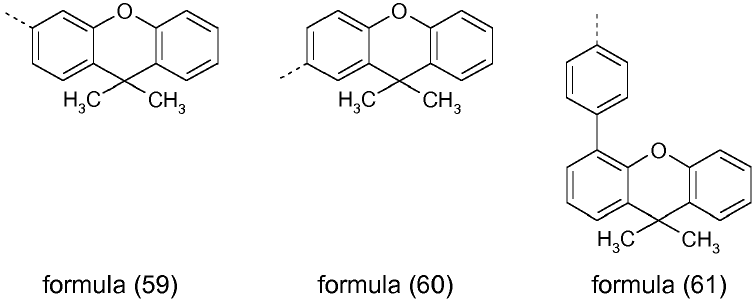 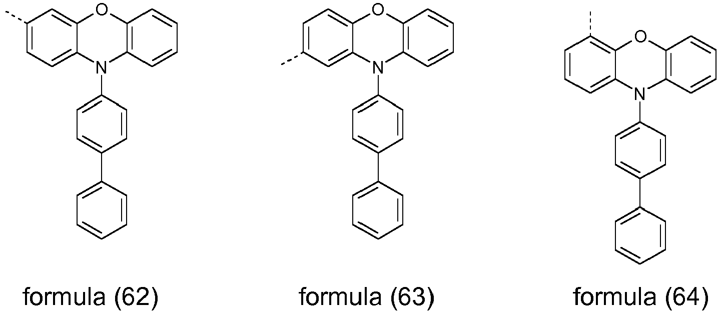 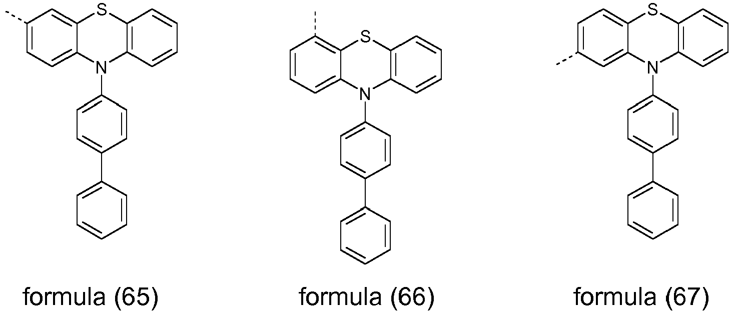 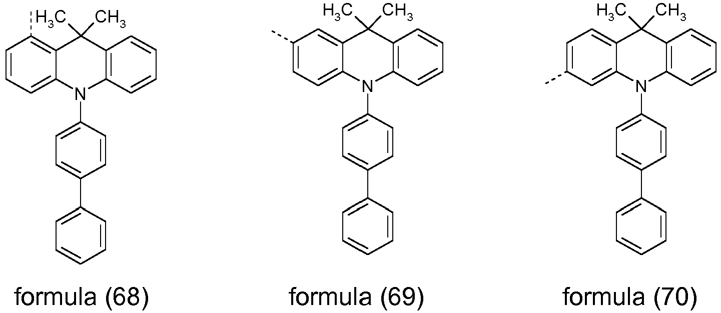 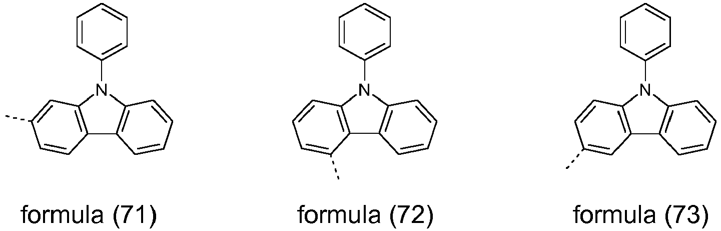 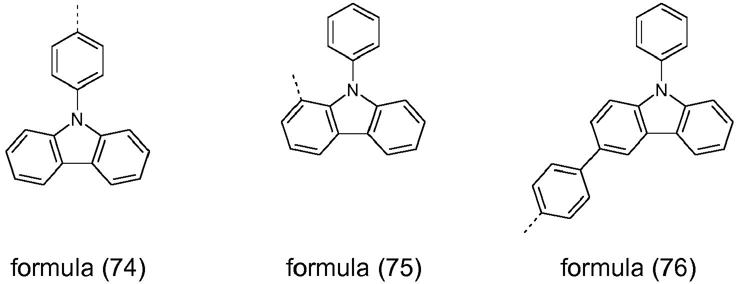 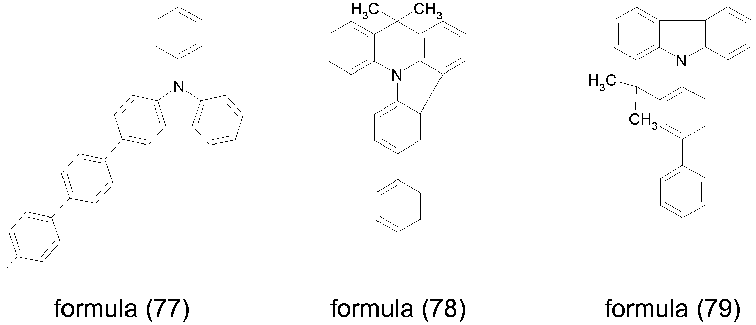 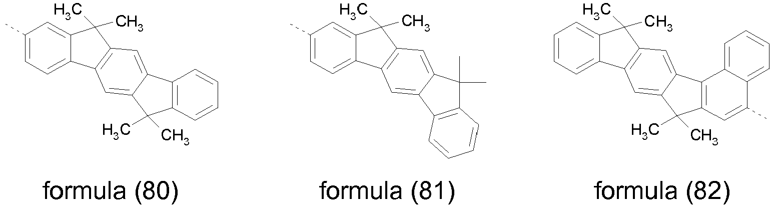 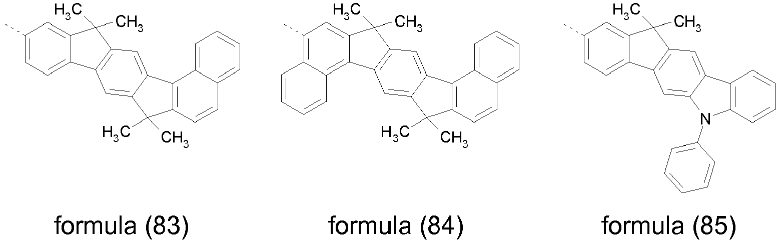 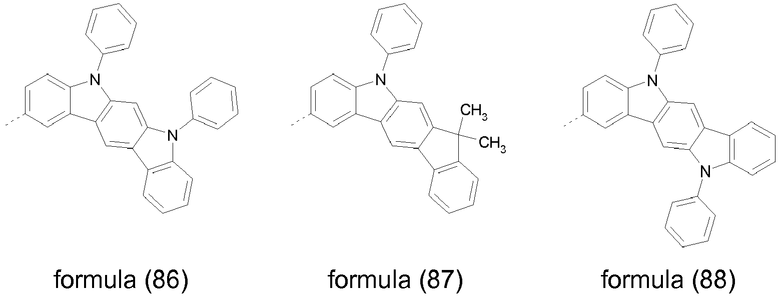 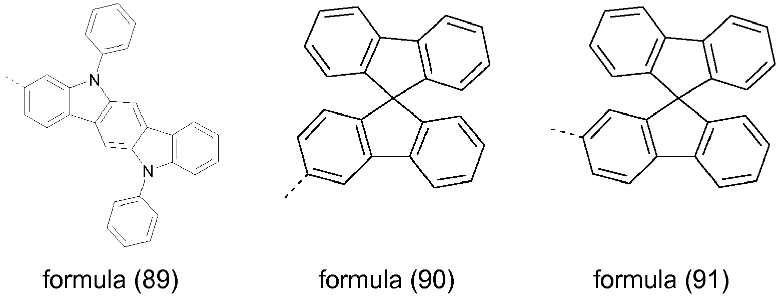 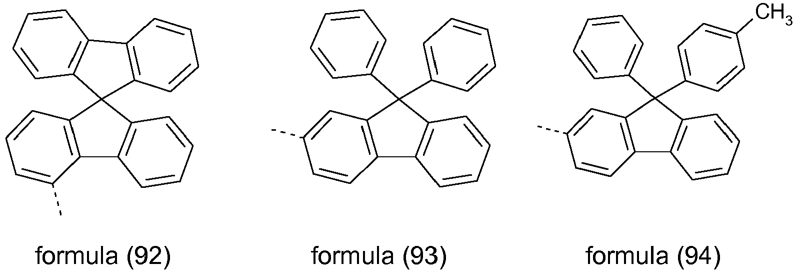 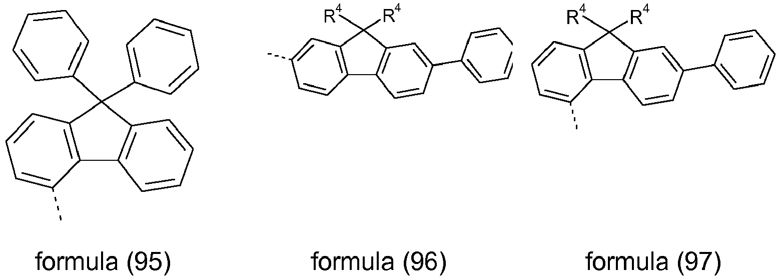 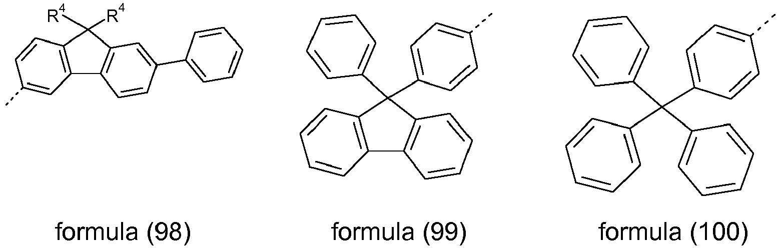
where the dashed line represents the bonding position and where the structures may be substituted by one or more radicals R⁴;
R¹ is on each occurrence, identically or differently, H, D, F, Cl, Br, I, C(=O)R², CN, Si(R²)₃, NO₂, P(=O)(R²)₂, S(=O)R², S(=O)₂R², a straight-chain alkyl, alkoxy or thioalkyl group having 1 to 20 C atoms or a branched or cyclic alkyl, alkoxy or thioalkyl group having 3 to 20 C atoms or an alkenyl or alkynyl group having 2 to 20 C atoms, where the above-mentioned groups may in each case be substituted by one or more radicals R² and where one or more CH₂ groups in the above-mentioned groups may be replaced by -R²C=CR²-, -C≡C-, Si(R²)₂, C=O, C=S, C=NR², -C(=O)O-, -C(=O)NR²-, P(=O)(R²), -O-, -S-, SO or SO₂ and where one or more H atoms in the above-mentioned groups may be replaced by D, F, Cl, Br, I, CN or NO₂, or an aromatic ring system having 6 to 30 aromatic ring atoms, which may in each case be substituted by one or more radicals R², where two or more radicals R¹ may be linked to one another and may form a ring;
R⁴ is on each occurrence, identically or differently, H, D, F, Cl, Br, I, C(=O)R², CN, Si(R²)₃, NR², NO₂, P(=O)(R²)₂, S(=O)R², S(=O)₂R², a straight-chain alkyl, alkoxy or thioalkyl group having 1 to 20 C atoms or a branched or cyclic alkyl, alkoxy or thioalkyl group having 3 to 20 C atoms, where the above-mentioned groups may in each case be substituted by one or more radicals R² and where one or more CH₂ groups in the above-mentioned groups may be replaced by Si(R²)₂, C=O, C=S, C=NR², -C(=O)O-, -C(=O)NR²-, P(=O)(R²), -O-, -S-, SO or SO₂ and where one or more H atoms in the above-mentioned groups may be replaced by D, F, Cl, Br, I, CN or NO₂, or an aromatic ring system having 6 to 30 aromatic ring atoms, which may in each case be substituted by one or more radicals R², where two or more radicals R⁴ may be linked to one another and may form a ring;
R² is on each occurrence, identically or differently, H, D, F, Cl, Br, I, C(=O)R³, CN, Si(R³)₃, NO₂, P(=O)(R³)₂, S(=O)R³, S(=O)₂R³, a straight-chain alkyl, alkoxy or thioalkyl group having 1 to 20 C atoms or a branched or cyclic alkyl, alkoxy or thioalkyl group having 3 to 20 C atoms or an alkenyl or alkynyl group having 2 to 20 C atoms, where the above-mentioned groups may in each case be substituted by one or more radicals R³ and where one or more CH₂ groups in the above-mentioned groups may be replaced by -R³C=CR³-, -C≡C-, Si(R³)₂, C=O, C=S, C=NR³, -C(=O)O-, -C(=O)NR³-, P(=O)(R³), -O-, -S-, SO or SO₂ and where one or more H atoms in the above-mentioned groups may be replaced by D, F, Cl, Br, I, CN or NO₂, or an aromatic or heteroaromatic ring system having 5 to 30 aromatic ring atoms, which may in each case be substituted by one or more radicals R³, or an aryloxy or heteroaryloxy group having 5 to 30 aromatic ring atoms, which may be substituted by one or more radicals R³, where two or more radicals R² may be linked to one another and may form a ring;
R³ is on each occurrence, identically or differently, H, D, F or an aliphatic, aromatic or heteroaromatic organic radical having 1 to 20 C atoms, in which, in addition, one or more H atoms may be replaced by D or F; two or more substituents R³ may be linked to one another and may form a ring;
where the compound of the formula (1), besides the phenanthrene, contains no further condensed aromatic or heteroaromatic ring having more than 10 ring atoms and
where the radicals R¹ on the phenanthrene in formula (1) contain no further amine groups.

2. Compound according to Claim 1, **characterised in that** R¹ in positions 1, 2 and 4-8 of the phenanthrene in the compound of the formula (1) is equal to H.

3. Compound according to one or more of Claims 1 to 3, **characterised in that** R¹ in positions 1, 2 and 4-10 of the phenanthrene in the compound of the formula (1) is equal to H.

4. Compound according to one or more of Claims 1 to 3, **characterised in that** the compound contains in total at least 26, preferably 32, very preferably 38 and very particularly preferably 42 ring atoms.

5. Compound according to one or more of Claims 1 to 4, **characterised in that** it contains only one amine group.

6. Process for the preparation of a compound according to one or more of Claims 1 to 5 by means of one-step Buchwald coupling by reaction of a phenanthrene derivative which contains a leaving group with Ar²-NH-Ar¹.

7. Process for the preparation of a compound according to one or more of Claims 1 to 5 by means of two-step Buchwald coupling by stepwise reaction of a phenanthrene derivative which contains a leaving group with (1) Ar²-NH₂ and (2) D-Ar¹, where D represents a leaving group.

8. Oligomer, polymer or dendrimer containing one or more compounds according to one or more of Claims 1 to 5, where the bond(s) to the polymer, oligomer or dendrimer may be localised at any desired positions in formula (1) that are substituted by R¹, R⁴ or R².

9. Composition comprising one or more compounds according to one or more of Claims 1 to 5 and at least one further organically functional material selected from the group consisting of fluorescent emitters, phosphorescent emitters, host materials, matrix materials, electron-transport materials, electron-injection materials, hole-conductor materials, hole-injection materials, electron-blocking materials and hole-blocking materials.

10. Formulation comprising at least one compound according to one or more of Claims 1 to 5 or at least one polymer, oligomer or dendrimer according to Claim 8 or at least one composition according to Claim 9 and at least one solvent.

11. Electronic device containing at least one compound according to one or more of Claims 1 to 5 or at least one polymer, oligomer or dendrimer according to Claim 8 or at least one composition according to Claim 9.

12. Electronic device according to Claim 11, **characterised in that** it is selected from organic integrated circuits (O-ICs), organic field-effect transistors (O-FETs), organic thin-film transistors (O-TFTs), organic light-emitting transistors (O-LETs), organic solar cells (O-SCs), organic optical detectors, organic photoreceptors, organic field-quench devices (O-FQDs), light-emitting electrochemical cells (LECs), organic laser diodes (O-lasers) and organic electroluminescent devices (OLEDs).

13. Electronic device according to Claim 11 which is selected from the group of organic electroluminescent devices, in particular an organic light-emitting diode (OLED), **characterised in that** the compound according to one or more of Claims 1 to 5 or the polymer, oligomer or dendrimer according to Claim 8 or a composition according to Claim 9 is employed in one or more of the following functions:
- as hole-transport material in a hole-transport or hole-injection layer,
- as matrix material in an emitting layer,
- as electron-blocking material,
- as exciton-blocking material.

## Revendications

1. Composé de la formule générale (1) : 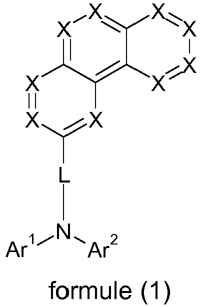 dans laquelle ce qui suit s'applique aux symboles et aux indices qui sont rencontrés :
X est pour chaque occurrence CR¹ ;
L est une liaison simple ;
Ar¹ , Ar² sont sélectionnés pour chaque occurrence, de manière identique ou différente, parmi les groupes qui suivent, dans lesquels au moins l'un des radicaux Ar¹ et Ar² contient plus d'un cycle : 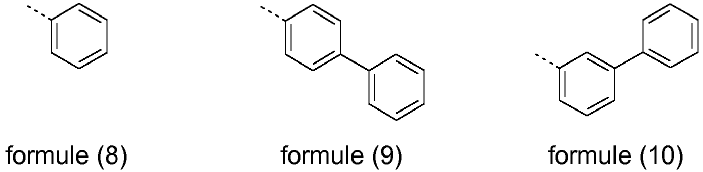 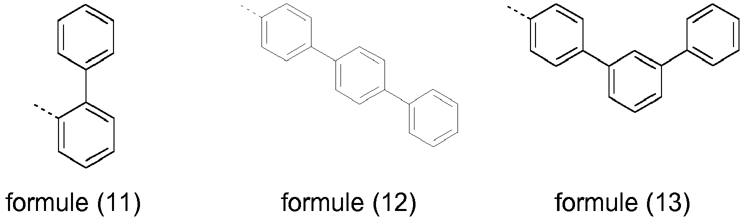 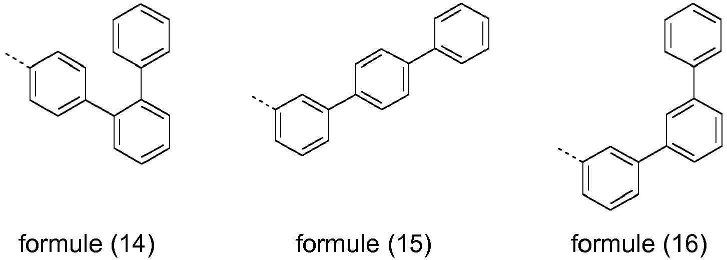 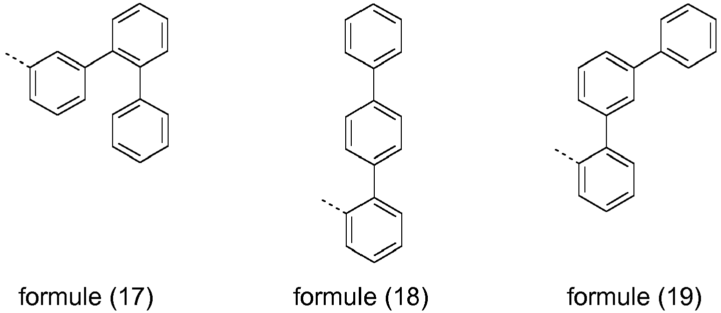 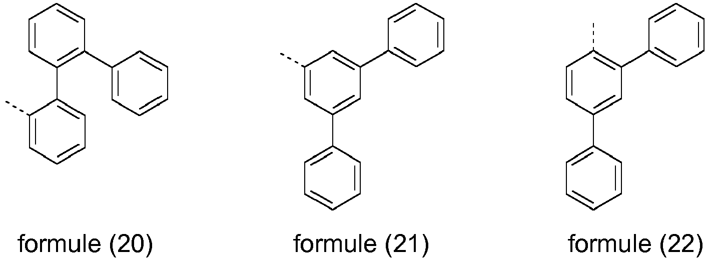 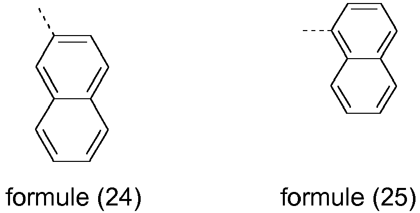 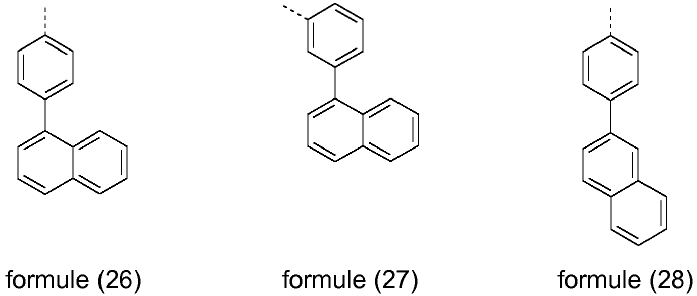 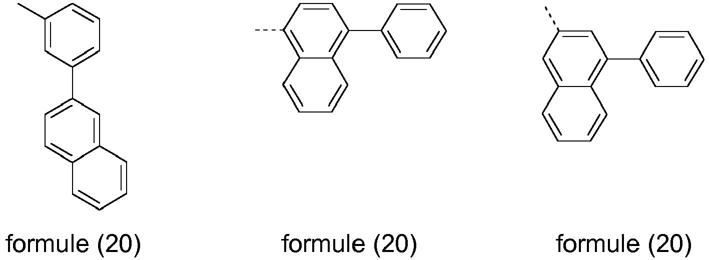 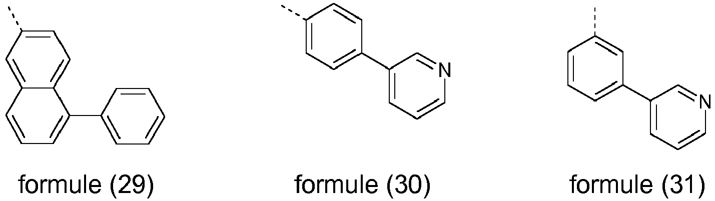 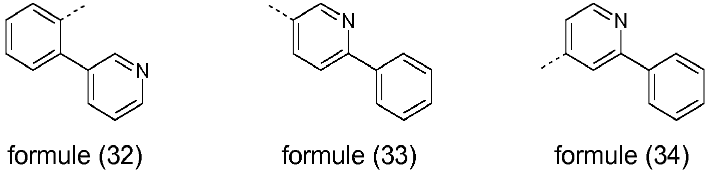 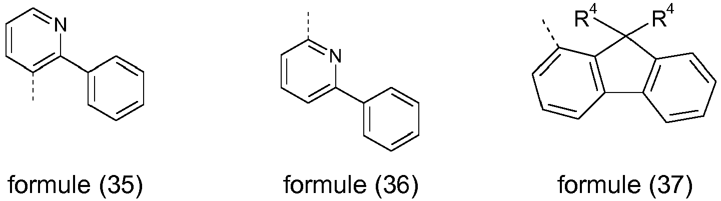 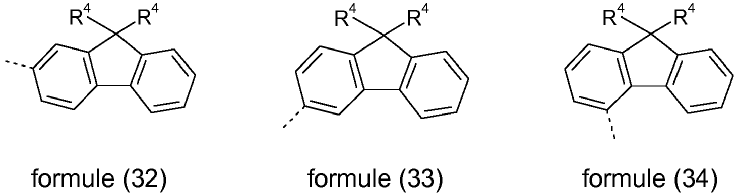 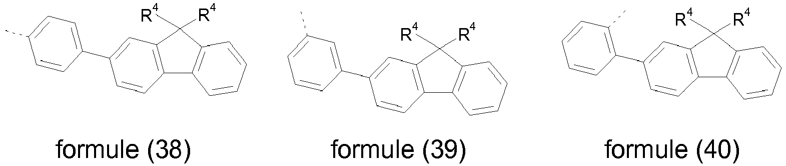 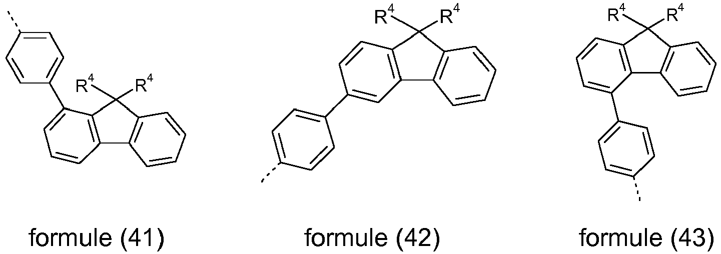 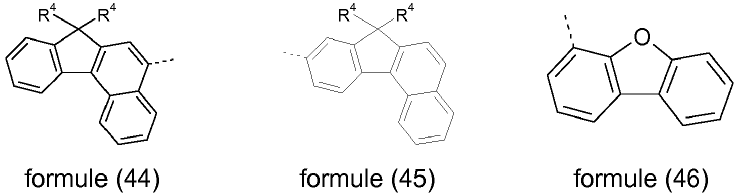 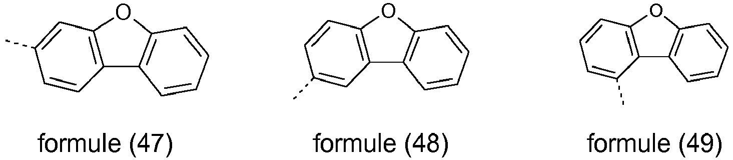 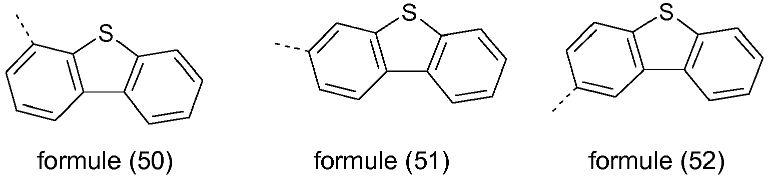 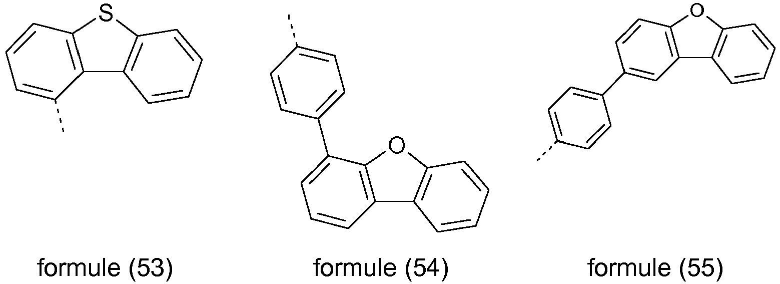 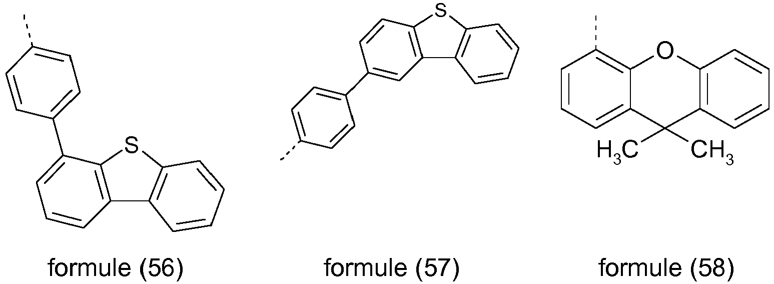 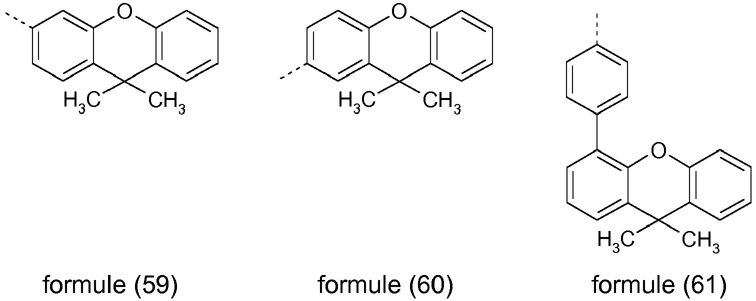 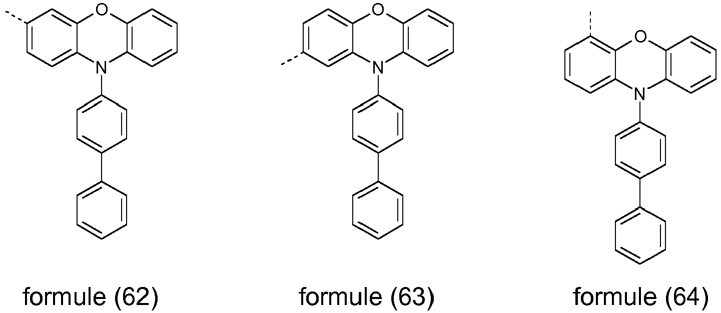 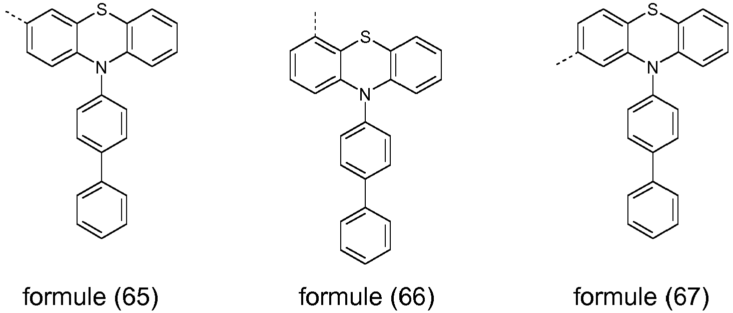 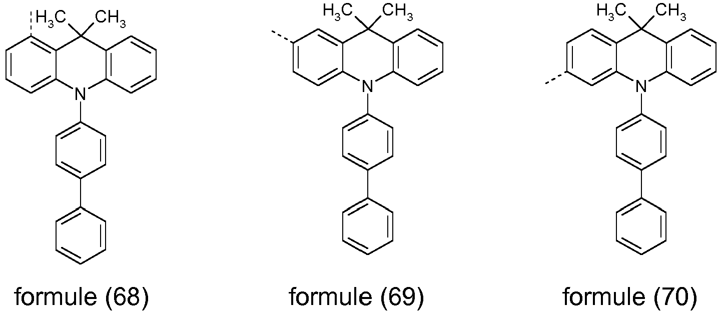 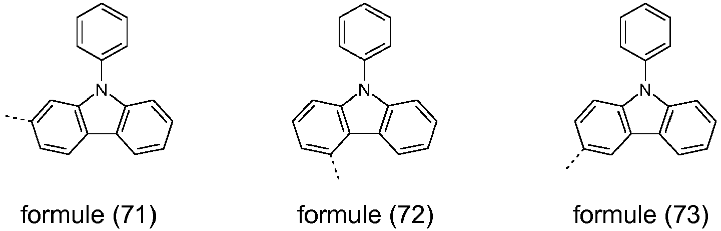 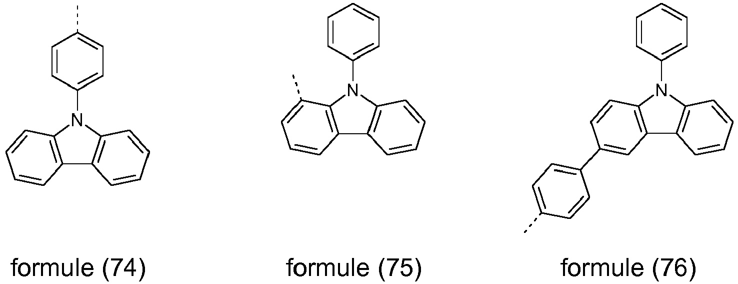 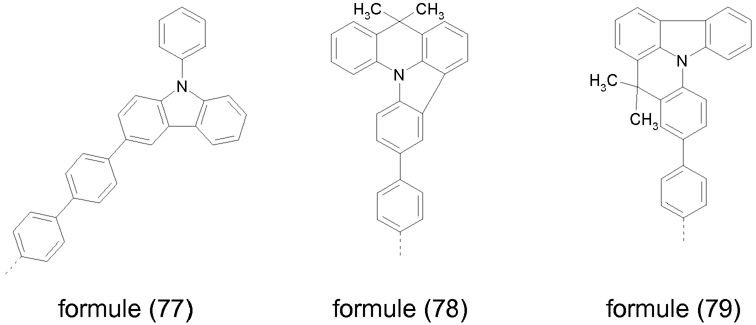 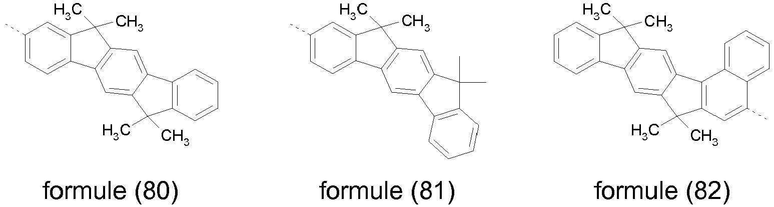 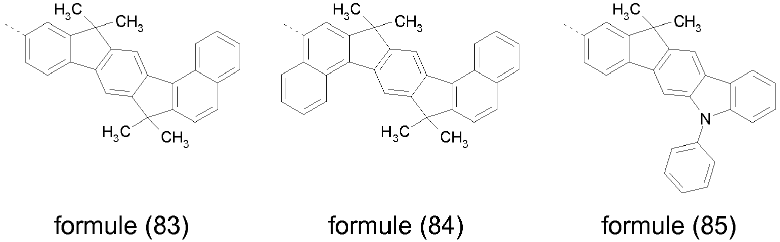 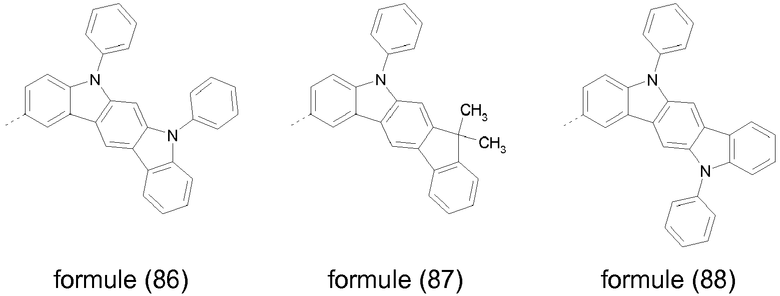 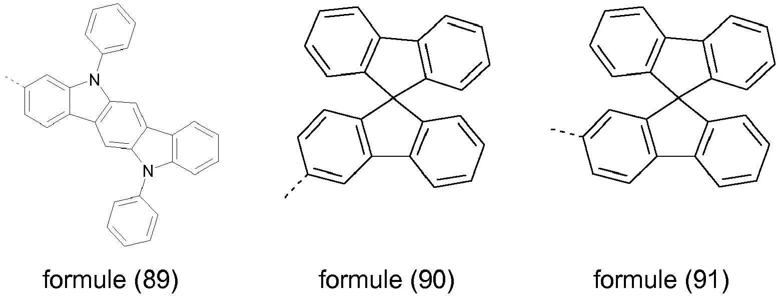 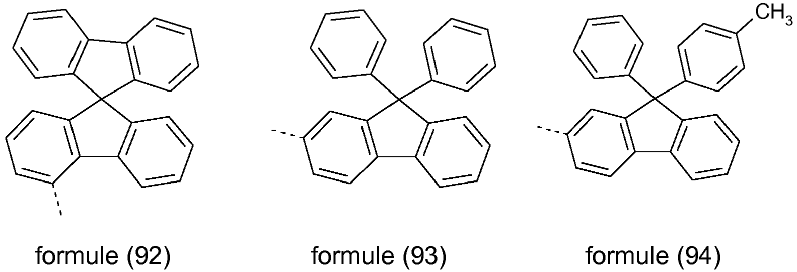 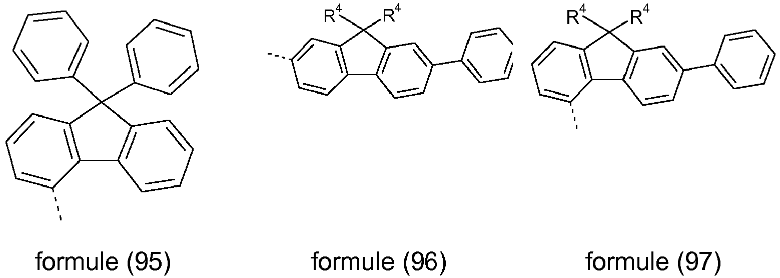 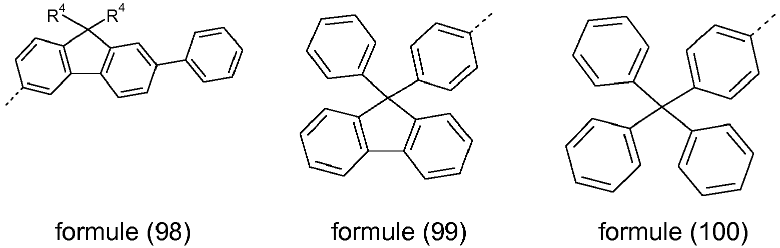
dans lesquels la ligne en pointillés représente la position de liaison et dans lesquels les structures peuvent être substituées par un radical ou par plusieurs radicaux R⁴ ;
R¹ est pour chaque occurrence, de manière identique ou différente, H, D, F, Cl, Br, I, C(=O)R², CN, Si(R²)₃, NO₂, P(=O)(R²)₂, S(=O)R², S(=O)₂R², un groupe alkyle, alcoxy ou thioalkyle en chaîne droite qui comporte de 1 à 20 atome(s) de C ou un groupe alkyle, alcoxy ou thioalkyle ramifié ou cyclique qui comporte de 3 à 20 atomes de C ou un groupe alkényle ou alkynyle qui comporte de 2 à 20 atomes de C, où les groupes qui ont été mentionnés ci-avant peuvent dans chaque cas être substitués par un radical ou par plusieurs radicaux R² et où un ou plusieurs groupe(s) CH₂ dans les groupes qui ont été mentionnés ci-avant peut/peuvent être remplacé(s) par -R²C=CR²-, -C≡C-, Si(R²)₂, C=O, C=S, C=NR², -C(=O)O-, -C(=O)NR²⁻, P(=O)(R²), -O-, -S-, SO ou SO₂ et où un ou plusieurs atome(s) de H dans les groupes qui ont été mentionnés ci-avant peut/peuvent être remplacé(s) par D, F, Cl, Br, I, CN ou NO₂, ou un système de cycle aromatique qui comporte de 6 à 30 atomes de cycle aromatique, lequel peut dans chaque cas être substitué par un radical ou par plusieurs radicaux R², où deux radicaux R¹ ou plus peuvent être liés l'un à l'autre ou les uns aux autres et peuvent former un cycle ;
R⁴ est pour chaque occurrence, de manière identique ou différente, H, D, F, Cl, Br, I, C(=O)R², CN, Si(R²)₃, NR², NO₂, P(=O)(R²)₂, S(=O)R², S(=O)₂R², un groupe alkyle, alcoxy ou thioalkyle en chaîne droite qui comporte de 1 à 20 atome(s) de C ou un groupe alkyle, alcoxy ou thioalkyle ramifié ou cyclique qui comporte de 3 à 20 atomes de C, où les groupes qui ont été mentionnés ci-avant peuvent dans chaque cas être substitués par un radical ou par plusieurs radicaux R² et où un ou plusieurs groupe(s) CH₂ dans les groupes qui ont été mentionnés ci-avant peut/peuvent être remplacé(s) par Si(R²)₂, C=O, C=S, C=NR², -C(=O)O-, -C(=O)NR²-, P(=O)(R²), -O-, -S-, SO ou SO₂ et où un ou plusieurs atome(s) de H dans les groupes qui ont été mentionnés ci-avant peut/peuvent être remplacé(s) par D, F, Cl, Br, I, CN ou NO₂, ou un système de cycle aromatique qui comporte de 6 à 30 atomes de cycle aromatique, lequel peut dans chaque cas être substitué par un radical ou par plusieurs radicaux R², où deux radicaux R⁴ ou plus peuvent être liés l'un à l'autre ou les uns aux autres et peuvent former un cycle ;
R² est pour chaque occurrence, de manière identique ou différente, H, D, F, Cl, Br, I, C(=O)R³, CN, Si(R³)₃, NO₂, P(=O)(R³)₂, S(=O)R³, S(=O)₂R³, un groupe alkyle, alcoxy ou thioalkyle en chaîne droite qui comporte 1 à 20 atome(s) de C ou un groupe alkyle, alcoxy ou thioalkyle ramifié ou cyclique qui comporte de 3 à 20 atomes de C ou un groupe alkényle ou alkynyle qui comporte de 2 à 20 atomes de C, où les groupes qui ont été mentionnés ci-avant peuvent dans chaque cas être substitués par un radical ou par plusieurs radicaux R³ et où un ou plusieurs groupe(s) CH₂ dans les groupes qui ont été mentionnés ci-avant peut/peuvent être remplacé(s) par -R³C=CR³-, -C≡C-, Si(R³)₂, C=O, C=S, C=NR³, -C(=O)O-, -C(=O)NR³-, P(=O)(R³), -O-, -S-, SO ou SO₂ et où un ou plusieurs atome(s) de H dans les groupes qui ont été mentionnés ci-avant peut/peuvent être remplacé(s) par D, F, Cl, Br, I, CN ou NO₂, ou un système de cycle aromatique ou hétéroaromatique qui comporte de 5 à 30 atomes de cycle aromatique, lequel peut dans chaque cas être substitué par un radical ou par plusieurs radicaux R³, ou un groupe aryloxy ou hétéroaryloxy qui comporte de 5 à 30 atomes de cycle aromatique, lequel peut être substitué par un radical ou par plusieurs radicaux R³, où deux radicaux R² ou plus peuvent être liés l'un à l'autre ou les uns aux autres et peuvent former un cycle ;
R³ est pour chaque occurrence, de manière identique ou différente, H, D, F ou un radical organique aliphatique, aromatique ou hétéroaromatique qui comporte de 1 à 20 atome(s) de C, où, en outre, un ou plusieurs atome(s) de H peut/peuvent être remplacé(s) par D ou F ; deux substituants R³ ou plus peuvent être liés l'un à l'autre ou les uns aux autres et peuvent former un cycle ;
où le composé de la formule (1), à part le phénanthrène, ne contient pas un autre cycle aromatique ou hétéroaromatique condensé qui comporte plus de 10 atomes de cycle ; et
où les radicaux R¹ sur le phénanthrène dans la formule (1) ne contiennent pas d'autres groupes amine.

2. Composé selon la revendication 1, **caractérisé en ce que** R¹ au niveau des positions 1, 2 et 4 à 8 du phénanthrène dans le composé de la formule (1) est égal à H.

3. Composé selon une ou plusieurs des revendications 1 et 2, **caractérisé en ce que** R¹ au niveau des positions 1, 2 et 4 à 10 du phénanthrène dans le composé de la formule (1) est égal à H.

4. Composé selon une ou plusieurs des revendications 1 à 3, **caractérisé en ce que** le composé contient au total au moins 26 atomes de cycle, de préférence 32 atomes de cycle, de façon très préférable 38 atomes de cycle et de façon particulièrement très préférable, 42 atomes de cycle.

5. Composé selon une ou plusieurs des revendications 1 à 4, **caractérisé en ce qu'**il contient seulement un groupe amine.

6. Procédé pour la préparation d'un composé selon une ou plusieurs des revendications 1 à 5 au moyen d'un couplage de Buchwald à une seule étape au moyen de la réaction d'un dérivé de phénanthrène qui contient un groupe partant avec Ar²-NH-Ar¹.

7. Procédé pour la préparation d'un composé selon une ou plusieurs des revendications 1 à 5 au moyen d'un couplage de Buchwald à deux étapes au moyen d'une réaction par pas d'un dérivé de phénanthrène qui contient un groupe partant avec (1) Ar²-NH₂ et (2) D-Ar¹, où D représente un groupe partant.

8. Oligomère, polymère ou dendrimère contenant un ou plusieurs composé(s) selon une ou plusieurs des revendications 1 à 5, où la/les liaison(s) sur le polymère, l'oligomère ou le dendrimère peut/peuvent être localisée(s) au niveau de n'importe quelles positions souhaitées dans la formule (1) qui sont substituées par R¹, R⁴ ou R².

9. Composition comprenant un ou plusieurs composés selon une ou plusieurs des revendications 1 à 5 et au moins un autre matériau organiquement fonctionnel qui est sélectionné parmi le groupe qui est constitué par les émetteurs fluorescents, les émetteurs phosphorescents, les matériaux hôtes, les matériaux de matrice, les matériaux de transport d'électrons, les matériaux d'injection d'électrons, les matériaux conducteurs de trous, les matériaux d'injection de trous, les matériaux de blocage d'électrons et les matériaux de blocage de trous.

10. Formulation comprenant au moins un composé selon une ou plusieurs des revendications 1 à 5 ou au moins un polymère, un oligomère ou un dendrimère selon la revendication 8 ou au moins une composition selon la revendication 9 et au moins un solvant.

11. Dispositif électronique contenant au moins un composé selon une ou plusieurs des revendications 1 à 5 ou au moins un polymère, un oligomère ou un dendrimère selon la revendication 8 ou au moins une composition selon la revendication 9.

12. Dispositif électronique selon la revendication 11, **caractérisé en ce qu'**il est sélectionné parmi les circuits intégrés organiques (O-IC), les transistors à effet de champ organiques (O-FET), les transistors à film mince organiques (O-TFT), les transistors à émission de lumière organiques (O-LET), les cellules solaires organiques (O-SC), les détecteurs optiques organiques, les photorécepteurs organiques, les dispositifs à extinction de champ organiques (O-FQD), les cellules électrochimiques à émission de lumière (LEC), les diodes laser organiques (O-laser) et les dispositifs électroluminescents organiques (OLED).

13. Dispositif électronique selon la revendication 11, lequel est sélectionné parmi le groupe constitué par les dispositifs électroluminescents organiques, en particulier une diode émettrice de lumière organique (OLED), **caractérisé en ce que** le composé selon une ou plusieurs des revendications 1 à 5 ou le polymère, l'oligomère ou le dendrimère selon la revendication 8 ou une composition selon la revendication 9 est utilisé(e) pour une ou plusieurs des fonctions qui suivent :
- en tant que matériau de transport de trous dans une couche de transport de trous ou d'injection de trous ;
- en tant que matériau de matrice dans une couche d'émission ;
- en tant que matériau de blocage d'électrons ;
- en tant que matériau de blocage d'excitons.
